# EUROPEAN PATENT APPLICATION

(11) **EP 4 717 706 A1**
(43) Date of publication of application: **01.04.2026**
(21) Application number: 24811156.9
(22) Date of filing: 22.05.2024
(51) Int. Cl.: C07K 14/705, C12N 1/21, C12N 7/02, C12N 15/12, C12N 15/70, C12P 21/02, C07K 17/00

(54) **MODIFIED RECOMBINANT ADENO-ASSOCIATED VIRUS (AAV)-BINDING PROTEIN AND METHOD FOR PURIFYING AAV**

(30) Priority: 23.05.2023 JP 2023084423; 22.01.2024 JP 2024007357; 22.01.2024 JP 2024007358; 10.04.2024 JP 2024063328
(71) Applicant: Tosoh Corporation, Yamaguchi 746-8501 (JP)
(72) Inventor: IWABUCHI, Kiyoshige, Ayase-shi, Kanagawa 252-1123 (JP); KURIHARA, Kento, Ayase-shi, Kanagawa 252-1123 (JP); MANABE, Yuriko, Ayase-shi, Kanagawa 252-1123 (JP); YOSHIDA, Kouhei, Ayase-shi, Kanagawa 252-1123 (JP); OMURA, Keita, Ayase-shi, Kanagawa 252-1123 (JP); TANAKA, Toru, Ayase-shi, Kanagawa 252-1123 (JP)
(74) Representative: Novagraaf International SA
(86) International application number: PCT/JP2024/018896
(87) International publication number: WO 2024/242150

(57) **Abstract**

The present disclosure relates to a protein having affinity for an Adeno-Associated Virus (AAV). Provided are: (i) an AAV-binding protein comprising at least the amino acid residues from serine at position 25 to aspartic acid at position 213 of the amino acid sequence set forth in SEQ ID NO: 2, provided that one or more of particular amino acid substitutions have occurred at the amino acid residues from position 25 to position 213, the protein having AAV-binding activity; (ii) an AAV-binding protein comprising at least the amino acid residues from serine at position 25 to aspartic acid at position 213 of the amino acid sequence set forth in SEQ ID NO: 2, provided that one or more of the particular amino acid substitutions have occurred at the amino acid residues from position 25 to position 213, and any one or more of substitution, deletion, insertion or addition of one or several amino acid residues at one or several positions, other than the particular amino acid substitutions, have further occurred, the protein having AAV-binding activity; and (iii) an AAV-binding protein comprising an amino acid sequence having a homology of 70% or more to the entire amino acid sequence in which one or more of the particular amino acid substitutions have occurred in the amino acid sequence from serine at position 25 to aspartic acid at position 213 of the amino acid sequence set forth in SEQ ID NO: 2, wherein the at least any one amino acid substitution is retained, the protein having AAV-binding activity.

## Description

### Technical Field

The present disclosure relates to a protein having affinity for an Adeno-Associated Virus (AAV). In an aspect, the present disclosure relates to, for example, an improved recombinant AAV-binding protein with enhanced alkaline resistance.

In an aspect, furthermore, the present disclosure relates to a method for purifying an adeno-associated virus (AAV). Moreover, in an aspect, the present disclosure relates to, for example, a method by which an AAV vector including a gene can be easily purified.

### Background Art

Adeno-associated viruses (AAVs) are non-enveloped viruses classified into the genus Dependovirus of the family Parvoviridae. AAV capsid particles are composed of three types of proteins (VP1, VP2 and VP3), in which approximately 60 protein molecules are mixed in a ratio of VP1:VP2:VP3 = approximately 1:1:10, collectively forming the shape of a regular icosahedron having a diameter of 20 nm to 30 nm.

Due to the lack of autonomous proliferation capacity, the replication of AAVs in the natural world relies on helper viruses, such as adenoviruses and herpesviruses. In the presence of a helper virus, the AAV genome is replicated within host cells. Complete AAV particles containing the AAV genome are formed, and the AAV particles are released from the host cells. In the absence of a helper virus, however, the AAV genome is in a state in which it is maintained in the episome or integrated into the host chromosome (latent state).

AAVs are capable of infecting cells of a wide variety of species including humans. They infect even non-dividing cells that have completed differentiation, such as blood cells, muscles and nerve cells, are unlikely to cause adverse reactions because it is not pathogenic to humans, and its viral particles are physicochemically stable. For such reasons, AAVs are attracting attention for its usefulness as vectors for gene introduction intended for the treatment of congenital genetic diseases.

The manufacture of a recombinant AAV vector (hereinafter also referred to simply as an AAV vector) is typically performed by introducing nucleic acids encoding essential elements for the formation of AAV particles into cells to prepare cells capable of producing an AAV (hereinafter also referred to as AAV-producing cells) and culturing the cells to cause them to express the essential elements for the formation of AAV particles. The manufactured AAV vector is recovered and purified from the AAV-producing cells, whereby a therapeutic AAV vector formulation is obtained.

An example of a method for recovering and purifying an AAV vector from AAV-producing cells is a method utilizing affinity chromatography based on binding affinity for the AAV, in which an adsorbent including an insoluble support and an AAV-binding protein immobilized on the support is used. In this method, an AAV vector can be recovered and purified from a solution containing the vector in which impurities are also present. As a specific example, in Patent Literature 1, a polypeptide includes extracellular domains 1 (PKD1) and 2 (PKD2) of KIAA0319L (UniProt No. Q8IZA0), provided that at least one amino acid residue at a particular position in these domains has been substituted with a different amino acid residue. This polypeptide, which exhibits improved stability against heat, acids and alkalis by virtue of the substitution, is used as the AAV-binding protein immobilized on the insoluble support (hereinafter also referred to simply as "ligand protein"), whereby purification of an AAV vector with high purity is achieved.

When such an adsorbent is used in purification applications, however, the purification of the AAV is typically followed by alkali cleaning for residual AAV and impurities using a high-concentration (e.g., 0.1 M or more and 0.5 M or less) aqueous solution of sodium hydroxide. Accordingly, there is a need for the creation of a ligand protein that can withstand this alkali cleaning.

The manufactured AAV vectors include those including a gene (Full AAV vectors) and those lacking a gene (Empty AAV vectors). Of these, the Empty AAV vectors may reduce efficacy as a therapeutic drug and may cause adverse reactions resulting from overdosage (Non-Patent Literature 2). Using the product as a therapeutic AAV vector formulation, therefore, requires purifying the Full AAV vectors, from among the manufactured AAV vectors, with high purity.

Known purification methods for AAV vectors include methods utilizing affinity chromatography in which an AAV adsorbent including an insoluble support and an AAV-binding protein immobilized on the support is used (Patent Literature 1 and 2 and Non-Patent Literature 2). Even when purification is performed by this type of affinity chromatography, however, it has been difficult to remove Empty AAV vectors.

A known method for obtaining Full AAV vectors is the method of ultracentrifuging a solution containing manufactured AAV vectors and obtaining a fraction enriched in Full AAV vectors. Performing ultracentrifugation, however, requires complicated steps, such as preparing density gradient solutions, performing ultracentrifugation and obtaining the fraction, and obtaining the Full AAV vectors generally takes several days. It is, furthermore, difficult to distinguish the fraction enriched in Full AAV vectors from others. During fraction collection, therefore, the layer before or after the intended one may be obtained, and there is also a high risk of contamination.

In addition, the method of separating Full AAV vectors and Empty AAV vectors using Tosoh TSKgel Q-STAT as an anion-exchange gel and choline chloride as an eluent has been reported (Non-Patent Literature 3).

### Prior Art Literatures

### Patent Literature

Patent Literature 1: WO 2021/106882
Patent Literature 2: Japanese Translation of PCT International Application Publication No. 2018-507707

### Non-Patent Literature

Non-Patent Literature 1: Gerard A et al., Pharm Res, 36, 29 (2019)
Non-Patent Literature 2: Kai G et al., Mol Ther Methods Clin Dev, 1:9 (2014)
Non-Patent Literature 3: Sam Kurth et al., Anal Biochem, 686,115421 (2024)

### Summary of Invention

### Technical Problem

As stated above, the adeno-associated virus (AAV)-binding protein disclosed in WO 2021/106882 exhibits improved stability against heat, acids and alkalis compared with the wild type (the AAV-binding protein without amino acid substitutions). There has been, however, a demand for a ligand protein that can withstand alkali cleaning more effectively than the AAV-binding protein disclosed in the publication.

In an aspect, therefore, an object of the present disclosure is to provide an AAV-binding protein with improved alkaline resistance compared with the AAV-binding protein disclosed in the above publication.

As stated above, AAV vectors lacking a gene (Empty AAV vectors) may reduce efficacy as a therapeutic drug and may cause adverse reactions resulting from overdosage. Using the product as a therapeutic AAV vector formulation, therefore, has required purifying AAV vectors including a gene (Full AAV vectors), from among the manufactured AAV vectors, with high purity.

In an aspect, therefore, an object of the present disclosure is to provide a method by which Full AAV vectors can be purified with high purity and with ease. In an example of such an aspect, an object of the present disclosure is to provide, for example, a method by which Full AAV vectors can be purified with high purity and with ease from a solution containing AAV vectors obtained by culturing AAV-producing cells.

### Solution to Problem

As a result of intensive studies to solve the above problems, the inventors found that substituting particular one or more of the amino acid residues constituting an adeno-associated virus (AAV)-binding protein with different amino acid residues significantly improves alkaline resistance.

The inventors, furthermore, found that Full AAV vectors can be purified with high purity by purifying a solution containing AAV vectors by affinity chromatography using an AAV-binding protein as the ligand and then further subjecting the solution to anion-exchange chromatography.

That is, the present invention includes the aspects of [1] to [15] below.
[1] An AAV-binding protein selected from any of (i) to (iii) below:
   (i) an AAV-binding protein comprising at least amino acid residues from serine at position 25 to aspartic acid at position 213 of an amino acid sequence set forth in SEQ ID NO: 2, provided that at least any one of amino acid substitutions of (1) to (74) below has occurred at the amino acid residues from position 25 to position 213, the protein having AAV-binding activity;
      (1) substitution of threonine at position 139 of SEQ ID NO: 2 with alanine,
      (2) substitution of isoleucine at position 32 of SEQ ID NO: 2 with asparagine,
      (3) substitution of leucine at position 34 of SEQ ID NO: 2 with glutamine,
      (4) substitution of leucine at position 41 of SEQ ID NO: 2 with proline,
      (5) substitution of asparagine at position 42 of SEQ ID NO: 2 with lysine or serine,
      (6) substitution of valine at position 45 of SEQ ID NO: 2 with alanine,
      (7) substitution of valine at position 48 of SEQ ID NO: 2 with alanine,
      (8) substitution of lysine at position 51 of SEQ ID NO: 2 with arginine,
      (9) substitution of glutamic acid at position 53 of SEQ ID NO: 2 with glycine,
      (10) substitution of threonine at position 56 of SEQ ID NO: 2 with alanine or serine,
      (11) substitution of tyrosine at position 57 of SEQ ID NO: 2 with phenylalanine,
      (12) substitution of aspartic acid at position 58 of SEQ ID NO: 2 with glycine,
      (13) substitution of glutamine at position 60 of SEQ ID NO: 2 with lysine,
      (14) substitution of threonine at position 63 of SEQ ID NO: 2 with alanine,
      (15) substitution of glutamic acid at position 73 of SEQ ID NO: 2 with aspartic acid,
      (16) substitution of glutamine at position 78 of SEQ ID NO: 2 with arginine,
      (17) substitution of isoleucine at position 79 of SEQ ID NO: 2 with phenylalanine,
      (18) substitution of leucine at position 80 of SEQ ID NO: 2 with proline,
      (19) substitution of lysine at position 81 of SEQ ID NO: 2 with arginine,
      (20) substitution of leucine at position 82 of SEQ ID NO: 2 with serine,
      (21) substitution of asparagine at position 84 of SEQ ID NO: 2 with aspartic acid or serine,
      (22) substitution of leucine at position 89 of SEQ ID NO: 2 with proline,
      (23) substitution of tyrosine at position 90 of SEQ ID NO: 2 with phenylalanine or asparagine,
      (24) substitution of glutamic acid at position 91 of SEQ ID NO: 2 with glycine,
      (25) substitution of tyrosine at position 92 of SEQ ID NO: 2 with serine,
      (26) substitution of alanine at position 94 of SEQ ID NO: 2 with threonine,
      (27) substitution of valine at position 95 of SEQ ID NO: 2 with isoleucine,
      (28) substitution of glutamic acid at position 97 of SEQ ID NO: 2 with aspartic acid,
      (29) substitution of histidine at position 102 of SEQ ID NO: 2 with arginine,
      (30) substitution of valine at position 107 of SEQ ID NO: 2 with alanine or isoleucine,
      (31) substitution of valine at position 109 of SEQ ID NO: 2 with glutamic acid,
      (32) substitution of threonine at position 110 of SEQ ID NO: 2 with alanine or serine,
      (33) substitution of glutamic acid at position 114 of SEQ ID NO: 2 with glycine,
      (34) substitution of proline at position 115 of SEQ ID NO: 2 with serine,
      (35) substitution of valine at position 125 of SEQ ID NO: 2 with alanine,
      (36) substitution of aspartic acid at position 148 of SEQ ID NO: 2 with alanine,
      (37) substitution of glutamic acid at position 166 of SEQ ID NO: 2 with aspartic acid,
      (38) substitution of glutamic acid at position 167 of SEQ ID NO: 2 with aspartic acid,
      (39) substitution of alanine at position 174 of SEQ ID NO: 2 with proline,
      (40) substitution of serine at position 179 of SEQ ID NO: 2 with threonine,
      (41) substitution of glutamine at position 180 of SEQ ID NO: 2 with asparagine,
      (42) substitution of asparagine at position 185 of SEQ ID NO: 2 with lysine,
      (43) substitution of lysine at position 210 of SEQ ID NO: 2 with glutamic acid,
      (44) substitution of aspartic acid at position 213 of SEQ ID NO: 2 with asparagine,
      (45) substitution of glycine at position 27 of SEQ ID NO: 2 with aspartic acid,
      (46) substitution of aspartic acid at position 30 of SEQ ID NO: 2 with glycine,
      (47) substitution of glutamine at position 31 of SEQ ID NO: 2 with arginine,
      (48) substitution of proline at position 35 of SEQ ID NO: 2 with alanine,
      (49) substitution of glutamic acid at position 38 of SEQ ID NO: 2 with valine,
      (50) substitution of glutamine at position 60 of SEQ ID NO: 2 with arginine,
      (51) substitution of glutamic acid at position 75 of SEQ ID NO: 2 with aspartic acid,
      (52) substitution of histidine at position 76 of SEQ ID NO: 2 with proline,
      (53) substitution of tyrosine at position 92 of SEQ ID NO: 2 with asparagine,
      (54) substitution of glycine at position 105 of SEQ ID NO: 2 with glutamic acid,
      (55) substitution of valine at position 111 of SEQ ID NO: 2 with alanine,
      (56) substitution of glutamic acid at position 112 of SEQ ID NO: 2 with lysine,
      (57) substitution of arginine at position 119 of SEQ ID NO: 2 with histidine,
      (58) substitution of glutamine at position 128 of SEQ ID NO: 2 with arginine,
      (59) substitution of phenylalanine at position 129 of SEQ ID NO: 2 with leucine,
      (60) substitution of leucine at position 134 of SEQ ID NO: 2 with proline,
      (61) substitution of serine at position 146 of SEQ ID NO: 2 with glycine,
      (62) substitution of threonine at position 147 of SEQ ID NO: 2 with serine,
      (63) substitution of aspartic acid at position 150 of SEQ ID NO: 2 with valine,
      (64) substitution of glutamic acid at position 158 of SEQ ID NO: 2 with lysine,
      (65) substitution of lysine at position 168 of SEQ ID NO: 2 with arginine,
      (66) substitution of serine at position 170 of SEQ ID NO: 2 with arginine,
      (67) substitution of threonine at position 187 of SEQ ID NO: 2 with arginine,
      (68) substitution of threonine at position 191 of SEQ ID NO: 2 with isoleucine,
      (69) substitution of alanine at position 198 of SEQ ID NO: 2 with valine,
      (70) substitution of threonine at position 199 of SEQ ID NO: 2 with alanine,
      (71) substitution of leucine at position 206 of SEQ ID NO: 2 with methionine,
      (72) substitution of asparagine at position 209 of SEQ ID NO: 2 with glutamic acid,
      (73) substitution of valine at position 212 of SEQ ID NO: 2 with isoleucine and
      (74) substitution of aspartic acid at position 213 of SEQ ID NO: 2 with glycine;
   (ii) an AAV-binding protein comprising at least amino acid residues from serine at position 25 to aspartic acid at position 213 of an amino acid sequence set forth in SEQ ID NO: 2, provided that at least any one of the amino acid substitutions of (1) to (74) has occurred at the amino acid residues from position 25 to position 213, and any one or more of substitution, deletion, insertion or addition of one or several amino acid residues at one or several positions, other than the amino acid substitutions specified in (1) to (74), have further occurred, the protein having AAV-binding activity; or
   (iii) an AAV-binding protein comprising an amino acid sequence having a homology of 70% or more to an entire amino acid sequence in which at least any one of the amino acid substitutions of (1) to (74) has occurred in an amino acid sequence from serine at position 25 to aspartic acid at position 213 of an amino acid sequence set forth in SEQ ID NO: 2, wherein the at least any one amino acid substitution is retained, the protein having AAV-binding activity.
[2] The AAV-binding protein according to [1] selected from any of (iv) to (vi) below:
   (iv) an AAV-binding protein comprising at least amino acid residues from serine at position 25 to aspartic acid at position 213 of an amino acid sequence set forth in SEQ ID NO: 2, provided that at least an amino acid substitution of (1) below has occurred at the amino acid residues from position 25 to position 213, the protein having AAV-binding activity;
      (1) substitution of threonine at position 139 of SEQ ID NO: 2 with alanine;
   (v) an AAV-binding protein comprising at least amino acid residues from serine at position 25 to aspartic acid at position 213 of an amino acid sequence set forth in SEQ ID NO: 2, provided that the amino acid substitution of (1) has occurred at the amino acid residues from position 25 to position 213, and any one or more of substitution, deletion, insertion or addition of one or several amino acid residues at one or several positions, other than the amino acid substitution specified in (1), has further occurred, the protein having AAV-binding activity; or
   (vi) an AAV-binding protein comprising an amino acid sequence having a homology of 70% or more to an entire amino acid sequence in which at least the amino acid substitution of (1) has occurred in an amino acid sequence from serine at position 25 to aspartic acid at position 213 of an amino acid sequence set forth in SEQ ID NO: 2, wherein the at least any one amino acid substitution is retained, the protein having AAV-binding activity.
[3] The AAV-binding protein according to [1] or [2], wherein the protein includes at least amino acid residues from serine at position 25 to aspartic acid at position 213 of an amino acid sequence set forth in SEQ ID NO: 2, provided that at least amino acid substitutions specified in any of (A) to (P) below have occurred at the amino acid residues from position 25 to position 213, the protein having AAV-binding activity;
   (A) substitution of asparagine at position 42 of SEQ ID NO: 2 with lysine, substitution of glutamic acid at position 114 of SEQ ID NO: 2 with glycine and substitution of threonine at position 139 of SEQ ID NO: 2 with alanine,
   (B) substitution of lysine at position 51 of SEQ ID NO: 2 with arginine, substitution of threonine at position 139 of SEQ ID NO: 2 with alanine and substitution of alanine at position 174 of SEQ ID NO: 2 with proline,
   (C) substitution of asparagine at position 42 of SEQ ID NO: 2 with lysine, substitution of glutamic acid at position 114 of SEQ ID NO: 2 with glycine, substitution of threonine at position 139 of SEQ ID NO: 2 with alanine and substitution of alanine at position 174 of SEQ ID NO: 2 with proline,
   (D) substitution of lysine at position 51 of SEQ ID NO: 2 with arginine, substitution of glutamic acid at position 114 of SEQ ID NO: 2 with glycine, substitution of threonine at position 139 of SEQ ID NO: 2 with alanine and substitution of alanine at position 174 of SEQ ID NO: 2 with proline,
   (E) substitution of asparagine at position 42 of SEQ ID NO: 2 with lysine, substitution of lysine at position 51 of SEQ ID NO: 2 with arginine, substitution of glutamic acid at position 114 of SEQ ID NO: 2 with glycine, substitution of threonine at position 139 of SEQ ID NO: 2 with alanine and substitution of alanine at position 174 of SEQ ID NO: 2 with proline,
   (F) substitution of asparagine at position 42 of SEQ ID NO: 2 with lysine, substitution of glutamic acid at position 114 of SEQ ID NO: 2 with glycine, substitution of threonine at position 139 of SEQ ID NO: 2 with alanine, substitution of alanine at position 174 of SEQ ID NO: 2 with proline and substitution of glutamine at position 180 of SEQ ID NO: 2 with asparagine,
   (G) substitution of asparagine at position 42 of SEQ ID NO: 2 with lysine, substitution of isoleucine at position 79 of SEQ ID NO: 2 with phenylalanine, substitution of glutamic acid at position 114 of SEQ ID NO: 2 with glycine, substitution of threonine at position 139 of SEQ ID NO: 2 with alanine, substitution of alanine at position 174 of SEQ ID NO: 2 with proline, substitution of glutamine at position 180 of SEQ ID NO: 2 with asparagine and substitution of threonine at position 187 of SEQ ID NO: 2 with arginine,
   (H) substitution of asparagine at position 42 of SEQ ID NO: 2 with lysine, substitution of valine at position 107 of SEQ ID NO: 2 with alanine, substitution of glutamic acid at position 114 of SEQ ID NO: 2 with glycine, substitution of threonine at position 139 of SEQ ID NO: 2 with alanine, substitution of alanine at position 174 of SEQ ID NO: 2 with proline, substitution of glutamine at position 180 of SEQ ID NO: 2 with asparagine and substitution of threonine at position 187 of SEQ ID NO: 2 with arginine,
   (I) substitution of asparagine at position 42 of SEQ ID NO: 2 with lysine, substitution of glutamic acid at position 114 of SEQ ID NO: 2 with glycine, substitution of threonine at position 139 of SEQ ID NO: 2 with alanine, substitution of lysine at position 168 of SEQ ID NO: 2 with arginine, substitution of alanine at position 174 of SEQ ID NO: 2 with proline, substitution of glutamine at position 180 of SEQ ID NO: 2 with asparagine and substitution of threonine at position 187 of SEQ ID NO: 2 with arginine,
   (J) substitution of asparagine at position 42 of SEQ ID NO: 2 with lysine, substitution of isoleucine at position 79 of SEQ ID NO: 2 with phenylalanine, substitution of valine at position 107 of SEQ ID NO: 2 with alanine, substitution of glutamic acid at position 114 of SEQ ID NO: 2 with glycine, substitution of threonine at position 139 of SEQ ID NO: 2 with alanine, substitution of lysine at position 168 of SEQ ID NO: 2 with arginine, substitution of alanine at position 174 of SEQ ID NO: 2 with proline and substitution of glutamine at position 180 of SEQ ID NO: 2 with asparagine,
   (K) substitution of asparagine at position 42 of SEQ ID NO: 2 with lysine, substitution of isoleucine at position 79 of SEQ ID NO: 2 with phenylalanine, substitution of valine at position 107 of SEQ ID NO: 2 with alanine, substitution of glutamic acid at position 114 of SEQ ID NO: 2 with glycine, substitution of threonine at position 139 of SEQ ID NO: 2 with alanine, substitution of lysine at position 168 of SEQ ID NO: 2 with arginine, substitution of alanine at position 174 of SEQ ID NO: 2 with proline, substitution of glutamine at position 180 of SEQ ID NO: 2 with asparagine and substitution of threonine at position 187 of SEQ ID NO: 2 with arginine,
   (L) substitution of glycine at position 27 of SEQ ID NO: 2 with aspartic acid, substitution of asparagine at position 42 of SEQ ID NO: 2 with lysine, substitution of isoleucine at position 79 of SEQ ID NO: 2 with phenylalanine, substitution of valine at position 107 of SEQ ID NO: 2 with alanine, substitution of glutamic acid at position 114 of SEQ ID NO: 2 with glycine, substitution of threonine at position 139 of SEQ ID NO: 2 with alanine, substitution of lysine at position 168 of SEQ ID NO: 2 with arginine, substitution of alanine at position 174 of SEQ ID NO: 2 with proline and substitution of glutamine at position 180 of SEQ ID NO: 2 with asparagine,
   (M) substitution of asparagine at position 42 of SEQ ID NO: 2 with lysine, substitution of isoleucine at position 79 of SEQ ID NO: 2 with phenylalanine, substitution of valine at position 107 of SEQ ID NO: 2 with alanine, substitution of glutamic acid at position 114 of SEQ ID NO: 2 with glycine, substitution of threonine at position 139 of SEQ ID NO: 2 with alanine, substitution of lysine at position 168 of SEQ ID NO: 2 with arginine, substitution of alanine at position 174 of SEQ ID NO: 2 with proline, substitution of glutamine at position 180 of SEQ ID NO: 2 with asparagine and substitution of lysine at position 210 of SEQ ID NO: 2 with glutamic acid,
   (N) substitution of asparagine at position 42 of SEQ ID NO: 2 with lysine, substitution of isoleucine at position 79 of SEQ ID NO: 2 with phenylalanine, substitution of valine at position 107 of SEQ ID NO: 2 with alanine, substitution of glutamic acid at position 114 of SEQ ID NO: 2 with glycine, substitution of threonine at position 139 of SEQ ID NO: 2 with alanine, substitution of lysine at position 168 of SEQ ID NO: 2 with arginine, substitution of alanine at position 174 of SEQ ID NO: 2 with proline, substitution of glutamine at position 180 of SEQ ID NO: 2 with asparagine and substitution of valine at position 212 of SEQ ID NO: 2 with isoleucine and
   (O) substitution of glycine at position 27 of SEQ ID NO: 2 with aspartic acid, substitution of asparagine at position 42 of SEQ ID NO: 2 with lysine, substitution of isoleucine at position 79 of SEQ ID NO: 2 with phenylalanine, substitution of valine at position 107 of SEQ ID NO: 2 with alanine, substitution of glutamic acid at position 114 of SEQ ID NO: 2 with glycine, substitution of threonine at position 139 of SEQ ID NO: 2 with alanine, substitution of lysine at position 168 of SEQ ID NO: 2 with arginine, substitution of alanine at position 174 of SEQ ID NO: 2 with proline, substitution of glutamine at position 180 of SEQ ID NO: 2 with asparagine, substitution of lysine at position 210 of SEQ ID NO: 2 with glutamic acid and substitution of valine at position 212 of SEQ ID NO: 2 with isoleucine.
[4] A polynucleotide encoding the AAV-binding protein according to any of [1] to [3].
[5] An expression vector comprising the polynucleotide according to [4].
[6] A transformant obtained by transforming Escherichia coli with the expression vector according to [5].
[7] A method for manufacturing an AAV-binding protein, the method comprising a step of allowing an AAV-binding protein to be expressed by culturing the transformant according to [6] and a step of recovering the expressed AAV-binding protein from a resulting culture.
[8] An AAV adsorbent comprising an insoluble support and the AAV-binding protein according to any of [1] to [3] immobilized on the support.
[9] A column comprising the AAV adsorbent according to [8].
[10] A method for purifying or analyzing an AAV, the method comprising a step of applying a solution containing an AAV to the column according to [9] to cause the AAV to be adsorbed onto the adsorbent and a step of eluting the AAV adsorbed on the adsorbent using an eluent.
[11] The method according to [10] for purifying or analyzing an AAV, further comprising a step of washing, with an alkali solution, the AAV adsorbent that has completed the step of eluting the AAV.
[12] A method for purifying an AAV contained in a sample, the method comprising:
   a step of applying a sample containing an AAV to an adsorbent including an insoluble support and an AAV-binding protein immobilized on the insoluble support to cause the AAV to be adsorbed onto the adsorbent;
   a step of eluting the AAV adsorbed on the adsorbent;
   a step of applying an AAV-containing fraction eluted in the elution step to a support for anion-exchange chromatography to cause the AAV to be adsorbed onto the support for anion-exchange chromatography; and
   a step of eluting the AAV adsorbed on the support for anion-exchange chromatography.
[13] A method for purifying an AAV contained in a sample, the method comprising:
   a step of applying a sample containing an AAV to an adsorbent including an insoluble support and an AAV-binding protein immobilized on the insoluble support to cause the AAV to be adsorbed onto the adsorbent;
   a step of eluting the AAV adsorbed on the adsorbent;
   a step of applying an AAV-containing fraction eluted in the elution step to a support for anion-exchange chromatography to cause the AAV to be adsorbed onto the support for anion-exchange chromatography; and
   a step of eluting the AAV adsorbed on the support for anion-exchange chromatography, wherein:
      the AAV-binding protein is the AAV-binding protein according to any of [1] to [3].
[14] The purification method according [12] or [13], wherein the step of eluting the AAV adsorbed on the support for anion-exchange chromatography is a step of eluting the AAV adsorbed on the support using an eluent having an electrical conductivity of 13.5 mS/cm or less and then eluting the AAV remaining on the support using an eluent having an electrical conductivity of 15.0 mS/cm or more.
[15] The purification method according to [14], wherein the eluents contain choline chloride.

### Brief Description of Drawings

[Fig. 1] Fig. 1 is a chromatogram obtained by applying a solution containing an AAV vector (AAV8-EGFP) to affinity chromatography (an AVR29c column).
[Fig. 2] Fig. 2 is a chromatogram obtained by applying an AAV8-EGFP-containing fraction purified using an AVR29c column to anion-exchange chromatography (a GigaCap Q column).
[Fig. 3] Fig. 3 is a chromatogram obtained by applying a solution containing AAV8-EGFP directly to a GigaCap Q column.
[Fig. 4] Fig. 4 includes chromatograms obtained by applying each peak presented in the chromatogram in Fig. 3 to size-exclusion chromatography (a G6000PW_{XL} column).
[Fig. 5] Fig. 5 is a diagram in which the purity of AVR29c and AVR29c(-) was examined by SDS-PAGE (sodium dodecyl sulfate-polyacrylamide gel electrophoresis).
[Fig. 6] Fig. 6 is a diagram illustrating the results of an evaluation of the affinity of AVR29c or AVR29c(-) for VLP2.
[Fig. 7] Fig. 7 is a diagram illustrating the results of an evaluation of the elution of AAV8 adsorbed on an AVR29c column or an AVR29c(-) column.
[Fig. 8] Fig. 8 includes chromatograms obtained by applying AAV8-EGFP-containing fractions purified using an AVR29c column to an anion-exchange chromatography column (a SkillPak GigaCap Q column; capacity, 1.0 mL). The chromatograms are for cases in which the percentage of solution AEX-C, which was a 20 mmol/L Tris hydrochloride buffer (pH 9.0) containing 300 mmol/L choline chloride, mixed with solution AEX-A for the elution of impurities was adjusted to be (a) 49.0%, (b) 48.0%, (c) 47.0% and (d) 46.5%.
[Fig. 9] Fig. 9 includes chromatograms obtained by applying AAV8-EGFP-containing fractions purified using an AVR29c column to a SkillPak GigaCap Q column (capacity, 5.0 mL). (a) is a chromatogram obtained by affinity chromatography (AF) purification using an AVR29c column. (b) is a chromatogram obtained by anion-exchange chromatography (AEX) purification using a SkillPak GigaCap Q column.

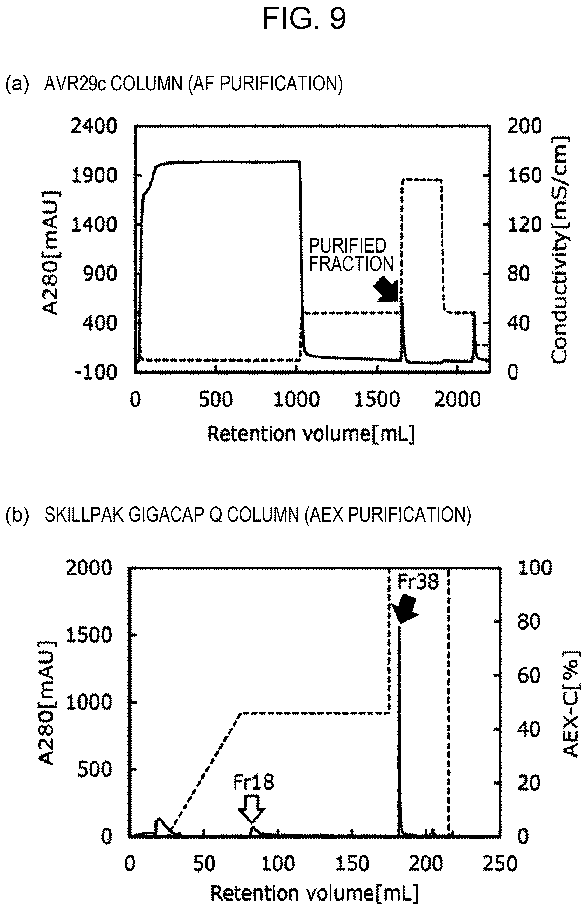

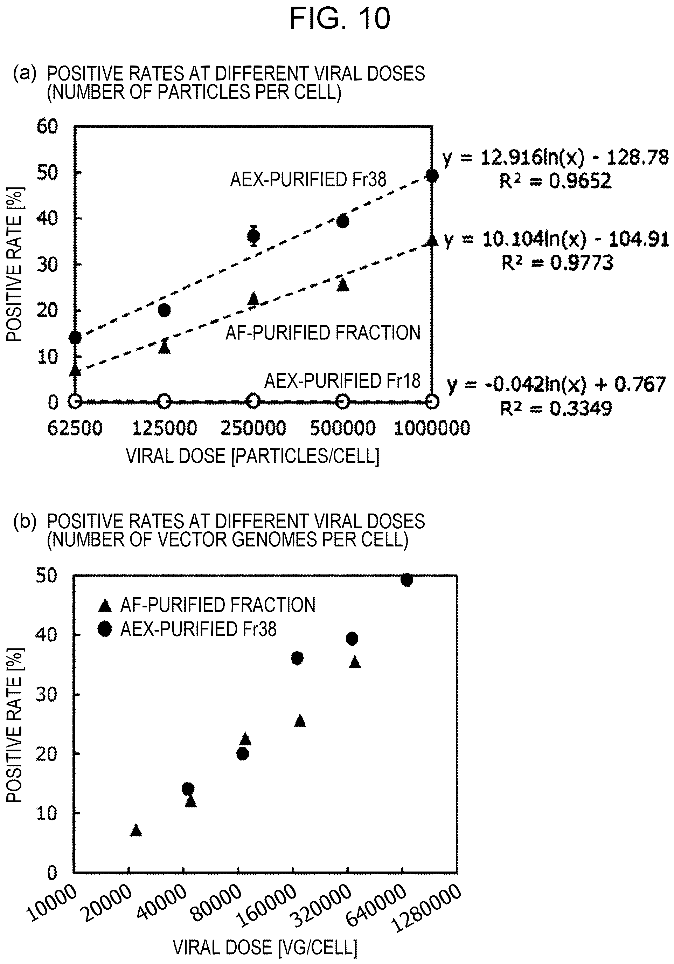

[Fig. 10] Fig. 10 includes diagrams in which positive rates in cellular infection performed using an AAV8-EGFP solution obtained in Example 24 (1) (the peak indicated by the black arrow in Fig. 9 (a)) and eluted fractions obtained in Example 24 (3) (the peak indicated by the white arrow in Fig. 9 (b)) and (4) (the peak indicated by the black arrow in Fig. 9 (b)) separately. It should be noted that in (a), the viral dose, indicated on the X-axis, is represented by the number of particles per cell. In (b), the viral dose, indicated on the X-axis, is represented by the number of vector genomes (VG) per cell.

### Description of Embodiments

In the following, the present disclosure will be described in detail.

As used herein, an AAV-binding protein is a protein including at least the amino acid residues from serine (S) at position 312 to aspartic acid (D) at position 500, which constitute regions corresponding to extracellular domains 1 (PKD1) and 2 (PKD2), of the amino acid sequence of KIAA0319L (UniProt No. Q8IZA0), which is a wild-type AAV-binding protein and is set forth in SEQ ID NO: 1, provided that amino acid substitutions at particular positions have occurred at the amino acid residues from position 312 to position 500. An AAV-binding protein according to the present disclosure, therefore, may include all or a subset of the other extracellular domains located closer to the C-terminus of the protein (domain 3 (PKD3), domain 4 (PKD4) and domain 5 (PKD5)). An AAV-binding protein according to the present disclosure, furthermore, may include all or a subset of signal sequences or cysteine-rich domains, such as the MANSC (Motif at N Terminus with Seven Cysteines) domain located closer to the N-terminus than PKD1. Moreover, an AAV-binding protein according to the present disclosure may include all or a subset of transmembrane domains and intracellular domains located closer to the N-terminus and/or the C-terminus than the extracellular domains.

The aforementioned amino acid substitutions at particular positions specifically involve at least the amino acid residues from serine at position 312 to aspartic acid at position 500 of the amino acid sequence set forth in SEQ ID NO: 1, provided that the substitutions include, at the amino acid residues from position 312 to position 500,
at least the amino acid substitutions V317D (this notation denotes that valine at position 317 of SEQ ID NO: 1 has been substituted with aspartic acid; the same applies hereinafter), N324H, V326A, A330V, Q334L, E335V, T341A, Y342S, K362E, K371N, F379Y, K380R, V381A, I382V, G390S, K399E, K467Q, S476R, S482T, N487D and N492D. When at least one of any amino acid substitution from among I319N, L321Q, L328P, N329K, N329S, V332A, E(V)335A (this notation denotes that glutamic acid at position 335 of SEQ ID NO: 1 was once substituted with valine in SEQ ID NO: 2 and then substituted with alanine; the same applies hereinafter), K338R, E340G, T343A, T343S, Y344F, D345G, Q347K, T350A, E360D, Q365R, I366F, L367P, K368R, L369S, K(N)371D, K(N)371S, L376P, Y377F, Y377N, E378G, F(Y)379S, V(A)381T, I(V)382I, E384D, H389R, V394A, V394I, V396E, T397A, T397S, E401G, P402S, V412A, T426A, D435A, E453D, E454D, A461P, S466T, K(Q)467N, N472K, K497E, D500N, G314D, V(D)317G, Q318R, P322A, E325V, Q347R, E362D, H363P, F(Y)379N, G392E, V398A, K(E)399K, R406H, Q415R, F416L, L421P, S433G, T434S, D437V, E445K, K455R, S457R, T474R, T478I, A485V, T486A, L493M, N496D, V499I and D500G has occurred at the same time, it is preferred because stability against alkalis is improved. In particular, an AAV-binding protein in which at least the amino acid substitution T426A has occurred is a more preferred form.

It should be noted that an AAV-binding protein consisting of the amino acid residues from serine at position 312 to aspartic acid at position 500 of the amino acid sequence set forth in SEQ ID NO: 1, provided that the amino acid substitutions V317D, N324H, V326A, A330V, Q334L, E335V, T341A, Y342S, K362E, K371N, F379Y, K380R, V381A, I382V, G390S, K399E, K467Q, S476R, S482T, N487D and N492D have occurred at the amino acid residues from position 312 to position 500, is identical to an AAV-binding protein consisting of the amino acid residues from serine at position 25 to aspartic acid at position 213 of the amino acid sequence set forth in SEQ ID NO: 2. The positions of the amino acid residues in SEQ ID NO: 2, furthermore, correspond to the positions of the amino acid residues in SEQ ID NO: 1 minus 287. Specifically, the amino acid substitution V317D corresponds to the substitution of the amino acid residue located at position 30 in SEQ ID NO: 2, and the amino acid substitution T426A corresponds to the substitution of the amino acid residue located at position 139 in SEQ ID NO: 2.

For the AAV-binding protein according to the present disclosure, there is no specific restriction on the number of amino acids substituted. Examples include the AAV-binding proteins specified in any of (A) to (P) below. These AAV-binding proteins are more preferred because stability against alkalis is improved.

(A) An AAV-binding protein that includes the amino acid residues from serine at position 25 to aspartic acid at position 213 of the amino acid sequence set forth in SEQ ID NO: 2 and in which at least the amino acid substitutions N329K, E401G and T426A have occurred at the amino acid residues from position 25 to position 213.
(B) An AAV-binding protein that includes the amino acid residues from serine at position 25 to aspartic acid at position 213 of the amino acid sequence set forth in SEQ ID NO: 2 and in which at least the amino acid substitutions K338R, T426A and A461P have occurred at the amino acid residues from position 25 to position 213.
(C) An AAV-binding protein that includes the amino acid residues from serine at position 25 to aspartic acid at position 213 of the amino acid sequence set forth in SEQ ID NO: 2 and in which at least the amino acid substitutions N329K, E401G, T426A and A461P have occurred at the amino acid residues from position 25 to position 213.
(D) An AAV-binding protein that includes the amino acid residues from serine at position 25 to aspartic acid at position 213 of the amino acid sequence set forth in SEQ ID NO: 2 and in which at least the amino acid substitutions K338R, E401G, T426A and A461P have occurred at the amino acid residues from position 25 to position 213.
(E) An AAV-binding protein that includes the amino acid residues from serine at position 25 to aspartic acid at position 213 of the amino acid sequence set forth in SEQ ID NO: 2 and in which at least the amino acid substitutions N329K, K338R, E401G, T426A and A461P have occurred at the amino acid residues from position 25 to position 213.
(F) An AAV-binding protein that includes the amino acid residues from serine at position 25 to aspartic acid at position 213 of the amino acid sequence set forth in SEQ ID NO: 2 and in which at least the amino acid substitutions N329K, E401G, T426A, A461P and K(Q)467N have occurred at the amino acid residues from position 25 to position 213.
(G) An AAV-binding protein that includes the amino acid residues from serine at position 25 to aspartic acid at position 213 of the amino acid sequence set forth in SEQ ID NO: 2 and in which at least the amino acid substitutions N329K, I366F, E401G, T426A, A461P, K(Q)467N and T474R have occurred at the amino acid residues from position 25 to position 213.
(H) An AAV-binding protein that includes the amino acid residues from serine at position 25 to aspartic acid at position 213 of the amino acid sequence set forth in SEQ ID NO: 2 and in which at least the amino acid substitutions N329K, V394A, E401G, T426A, A461P, K(Q)467N and T474R have occurred at the amino acid residues from position 25 to position 213.
(I) An AAV-binding protein that includes the amino acid residues from serine at position 25 to aspartic acid at position 213 of the amino acid sequence set forth in SEQ ID NO: 2 and in which at least the amino acid substitutions N329K, E401G, T426A, K455R, A461P, K(Q)467N and T474R have occurred at the amino acid residues from position 25 to position 213.
(J) An AAV-binding protein that includes the amino acid residues from serine at position 25 to aspartic acid at position 213 of the amino acid sequence set forth in SEQ ID NO: 2 and in which at least the amino acid substitutions N329K, I366F, V394A, E401G, T426A, K455R, A461P and K(Q)467N have occurred at the amino acid residues from position 25 to position 213.
(K) An AAV-binding protein that includes the amino acid residues from serine at position 25 to aspartic acid at position 213 of the amino acid sequence set forth in SEQ ID NO: 2 and in which at least the amino acid substitutions N329K, I366F, V394A, E401G, T426A, K455R, A461P, K(Q)467N and T474R have occurred at the amino acid residues from position 25 to position 213.
(L) An AAV-binding protein that includes the amino acid residues from serine at position 25 to aspartic acid at position 213 of the amino acid sequence set forth in SEQ ID NO: 2 and in which at least the amino acid substitutions G314D, N329K, I366F, V394A, E401G, T426A, K455R, A461P and K(Q)467N have occurred at the amino acid residues from position 25 to position 213.
(M) An AAV-binding protein that includes the amino acid residues from serine at position 25 to aspartic acid at position 213 of the amino acid sequence set forth in SEQ ID NO: 2 and in which at least the amino acid substitutions N329K, I366F, V394A, E401G, T426A, K455R, A461P, K(Q)467N and K497E have occurred at the amino acid residues from position 25 to position 213.
(N) An AAV-binding protein that includes the amino acid residues from serine at position 25 to aspartic acid at position 213 of the amino acid sequence set forth in SEQ ID NO: 2 and in which at least the amino acid substitutions N329K, I366F, V394A, E401G, T426A, K455R, A461P, K(Q)467N and V499I have occurred at the amino acid residues from position 25 to position 213.
(O) An AAV-binding protein that includes the amino acid residues from serine at position 25 to aspartic acid at position 213 of the amino acid sequence set forth in SEQ ID NO: 2 and in which at least the amino acid substitutions G314D, N329K, I366F, V394A, E401G, T426A, K455R, A461P, K(Q)467N, K497E and V499I have occurred at the amino acid residues from position 25 to position 213.

In (ii) and (v) above, "one or several" refers to, as an example, any of 1 to 50, 1 to 40, 1 to 30, 1 to 25, 1 to 20, 1 to 18, 1 to 15, 1 to 10, 1 to 9, 1 to 8, 1 to 7, 1 to 6, 1 to 5, 1 to 4, 1 to 3, 1 or 2 or 1, although depending partly on the positions of the amino acid residues within the three-dimensional structure of the AAV-binding protein and the types of the amino acid residues.

Examples of substitutions, deletions, insertions or additions as mentioned in (ii) and (v) above include the substitutions of amino acid residues disclosed in WO 2021/106882 and WO 2023/140197.

It should be noted that as the "substitution (...) of one or several amino acid residues" in (ii) and (v) above, a conservative substitution, in which substitution occurs between amino acids having similar physical properties and/or chemical properties, may occur, in addition to the aforementioned amino acid substitutions at particular positions. It is known to those skilled in the art that in the case of a conservative substitution, in general, the function of the protein is maintained between the protein in which the substitution has occurred and the protein in which the substitution has not occurred. An example of a conservative substitution is substitution between glycine and alanine, between serine and threonine or between glutamic acid and aspartic acid (Protein Structure and Function, Medical Sciences International, Ltd., 9, 2005). Another example of an amino acid substitution as described above, furthermore, is substitution for monomerizing the AAV-binding protein. A specific example is an amino acid substitution in which a cysteine residue, which is likely to form a higher-order structure, is replaced with a serine residue or a methionine residue.

Moreover, the "substitution, deletion, insertion or addition of one or several amino acid residues" in (ii) or (v) above also includes mutations that can also occur naturally (mutants or variants), which are based on factors such as differences in the origin of the AAV-binding protein or differences in species.

The homology of the amino acid sequence in (iii) and (vi) above only needs to be 70% or more; the sequence may have a higher homology (e.g., 80% or more, 85% or more, 90% or more or 95% or more). The "homology of the amino acid sequence" refers to homology to the entire amino acid sequence. "Homology" between amino acid sequences refers to the percentage of amino acid residues that are of the same kind in both amino acid sequences (Experimental Medicine, 31 (3), Yodosha Co., Ltd.). Homology between amino acid sequences can be determined using an alignment program, such as BLAST (Basic Local Alignment Search Tool) or FASTA.

In the present disclosure, a method is provided in which an AAV contained in a sample is purified using an adsorbent including an insoluble support and an AAV-binding protein immobilized on the support (hereinafter also referred to as "AAV adsorbent"). For the AAV-binding protein, there is no specific restriction as long as it is a polypeptide capable of binding to an AAV. Examples include laminin receptors, such as integrins, anti-AAV antibodies and AAV receptors (AAVRs).

An example of a preferred form of the AAV-binding protein when it is an anti-AAV antibody is a polypeptide that includes at least a variable region of a heavy-chain antibody (VHH) capable of binding to an AAV. An example of a preferred form of the AAV-binding protein when it is an AAVR, furthermore, is a polypeptide selected from any of <i> to <iii> below.
<i> A polypeptide that includes at least the amino acid residues from serine at position 312 to aspartic acid at position 500 of the amino acid sequence set forth in SEQ ID NO: 1,
<ii> a polypeptide that includes at least the amino acid residues from serine at position 312 to aspartic acid at position 500 of the amino acid sequence set forth in SEQ ID NO: 1, provided that any one or more of substitution, deletion, insertion or addition of one or several amino acid residues at one or several positions have occurred at the amino acid residues from position 312 to position 500, and that has AAV-binding activity or
<iii> a polypeptide that includes at least the amino acid residues from serine at position 312 to aspartic acid at position 500 of the amino acid sequence set forth in SEQ ID NO: 1, provided that the polypeptide has a homology of 70% or more to the entire amino acid sequence consisting of the amino acid residues from position 312 to position 500, and
   that has AAV-binding activity.

It should be noted that the amino acid sequence set forth in SEQ ID NO: 1 is the amino acid sequence of KIAA0319L (official database, UniProt; accession number, Q8IZA0), which is a form of an AAVR, and that the amino acid residues from serine (S) at position 312 to aspartic acid (D) at position 500 of the amino acid sequence set forth in SEQ ID NO: 1 constitute regions corresponding to extracellular domains 1 (PKD1) and 2 (PKD2) of KIAA0319L.

The polypeptide specified in any of <i> to <iii> above only needs to include the above-mentioned regions corresponding to PKD1 and PKD2 of KIAA0319L. For example, it may include all or a subset of regions corresponding to the other extracellular domains located closer to the C-terminus than PKD2 (domain 3 (PKD3), domain 4 (PKD4) and domain 5 (PKD5)), may include all or a subset of regions corresponding to signal sequences or cysteine-rich domains, such as the MANSC (Motif at N terminus with Seven Cysteines) domain located closer to the N-terminus than PKD1, or may include all or a subset of transmembrane domains and intracellular domains located closer to the N-terminus and/or the C-terminus than the extracellular domains.

Examples of <ii> above include the AAV-binding proteins disclosed in WO 2021/106882 and WO 2023/140197 and AAV-binding proteins having at least one or more of the amino acid substitutions specified in (I) to (LXIX) below;
(I) substitution of glycine at position 314 of SEQ ID NO: 1 with aspartic acid,
(II) substitution of valine at position 317 of SEQ ID NO: 1 with glycine,
(III) substitution of glutamine at position 318 of SEQ ID NO: 1 with arginine,
(IV) substitution of isoleucine at position 319 of SEQ ID NO: 1 with asparagine,
(V) substitution of leucine at position 321 of SEQ ID NO: 1 with glutamine,
(VI) substitution of proline at position 322 of SEQ ID NO: 1 with alanine,
(VII) substitution of glutamic acid at position 325 of SEQ ID NO: 1 with valine,
(VIII) substitution of leucine at position 328 of SEQ ID NO: 1 with proline,
(IX) substitution of asparagine at position 329 of SEQ ID NO: 1 with lysine or serine,
(X) substitution of valine at position 332 of SEQ ID NO: 1 with alanine,
(XI) substitution of glutamic acid at position 335 of SEQ ID NO: 1 with alanine,
(XII) substitution of lysine at position 338 of SEQ ID NO: 1 with arginine,
(XIII) substitution of glutamic acid at position 340 of SEQ ID NO: 1 with glycine,
(XIV) substitution of threonine at position 343 of SEQ ID NO: 1 with alanine or serine,
(XV) substitution of tyrosine at position 344 of SEQ ID NO: 1 with phenylalanine,
(XVI) substitution of aspartic acid at position 345 of SEQ ID NO: 1 with glycine,
(XVII) substitution of glutamine at position 347 of SEQ ID NO: 1 with lysine or arginine,
(XVIII) substitution of threonine at position 350 of SEQ ID NO: 1 with alanine,
(XIX) substitution of glutamic acid at position 360 of SEQ ID NO: 1 with aspartic acid,
(XX) substitution of lysine at position 362 of SEQ ID NO: 1 with aspartic acid,
(XXI) substitution of histidine at position 363 of SEQ ID NO: 1 with proline,
(XXII) substitution of glutamine at position 365 of SEQ ID NO: 1 with arginine,
(XXIII) substitution of isoleucine at position 366 of SEQ ID NO: 1 with phenylalanine,
(XXIV) substitution of leucine at position 367 of SEQ ID NO: 1 with proline,
(XXV) substitution of lysine at position 368 of SEQ ID NO: 1 with arginine,
(XXVI) substitution of leucine at position 369 of SEQ ID NO: 1 with serine,
(XXVII) substitution of lysine at position 371 of SEQ ID NO: 1 with aspartic acid or serine,
(XXVIII) substitution of leucine at position 376 of SEQ ID NO: 1 with proline,
(XXIX) substitution of tyrosine at position 377 of SEQ ID NO: 1 with phenylalanine or asparagine,
(XXX) substitution of glutamic acid at position 378 of SEQ ID NO: 1 with glycine,
(XXXI) substitution of phenylalanine at position 379 of SEQ ID NO: 1 with serine or asparagine,
(XXXII) substitution of valine at position 381 of SEQ ID NO: 1 with threonine,
(XXXIII) substitution of glutamic acid at position 384 of SEQ ID NO: 1 with aspartic acid,
(XXXIV) substitution of histidine at position 389 of SEQ ID NO: 1 with arginine,
(XXXV) substitution of glycine at position 392 of SEQ ID NO: 1 with glutamic acid,
(XXXVI) substitution of valine at position 394 of SEQ ID NO: 1 with alanine or isoleucine,
(XXXVII) substitution of valine at position 396 of SEQ ID NO: 1 with glutamic acid,
(XXXVIII) substitution of threonine at position 397 of SEQ ID NO: 1 with alanine or serine,
(XXXIV) substitution of valine at position 398 of SEQ ID NO: 1 with alanine,
(XL) substitution of glutamic acid at position 401 of SEQ ID NO: 1 with glycine,
(XLI) substitution of proline at position 402 of SEQ ID NO: 1 with serine,
(XLII) substitution of arginine at position 406 of SEQ ID NO: 1 with histidine,
(XLIII) substitution of valine at position 412 of SEQ ID NO: 1 with alanine,
(XLIV) substitution of glutamine at position 415 of SEQ ID NO: 1 with arginine,
(XLV) substitution of phenylalanine at position 416 of SEQ ID NO: 1 with leucine,
(XLVI) substitution of leucine at position 421 of SEQ ID NO: 1 with proline,
(XLVII) substitution of threonine at position 426 of SEQ ID NO: 1 with alanine,
(XLVIII) substitution of serine at position 433 of SEQ ID NO: 1 with glycine,
(XLIX) substitution of threonine at position 434 of SEQ ID NO: 1 with serine,
(L) substitution of aspartic acid at position 435 of SEQ ID NO: 1 with alanine,
(LI) substitution of aspartic acid at position 437 of SEQ ID NO: 1 with valine,
(LII) substitution of glutamic acid at position 445 of SEQ ID NO: 1 with lysine,
(LIII) substitution of glutamic acid at position 453 of SEQ ID NO: 1 with aspartic acid,
(LIV) substitution of glutamic acid at position 454 of SEQ ID NO: 1 with aspartic acid,
(LV) substitution of lysine at position 455 of SEQ ID NO: 1 with arginine,
(LVI) substitution of serine at position 457 of SEQ ID NO: 1 with arginine,
(LVII) substitution of alanine at position 461 of SEQ ID NO: 1 with proline,
(LVIII) substitution of serine at position 466 of SEQ ID NO: 1 with threonine,
(LIX) substitution of lysine at position 467 of SEQ ID NO: 1 with asparagine,
(LX) substitution of asparagine at position 472 of SEQ ID NO: 1 with lysine,
(LXI) substitution of threonine at position 474 of SEQ ID NO: 1 with arginine,
(LXII) substitution of threonine at position 478 of SEQ ID NO: 1 with isoleucine,
(LXIII) substitution of alanine at position 485 of SEQ ID NO: 1 with valine,
(LXIV) substitution of threonine at position 486 of SEQ ID NO: 1 with alanine,
(LXV) substitution of leucine at position 493 of SEQ ID NO: 1 with methionine,
(LXVI) substitution of asparagine at position 496 of SEQ ID NO: 1 with glutamic acid,
(LXVII) substitution of lysine at position 497 of SEQ ID NO: 1 with glutamic acid,
(LXVIII) substitution of valine at position 499 of SEQ ID NO: 1 with isoleucine and
(LXIX) substitution of aspartic acid at position 500 of SEQ ID NO: 1 with glycine or asparagine

Examples of substitutions, deletions, insertions or additions as mentioned in <ii> above, furthermore, include the substitutions of amino acid residues disclosed in WO 2021/106882 and WO 2023/140197 and the amino acid substitutions specified in (I) to (LXIX) above.

"One or several" in <ii> above refers to, as an example, any of 1 or more and 50 or fewer, 1 or more and 40 or fewer, 1 or more and 30 or fewer, 1 or more and 25 or fewer, 1 or more and 20 or fewer, 1 or more and 15 or fewer, 1 or more and 10 or fewer, 1 or more and 9 or fewer, 1 or more and 8 or fewer, 1 or more and 7 or fewer, 1 or more and 6 or fewer, 1 or more and 5 or fewer, 1 or more and 4 or fewer, 1 or more and 3 or fewer, 1 or more and 2 or fewer or 1, although depending partly on the positions of the amino acid residues within the three-dimensional structure of the AAVR and the types of the amino acid residues. The substitution of the "one or several" amino acid residues may occur, for example, at positions other than those of the substitutions of amino acid residues disclosed in WO 2021/106882 and WO 2023/140197 and those of the substitutions of amino acid residues specified in (I) to (LXIX) above, as long as the polypeptide retains AAV-binding activity.

It should be noted that as the "substitution (...) of one or several amino acid residues" in <ii> above, a conservative substitution, in which substitution occurs between amino acids having similar physical properties and/or chemical properties, may occur, in addition to the aforementioned amino acid substitutions at particular positions. It is known to those skilled in the art that in the case of a conservative substitution, in general, the function of the protein is maintained between the protein in which the substitution has occurred and the protein in which the substitution has not occurred. An example of a conservative substitution is substitution between glycine and alanine, between serine and threonine or between glutamic acid and aspartic acid (Protein Structure and Function, Medical Sciences International, Ltd., 9, 2005). The "substitution, deletion, insertion or addition of one or several amino acid residues" in <ii> above, furthermore, also includes mutations that can also occur naturally (mutants or variants), which are based on factors such as differences in the origin of the AAVR or differences in species.

The homology of the amino acid sequence in <iii> above only needs to be 70% or more; the sequence may have a higher homology (e.g., 80% or more, 85% or more, 90% or more or 95% or more). "Homology" between amino acid sequences refers to the percentage of amino acid residues that are of the same kind in both amino acid sequences (Experimental Medicine, 31 (3), Yodosha Co., Ltd.). Homology between amino acid sequences can be determined using an alignment program, such as BLAST (Basic Local Alignment Search Tool) or FASTA.

In an aspect, the AAV-binding protein according to the present disclosure may further include an oligopeptide, attached to its N-terminus or C-terminus, that is useful when separating the protein from a solution in the presence of impurities. Examples of such oligopeptides include polyhistidine, polylysine, polyarginine, polyglutamic acid and polyaspartic acid. Furthermore, an oligopeptide including cysteine, which is useful when immobilizing the AAV-binding protein onto a solid phase, such as a support for chromatography, may also be attached to the N-terminus or C-terminus of the AAV-binding protein.

For the length of an oligopeptide attached to the N-terminus or C-terminus of the AAV-binding protein, there is no specific restriction unless the affinity for the AAV or stability of the AAV-binding protein is impaired. When such an oligopeptide is attached to the AAV-binding protein according to the present disclosure, a polynucleotide encoding the oligopeptide may be produced, and then this polynucleotide may be attached to the N-terminus or C-terminus of the AAV-binding protein by gene engineering using a method known to those skilled in the art. Alternatively, such an oligopeptide may be chemically synthesized, and this oligopeptide may be attached to the N-terminus or C-terminus of the AAV-binding protein via chemical bonding.

To the N-terminus of the AAV-binding protein according to the present disclosure, furthermore, a signal peptide for promoting efficient expression in Escherichia coli, which is used as the host, may be attached. Examples of such signal peptides include signal peptides that induce protein secretion into the periplasmic space, such as PelB, OmpA, DsbA, DsbC, MalE and TorT (Japanese Unexamined Patent Application Publication No. 2011-097898). Moreover, the addition of a signal peptide to the N-terminus may be omitted. This is preferred because in this case the homogeneity of the protein is improved during protein preparation.

Examples of methods for producing a polynucleotide encoding the AAV-binding protein according to the present disclosure (hereinafter also referred to as "polynucleotide according to the present disclosure") include:
(I) the method of converting the amino acid sequence of the AAV-binding protein according to the present disclosure into a nucleotide sequence and artificially synthesizing a polynucleotide including this nucleotide sequence; and
(II) the method of preparing a polynucleotide including the full-length or a partial sequence of the AAV-binding protein, either directly and artificially or using a DNA amplification technique, such as PCR, for example from a cDNA for the AAV-binding protein, and ligating the prepared polynucleotide by an appropriate method.

When the amino acid sequence is converted into a nucleotide sequence in the method in (I) above, it is preferred to perform the conversion considering the codon usage frequency in Escherichia coli, which is the host to be transformed. Specifically, AGA/AGG/CGG/CGA for arginine (R), ATA for isoleucine (I), CTA for leucine (L), GGA for glycine (G) and CCC for proline (P) are used with low frequency (codons commonly referred to as rare codons); therefore, the conversion can be performed such that these codons are avoided. The analysis of the codon usage frequency is also possible through the utilization of a public database (e.g., the Codon Usage Database, available at the website of Kazusa DNA Research Institute).

When mutations are introduced into the polynucleotide according to the present disclosure, error-prone PCR can be used. For the reaction conditions for error-prone PCR, there is no specific limitation as long as the conditions are ones under which the desired mutations can be introduced into a polynucleotide encoding an AAV-binding protein. For example, by making the concentrations of the four substrate deoxynucleotides (dATP/dTTP/dCTP/dGTP) disproportionate, adding MnCl₂ at a concentration of 0.01 mM or more and 10 mM or less (preferably, 0.1 mM or more and 1 mM or less) to the PCR reaction solution and performing PCR, mutations can be introduced into a polynucleotide. An example of a method for mutagenesis other than error-prone PCR, furthermore, is the method of producing the mutant by introducing mutations into a polynucleotide including the full-length or a partial sequence of the AAV-binding protein, for example by bringing a chemical agent serving as the mutagen into contact with, and allowing it to act on, the polynucleotide or by irradiating the polynucleotide with ultraviolet radiation. The chemical agent used as the mutagen in this method can be, for example, a mutagenic agent commonly used by those skilled in the art, such as hydroxylamine, N-methyl-N'-nitro-N-nitrosoguanidine, nitrous acid, sulfurous acid or hydrazine.

When Escherichia coli as the host is transformed using a polynucleotide according to the present disclosure, the polynucleotide according to the present disclosure itself may be used, but it is more preferred to use an expression vector (e.g., a bacteriophage, cosmid or plasmid that is commonly used for transformation of prokaryotic or eukaryotic cells) in which the polynucleotide according to the present disclosure has been inserted at an appropriate position. It should be noted that for this expression vector, there is no specific restriction as long as it can stably exist and replicate within the host to be transformed (Escherichia coli). Examples include a pET plasmid vector, a pUC plasmid vector, a pTrc plasmid vector and a pCDF plasmid vector.

The appropriate position, furthermore, may refer to a position at which the insertion does not disrupt regions responsible for the replicating function, desired antibiotic marker or transferability of the expression vector. When the polynucleotide according to the present disclosure is inserted into the expression vector, it is preferred to insert the polynucleotide in a state in which it is linked to a functional polynucleotide, such as the promoter required for expression. Examples of such promoters include the trp promoter, the tac promoter, the trc promoter, the lac promoter, the T7 promoter, the recA promoter and the lpp promoter.

To transform Escherichia coli as the host using the expression vector produced by the method described above, into which a polynucleotide according to the present disclosure has been inserted (hereinafter also referred to simply as "expression vector according to the present disclosure"), transformation can be performed by a method commonly used by those skilled in the art. Specifically, transformation can be performed by a method described in a known publication, such as Molecular Cloning (Cold Spring Harbor Laboratory), 256, 1992. The transformants obtained by transformation performed using such a method are screened by an appropriate method, whereby a transformant capable of expressing the AAV-binding protein according to the present disclosure (hereinafter also referred to simply as "transformant according to the present disclosure") can be obtained.

To prepare an expression vector according to the present disclosure from a transformant according to the present disclosure, preparation from a culture obtained by culturing the transformant according to the present disclosure can be performed by alkali extraction or using a commercially available extraction kit, such as a QIAprep Spin Miniprep kit (manufactured by QIAGEN N.V.).

By culturing the transformant according to the present disclosure and recovering the AAV-binding protein according to the present disclosure from the resulting culture, the AAV-binding protein can be manufactured. It should be noted that as used herein, a culture also includes the medium used for culturing, as well as the cultured cells of the transformant according to the present disclosure themselves.

The transformant used in the protein manufacturing method can be cultured in a medium suitable for culturing the host of interest (Escherichia coli). An example of a preferred medium is LB (Luria-Bertani) medium supplemented with necessary nutrition sources. Incidentally, it is preferred to add a drug corresponding to a drug resistance gene included in the vector according to the present disclosure to the medium before culturing, so that the transformant is selectively grown depending on whether or not the vector has been introduced. For example, when the vector includes the kanamycin resistance gene, kanamycin can be added to the medium.

The medium, furthermore, may be supplemented with appropriate nutrition sources in addition to carbon, nitrogen and inorganic salt sources, and may contain one or more reducing agents selected from the group consisting of glutathione, cysteine, cystamine, thioglycolate and dithiothreitol as desired. The culturing temperature is generally 10°C or above and 40°C or below, preferably 20°C or above and 37°C or below, more preferably approximately 25°C, but can be selected according to the characteristics of the protein to be expressed. The pH of the medium is pH 6.8 or above and pH 7.4 or below, preferably approximately pH 7.0. When the vector according to the present disclosure includes an inducible promotor, furthermore, it is preferred to apply induction under conditions such that the AAV-binding protein according to the present disclosure can be successfully expressed.

An example of an inducer is IPTG (Isopropyl-β-D-thiogalactopyranoside). By measuring the turbidity (absorbance at 600 nm) of the culture broth, adding an appropriate amount of IPTG when it reaches approximately 0.5 or greater and 1.0 or less and then continuing culturing, the expression of the AAV-binding protein can be induced. The concentration of IPTG added can be selected as appropriate within the range of 0.005 mM to 1.0 mM, both inclusive, but preferably falls within the range of 0.01 mM to 0.5 mM, both inclusive. As for the various conditions regarding induction with IPTG, the induction can be performed under conditions well known in the technical field.

To recover the AAV-binding protein according to the present disclosure from the culture obtained by culturing the transformant according to the present disclosure, the AAV-binding protein can be separated/purified from the culture and recovered by a method suitable for the form of expression of the AAV-binding protein in the transformant. For example, when the protein is expressed in the culture supernatant, the bacterial cells can be separated through a centrifugation operation, and the AAV-binding protein can be purified from the resulting culture supernatant. When the protein is expressed within the cells (optionally including the periplasm), furthermore, the bacterial cells can be harvested through a centrifugation operation, then the AAV-binding protein can be extracted by disrupting the bacterial cells by adding, for example, an enzymatic treatment agent or a surfactant, and the protein can be purified thereafter.

To purify the AAV-binding protein according to the present disclosure, a method known in the technical field can be used. An example is separation/purification using liquid chromatography. Examples of types of liquid chromatography include ion-exchange chromatography, hydrophobic interaction chromatography, gel filtration chromatography and affinity chromatography. By performing a purification operation by combining such types of chromatography, the AAV-binding protein according to the present disclosure can be prepared with high purity.

For the method for measuring the binding activity of the resulting AAV-binding protein according to the present disclosure to the AAV, the binding activity to the AAV can be measured using, for example, Enzyme-Linked ImmunoSorbent Assay (hereinafter referred to as ELISA). The AAV used for the measurement of binding activity may be VLPs (virus-like particles). An AAV vector and VLPs of any serotype, furthermore, may be used as long as the VLPs exhibit binding activity to the AAV-binding protein according to the present disclosure.

The AAV-binding protein according to the present disclosure can be used for, for example, purification or analysis of an AAV. When the protein is used for this purpose, there is no specific limitation on the AAV to which it binds; it may be a naturally occurring AAV or may be an artificially produced AAV. Examples of naturally occurring AAVs include serotype 1 (AAV1), serotype 2 (AAV2), serotype 3 (AAV3), serotype 4 (AAV4), serotype 5 (AAV5), serotype 6 (AAV6), serotype 7 (AAV7), serotype 8 (AAV8), serotype 9 (AAV9), serotype 10 (AAV10), serotype 11 (AAV11), serotype 12 (AAV12) and serotype 13 (AAV13). Examples of artificially produced AAVs, furthermore, include AAVrh8, AAVrh10 and chimeric AAVs, which have characteristics (cell tropism and infectivity) of two or more of such serotypes.

The AAV-binding protein according to the present disclosure can be, for example, immobilized onto an insoluble support before use. That is, the purification or analysis of an AAV can specifically be conducted using, for example, an AAV adsorbent comprising an insoluble support and the AAV-binding protein according to the present disclosure immobilized on the insoluble support. Herein, an AAV adsorbent comprising an insoluble support and the AAV-binding protein according to the present disclosure immobilized on the insoluble support is also referred to as an AAV adsorbent according to the present disclosure. It should be noted that purification of an AAV is not limited to the purification of an AAV from a solution in which impurities are allowed to coexist; the purification of an AAV based on structure, properties or activity, for example, is also included.

The insoluble support as a component of the AAV adsorbent according to the present disclosure is not particularly restricted, as long as it is insoluble in the sample containing the AAV and the solutions used for purification (e.g., eluents, equilibrating solutions and washing liquids). Examples of insoluble supports include supports made from polysaccharides, such as agarose, alginates (alginic acid salts), carrageenan, chitin, cellulose, dextrin, dextran and starch, supports made from synthetic polymers, such as polyvinyl alcohol, polymethacrylates, poly(2-hydroxyethyl methacrylate) and polyurethane, and supports made from ceramics, such as silica. Of these, supports made from polysaccharides and supports made from synthetic polymers are particularly preferred as insoluble supports. Examples of such preferred supports include polymethacrylate gels into which hydroxy groups have been introduced, such as TOYOPEARL (manufactured by Tosoh Corporation), agarose gels, such as Sepharose (manufactured by Cytiva), and cellulose gels, such as Cellufine (manufactured by JNC Corporation). The shape of the insoluble support is not particularly restricted. For example, the support may be any of a granular material, a monolithic material, a film-shaped material or a fibrous material, and may be either porous or nonporous. In particular, it is preferred that the support be shaped such that it can be packed in a column.

When the AAV adsorbent is manufactured, the immobilization of the AAV-binding protein onto the insoluble support can be achieved via, for example, covalent bonding. Specifically, the AAV adsorbent can be manufactured by, for example, covalently bonding the AAV-binding protein and the insoluble support via active groups that the insoluble support has, thereby immobilizing the protein onto the insoluble support. Examples of such active groups include N-hydroxysuccinimide (NHS)-activated ester groups, epoxy groups, carboxy groups, maleimide groups, haloacetyl groups, tresyl groups, formyl groups and haloacetamide groups. The insoluble support having active groups may be, for example, a commercially available insoluble support having active groups used as is or may be an insoluble support into which active groups have been introduced. Examples of commercially available supports having active groups include TOYOPEARL AF-Epoxy-650M and TOYOPEARL AF-Tresyl-650M (both manufactured by Tosoh Corporation), HiTrap NHS-activated HP Columns, NHS-activated Sepharose 4 Fast Flow and Epoxy-activated Sepharose 6B (all manufactured by Cytiva) and SulfoLink Coupling Resin (manufactured by Thermo Fisher Scientific Inc.).

An example of a method for introducing active groups onto the surface of the support is the method of allowing one of compounds having two or more active sites to react with, for example, hydroxy groups, epoxy groups, carboxy groups or amino groups present on the surface of the support.

Examples of compounds that introduce epoxy groups to hydroxy groups or amino groups present on the surface of the support include epichlorohydrin, ethanediol diglycidyl ether, butanediol diglycidyl ether and hexanediol diglycidyl ether.

Examples of compounds that introduce carboxy groups to epoxy groups present on the surface of the support, furthermore, include 2-mercaptoacetic acid, 3-mercaptopropionic acid, 4-mercaptobutyric acid, 6-mercaptobutyric acid, glycine, 3-aminopropionic acid, 4-aminobutyric acid and 6-aminohexanoic acid.

Moreover, examples of compounds that introduce maleimide groups to hydroxy groups, epoxy groups, carboxy groups or amino groups present on the surface of the support include N-(ε-maleimidocaproic acid)hydrazide, N-(ε-maleimidopropionic acid)hydrazide, 4-(4-N-maleimidophenyl)acetic acid hydrazide, 2-aminomaleimide, 3-aminomaleimide, 4-aminomaleimide, 6-aminomaleimide, 1-(4-aminophenyl)maleimide, 1-(3-aminophenyl)maleimide, 4-(maleimido)phenyl isocyanate, 2-maleimidoacetic acid, 3-maleimidopropionic acid, 4-maleimidobutyric acid, 6-maleimidohexanoic acid, N-(α-maleimidoacetoxy)succinimide ester, (m-maleimidobenzoyl) N-hydroxysuccinimide ester, succinimidyl-4-(maleimidomethyl)cyclohexane-1-carbonyl-(6-aminohexanoic acid), succinimidyl-4-(maleimidomethyl)cyclohexane-1-carboxylic acid, (p-maleimidobenzoyl) N-hydroxysuccinimide ester and (m-maleimidobenzoyl) N-hydroxysuccinimide ester.

Examples of compounds that introduce haloacetyl groups to hydroxy groups or amino groups present on the surface of the support, furthermore, include chloroacetic acid, bromoacetic acid, iodoacetic acid, chloroacetyl chloride, bromoacetyl chloride, bromoacetyl bromide, chloroacetic anhydride, bromoacetic anhydride, iodoacetic anhydride, 2-(iodoacetamido)acetic acid-N-hydroxysuccinimide ester, 3-(bromoacetamido)propionic acid-N-hydroxysuccinimide ester and 4-(iodoacetyl)aminobenzoic acid-N-hydroxysuccinimide ester.

In addition, the method of allowing an ω-alkenylalkane glycidyl ether to react with hydroxy groups or amino groups present on the surface of the support and then halogenating the ω-alkenyl moiety with a halogenating agent to activate it is also an example of a method for introducing active groups onto the surface of the support. Examples of ω-alkenylalkane glycidyl ethers include allyl glycidyl ether, 3-butenyl glycidyl ether and 4-pentenyl glycidyl ether. Examples of halogenating agents include N-chlorosuccinimide, N-bromosuccinimide and N-iodosuccinimide.

The method of introducing the active groups to carboxy groups present on the surface of the support using a condensing agent and an additive, furthermore, is also an example of a method for introducing active groups onto the surface of the support. Examples of condensing agents include 1-ethyl-3-(3-dimethylaminopropyl)carbodiimide (EDC), dicyclohexylcarbodiamide and carbonyldiimidazole. Examples of additives include NHS, 4-nitrophenol and 1-hydroxybenztriazole.

The immobilization of the AAV-binding protein onto the insoluble support can be conducted in, for example, a buffer. Examples of buffers include acetate buffer, phosphate buffer, MES (2-MorpholinoEthaneSulfonic acid) buffer, HEPES (4-(2-HydroxyEthyl)-1-PiperazineEthaneSulfonic acid) buffer, Tris (Tris(hydroxymethyl)aminomethane) buffer and borate buffer. The reaction temperature during the immobilization can be set as appropriate, for example according to conditions such as the reactivity of the active groups and the stability of the AAV-binding protein. The reaction temperature during the immobilization may be, for example, 4°C or above and 50°C or below. Preferably, the reaction temperature may be 10°C or above and 35°C or below.

The AAV adsorbent according to the present disclosure can be, for example, packed into a column and used for purification or analysis of an AAV. Specifically, for example, by applying a solution containing an AAV to a column packed with the AAV adsorbent according to the present disclosure (hereinafter also referred to simply as "column according to the present disclosure") to cause the AAV to be adsorbed onto the adsorbent and eluting the AAV adsorbed on the adsorbent, purification or analysis of an AAV can be performed. That is, in an aspect, the present disclosure provides a method for purifying or analyzing an AAV, the method comprising a step of applying a solution containing an AAV to a column according to the present disclosure to cause the AAV to be adsorbed onto the adsorbent and a step of eluting the AAV adsorbed on the adsorbent. The purification of an AAV using a column according to the present disclosure can be conducted based on, for example, the disclosure in WO 2021/106882.

By purifying an AAV using an AAV adsorbent according to the present disclosure, a purified AAV can be obtained. That is, the method for purifying an AAV may be, in an aspect, a method for manufacturing an AAV, and, specifically, may be a method for manufacturing a purified AAV. The AAV is obtained as, for example, an eluted fraction containing the AAV. That is, a fraction containing an eluted AAV can be collected. The collection of the AAV fraction can be performed by, for example, ordinary methods. Examples of methods for collecting the AAV fraction include the method of replacing the collection vessel at regular time intervals or regular volume intervals, the method of changing the collection vessel according to the shape of the chromatogram of the eluate and the method of collecting fractions using, for example, an automatic fraction collector, such as an autosampler. The recovery of the AAV from the fraction containing the AAV, furthermore, is also possible. The recovery of the AAV from the fraction containing the AAV can be performed by, for example, known methods used for the purification of proteins.

By washing the AAV adsorbent according to the present disclosure with an alkali, contamination by residual AAV and impurities can be prevented. That is, a method for washing an AAV adsorbent may be, in an aspect, a method that is performed after purification of an AAV and in which the AAV adsorbent is washed with an alkali. Since the AAV-binding protein according to the present disclosure also has resistance to acids, furthermore, the method may be one in which the AAV adsorbent is washed with a low-pH solution first and then washed with an alkali. An example of a washing alkali solution used is a 0.1 M to 0.5 M, both inclusive, aqueous solution of sodium hydroxide.

In an aspect, the method according to the present disclosure for purifying an AAV may mean purifying an AAV vector including a gene (a Full AAV vector).

In an aspect, the method according to the present disclosure for purifying an AAV comprises:
a step of applying a sample containing an AAV to an AAV adsorbent to cause the AAV to be adsorbed onto the adsorbent (hereinafter also referred to as "first adsorption step");
a step of eluting the AAV adsorbed on the adsorbent (hereinafter also referred to as "first elution step");
a step of applying the AAV-containing fraction eluted in the first elution step to a support for anion-exchange chromatography to cause the AAV to be adsorbed onto the support (hereinafter also referred to as "second adsorption step"); and
a step of eluting the AAV adsorbed on the support for anion-exchange chromatography (hereinafter also referred to as "second elution step"). It should be noted that configuring the AAV adsorbent and the support for anion-exchange chromatography in a form in which they are packed in columns (hereinafter also referred to as "AAV adsorbent column" and "anion-exchange column," respectively) is preferred because in that case the above steps can be easily carried out. In the following, a detailed description will be given, taking as an example a configuration in which an AAV adsorbent column and an anion-exchange column are used.

The sample containing an AAV can be applied to the AAV adsorbent column using, for example, liquid delivery means, such as a pump. It should be noted that herein, applying a liquid to a column is also expressed as "delivering a liquid to a column." In addition, the sample containing an AAV may be subjected to prior solvent replacement using an appropriate buffer before application to the AAV adsorbent column. Before the application of the sample containing an AAV to the AAV adsorbent column (i.e., before the first adsorption step), furthermore, the AAV adsorbent column may be equilibrated using an appropriate buffer (equilibrating solution). Through this equilibration, it is expected that the AAV can be, for example, purified with higher purity. The buffers used for solvent replacement or equilibration can be any neutral buffers, which have buffer capacity in the neutral region (herein referring to the range of pH 5.0 to 9.0, both inclusive, preferably pH 5.5 to 8.0, both inclusive). Specific examples include phosphate buffer, acetate buffer, succinate buffer, citrate buffer, Tris buffer, HEPES buffer and MES buffer. To such a buffer, 0.1 mmol/L to 50 mmol/L of a chelating agent, for example, may be further added. The buffer used for solvent replacement and the equilibrating solution may be the same or may not be the same.

When components other than the AAV, such as impurities, remain in the AAV adsorbent column after the passage of the sample containing an AAV through the AAV adsorbent column, furthermore, such components may be removed (washed away) from the AAV adsorbent column before the elution of the AAV adsorbed on the AAV adsorbent (i.e., before the elution step). The components other than the AAV can be removed from the AAV adsorbent column by using, for example, an appropriate buffer as a washing liquid. For this washing liquid, the description of the buffers used for solvent replacement or equilibration is applicable.

In an aspect, in the first elution step, the AAV adsorbed onto the AAV adsorbent through the first adsorption step can be eluted using, for example, a buffer having a lower pH than the equilibrating solution or washing liquid described above, a neutral buffer containing 800 mmol/L or more of chloride ions or anions ranked lower in the Hofmeister series or a buffer containing a chelating agent (e.g., ethylenediaminetetraacetic acid) adjusted to the pH to be used in the second adsorption step. By passing such an eluent through the AAV adsorbent column, the interactions between the AAV and the AAV-binding protein (ligand in the AAV adsorbent) within the column are weakened. As a result, the AAV adsorbed on the AAV adsorbent is eluted, and a fraction containing the AAV is obtained.

The AAV-containing fraction obtained in the first elution step may be, for example, adjusted to approximately pH 8.0 to 9.5 using Tris or may be diluted with the buffer to be used in the second adsorption step.

The anion-exchange column can be manufactured by, for example, immersing the support for anion-exchange chromatography in a 1 mol/L aqueous solution of sodium chloride and then packing it into a column. The buffer components used in the second adsorption step and the second elution step can be any buffer components having buffer capacity near pH 8.0 to 9.5, preferably are Tris.

In an aspect, examples of preferred salts to be contained in the eluent in the second elution step include sodium chloride, sodium acetate, ammonium chloride, ammonium acetate, tetramethylammonium chloride, magnesium chloride, calcium chloride, ammonium sulfate, tetraethylammonium chloride, choline chloride, acetylcholine chloride, carnitine hydrochloride and trimethylglycine. More preferably, the salt is tetraethylammonium chloride or choline chloride. Inclusion of choline chloride, in particular, in the eluent is preferred because it enables purification of Full AAVs having infectivity with high purity. Full AAV may refer to an AAV including a gene.

In the second elution step, for example, the concentration of the above-mentioned salt is increased to an appropriate level using a linear gradient to elute a first peak, and then the salt concentration is increased using a step gradient to elute a second peak. For example, when AAV8 is used, a fraction enriched in Empty AAVs is obtained in the first peak, and a fraction enriched in Full AAVs is obtained in the second peak.

In an aspect, it is preferred to set the electrical conductivity of the eluent used to elute the first peak to 13.5 mS/cm or less while setting the electrical conductivity of the eluent used to elute the second peak to 15.0 mS/cm or more because this improves the percentage of Full AAVs contained in the second peak. It is, furthermore, more preferred to set the electrical conductivity of the eluent used to elute the first peak to 5.0 mS/cm or more and 13.5 mS/cm or less while setting the electrical conductivity of the eluent used to elute the second peak to 15.0 mS/cm or more and 50.0 mS/cm or less. It is even more preferred to set the electrical conductivity of the eluent used to elute the first peak to 12.0 mS/cm or more and 13.5 mS/cm or less while setting the electrical conductivity of the eluent used to elute the second peak to 20.0 mS/cm or more and 40.0 mS/cm or less. For the method for measuring the electrical conductivity, a built-in conductivity monitor in a liquid chromatography system such as AKTA go (manufactured by Cytiva) can be used.

Herein, the percentage of AAV vectors including a gene is also referred to as a Full ratio. For the method for measuring the Full ratio, Mass Photometry, such as that performed using Refeyn Two (manufactured by Refeyn Ltd.), can be employed.

### EXAMPLES

The present disclosure will now be described in further detail using examples and reference examples. The present disclosure, however, is not limited to these examples. It should be noted that the reference examples do not constitute the present disclosure.

EXAMPLE 1 Preparation of an AAV Vector (Part 1)
(1) The Escherichia coli strain JM109 was transformed using the pRC2-mi342 Vector (manufactured by Takara Bio Inc.), which is a plasmid including a polynucleotide encoding capsids of AAV cerotype 2 (AAV2), and the pHelper Vector (manufactured by Takara Bio Inc.). The resulting transformant was shake-cultured overnight at 37°C in a 5-L baffled flask containing 1 L of 2× YT medium (1.6% (w/v) Tryptone, 1% (w/v) Yeast Extract and 0.5% (w/v) sodium chloride) containing 100 µg/mL carbenicillin.
(2) After the bacterial cells were harvested by centrifuging the culture broth in (1), large amounts of the pRC2-mi342 Vector and the pHelper Vector were prepared from the harvested bacterial cells using a Plasmid Mega Kit (manufactured by QIAGEN N.V.).
(3) HEK293T cells were cultured in five T-225 flasks (manufactured by Thermo Fisher Scientific Inc.) containing 40 mL of D-MEM medium (manufactured by FUJIFILM Wako Pure Chemical Corporation) containing 10% (v/v) bovine serum.
(4) Using the TransIT-ViruGEN Transfection Reagent (manufactured by Takara Bio Inc.), the pRC2-mi342 Vector and the pHelper Vector prepared in (2) were introduced into the HEK293T cells cultured in (3). The cells were statically cultured for 3 days under conditions of 5% carbon dioxide and 37°C.
(5) The cells cultured in (4) were harvested, and extraction and purification were performed using an AAVpro Purification Kit (manufactured by Takara Bio Inc.) to give VLP2 (virus-like particles, capsid protein particles of AAV serotype 2). Approximately 1 mL of VLP2 purification solution was prepared from the five T-225 flasks.

### EXAMPLE 2 Mutation Library Construction and Screening of AAV-Binding Proteins (Part 1)

Using a vector pET-AVR21 capable of expressing a polypeptide (SEQ ID NO: 2) including an AAV-binding protein AVR21 as the template, random mutagenesis was applied to the polynucleotide portion encoding the protein using error-prone PCR. It should be noted that in the polypeptide consisting of the amino acid sequence set forth in SEQ ID NO: 2, methionine (M) at position 1 to alanine (A) at position 22 form a PelB signal peptide (the 22 N-terminal amino acid residues of UniProt No. POC1C1), serine (S) at position 25 to aspartic acid (D) at position 213 form the AAV-binding protein AVR21, and histidine (H) from position 214 to position 219 form a tag sequence. AVR21, furthermore, is a polypeptide in which the following amino acid substitutions at 21 positions have occurred at the amino acid residues from position 312 to position 500 of SEQ ID NO: 1, which correspond to extracellular domains 1 (PKD1) and 2 (PKD2) of KIAA0319L (UniProt No. Q8IZA0), which is a wild-type AAV-binding protein (WO 2023/140197):
the substitution of valine (V) at position 317 of SEQ ID NO: 1 (position 30 of SEQ ID NO: 2) with aspartic acid (D);
the substitution of asparagine (N) at position 324 of SEQ ID NO: 1 (position 37 of SEQ ID NO: 2) with histidine (H) ;
the substitution of valine (V) at position 326 of SEQ ID NO: 1 (position 39 of SEQ ID NO: 2) with alanine (A);
the substitution of alanine (A) at position 330 of SEQ ID NO: 1 (position 43 of SEQ ID NO: 2) with valine (V);
the substitution of glutamine (Q) at position 334 of SEQ ID NO: 1 (position 47 of SEQ ID NO: 2) with leucine (L);
the substitution of glutamic acid (E) at position 335 of SEQ ID NO: 1 (position 48 of SEQ ID NO: 2) with valine (V) ;
the substitution of threonine (T) at position 341 of SEQ ID NO: 1 (position 54 of SEQ ID NO: 2) with alanine (A);
the substitution of tyrosine (Y) at position 342 of SEQ ID NO: 1 (position 55 of SEQ ID NO: 2) with serine (S);
the substitution of lysine (K) at position 362 of SEQ ID NO: 1 (position 75 of SEQ ID NO: 2) with glutamic acid (E);
the substitution of lysine (K) at position 371 of SEQ ID NO: 1 (position 84 of SEQ ID NO: 2) with asparagine (N);
the substitution of phenylalanine (F) at position 379 of SEQ ID NO: 1 (position 92 of SEQ ID NO: 2) with tyrosine (Y);
the substitution of lysine (K) at position 380 of SEQ ID NO: 1 (position 93 of SEQ ID NO: 2) with arginine (R);
the substitution of valine (V) at position 381 of SEQ ID NO: 1 (position 94 of SEQ ID NO: 2) with alanine (A);
the substitution of isoleucine (I) at position 382 of SEQ ID NO: 1 (position 95 of SEQ ID NO: 2) with valine (V);
the substitution of glycine (G) at position 390 of SEQ ID NO: 1 (position 103 of SEQ ID NO: 2) with serine (S);
the substitution of lysine (K) at position 399 of SEQ ID NO: 1 (position 112 of SEQ ID NO: 2) with glutamic acid (E);
the substitution of lysine (K) at position 467 of SEQ ID NO: 1 (position 180 of SEQ ID NO: 2) with glutamine (Q);
the substitution of serine (S) at position 476 of SEQ ID NO: 1 (position 189 of SEQ ID NO: 2) with arginine (R);
the substitution of serine (S) at position 482 of SEQ ID NO: 1 (position 195 of SEQ ID NO: 2) with threonine (T);
the substitution of asparagine (N) at position 487 of SEQ ID NO: 1 (position 200 of SEQ ID NO: 2) with aspartic acid (D); and
the substitution of asparagine (N) at position 492 of SEQ ID NO: 1 (position 205 of SEQ ID NO: 2) with aspartic acid (D).

(1) Error-prone PCR was performed using a vector pET-AVR21 capable of expressing a polypeptide (SEQ ID NO: 2) including AVR21 as the template. The error-prone PCR was performed by preparing a reaction solution having the composition presented in Table 1, then subjecting the reaction solution to heat treatment at 98°C for 2 minutes, carrying out 30 cycles of reaction, in which a first step at 98°C for 30 seconds, a second step at 55°C for 20 seconds and a third step at 72°C for 90 seconds constituted one cycle, and finally applying heat treatment at 72°C for 5 minutes. As a result of the error-prone PCR, mutations were successfully introduced into the polynucleotide encoding the AAV-binding protein. The average mutation introduction rate was 1.4 amino acid mutations per molecule.

**[Table 1]**

| Composition | Volume |
|---|---|
| 5 ng/µL template | 1 µL |
| 10 µM PCR primer (SEQ ID NO: 3) | 2 µL |
| 10 µM PCR primer (SEQ ID NO: 4) | 2 µL |
| 10 mM MnCl₂ | 1.5 µL |
| 2.5 mM dNTPs | 4 µL |
| 10× Ex Taq Buffer (manufactured by Takara Bio Inc.) | 5 µL |
| GoTaq polymerase (manufactured by Promega Corporation) | 0.5 µL |
| H₂O | Up to 50 µL |

(2) After purification, the PCR product obtained in (1) was digested with the restriction enzymes NcoI and XhoI. The digested product was ligated into an expression vector pET26b (manufactured by Merck millipore Corporation) that had been predigested with the same restriction enzymes.
(3) After completion of the ligation reaction, the Escherichia coli strain BL21(DE3) was transformed using the reaction solution, and the transformants were cultured on an LB (Luria-Bertani) plate medium containing 50 µg/mL kanamycin (18 hours at 37°C). Then the colonies that formed on the plate were defined as a random mutant library.
(4) The random mutant library (transformants) constructed in (3) was inoculated into 200 µL of 2YT liquid medium containing 50 µg/mL kanamycin and shake-cultured overnight at 37°C using a 96-well deep-well plate.
(5) The culture broth in (4) was centrifuged, and the resulting culture supernatant was diluted two-fold with ultrapure water. 60 µL of the diluted culture broth and 60 µL of a 0.5 M aqueous solution of sodium hydroxide were mixed, and the resulting mixture was subjected to alkali treatment for 30 minutes at 30°C.
(6) The binding activity between the AAV-binding protein and VLP2 after the treatment in (5) and the binding activity between the AAV-binding protein and VLP2 without the treatment in (5) were evaluated by ELISA (Enzyme-Linked Immuno Sorbent Assay) as described below.
(6-1) The VLP2 prepared in Example 1 was diluted 200-fold with a 20 mM Tris hydrochloride buffer (pH 7.4) containing 150 mM sodium chloride. 100 µL/well of the resulting solution was added to a 96-well microplate (manufactured by Thermo Fisher Scientific Inc.), and immobilization was performed (18 hours at 4°C). After completion of immobilization, blocking was performed using a 20 mM Tris hydrochloride buffer (pH 7.4) containing 2% (w/v) SKIM MILK (manufactured by Becton, Dickinson and Company) and 150 mM sodium chloride.
(6-2) The wells were washed with a washing buffer (a 20 mM Tris hydrochloride buffer (pH 7.4) containing 0.05% (w/v) Tween 20 (manufactured by Sigma-Aldrich Co. LLC) and 150 mM sodium chloride). Then the culture supernatant prepared in (3) was added, and the AAV-binding protein and VLP2 were allowed to react (1 hour at 30°C).
(6-3) After completion of the reaction, the wells were washed with the above-mentioned washing buffer, and 100 µL/well of Anti-6 His Antibody (manufactured by Bethyl Laboratories, Inc.), diluted to 100 ng/mL, was added.
(6-4) After the wells were allowed to react for 1 hour at 30°C and washed with the above-mentioned washing buffer, 50 µL/well of TMB Peroxidase Substrate (manufactured by KPL, Inc.) was added. Color development was terminated by adding 50 µL/well of 1 M phosphoric acid, and the absorbance at 450 nm was measured using a microplate reader (manufactured by Tecan Trading AG).
The remaining activity was calculated by dividing the binding activity between the AAV-binding protein and VLP2 after alkali treatment by the binding activity between the AAV-binding protein and VLP2 without alkali treatment.
(7) A random mutant library of approximately 1800 strains was evaluated by the method in (6), and transformants expressing AAV-binding proteins with improved remaining activity compared with AVR21, the parent molecule, were selected therefrom. The selected transformants were cultured, and expression vectors were prepared using a QIAprep Spin Miniprep kit (manufactured by QIAGEN N.V.).
(8) For the sequence of the AAV-binding protein-encoding polynucleotide region inserted in the resulting expression vectors, the positions of amino acid substitutions were identified by analyzing the nucleotide sequence using Genetic Analyzer 3500 (manufactured by Thermo Fisher Scientific Inc.), a fully automatic DNA sequencer. It should be noted that in this analysis, oligonucleotides consisting of the sequence set forth in SEQ ID NO: 3 (5'-TAATACgACTCACTATAggg-3') or SEQ ID NO: 4 (5'-ATgCTAgTTATTgCTCAgCgg-3') were used as sequencing primers.

For the AAV-binding proteins expressed by the transformants selected in (7) above, a summary of the positions of amino acid substitutions with respect to AVR21 and the remaining activity [%] after alkali treatment is presented in Table 2.

It can be concluded that AAV-binding proteins in which at least one of any amino acid substitution from among I319N (this notation indicates that isoleucine at position 319 of SEQ ID NO: 1 (position 32 of SEQ ID NO: 2) has been substituted with asparagine; the same applies hereinafter), L321Q, L328P, N329K, N329S, V332A, E(V)335A (this notation denotes that glutamic acid at position 335 of SEQ ID NO: 1 (position 48 of SEQ ID NO: 2) was once substituted with valine in SEQ ID NO: 2 and then substituted with alanine; the same applies hereinafter), K338R, E340G, T343A, T343S, Y344F, D345G, Q347K, T350A, E360D, Q365R, I366F, L367P, K368R, L369S, K(N)371D, K(N)371S, L376P, Y377F, Y377N, E378G, F(Y)379S, V(A)381T, I(V)382I, E384D, H389R, V394A, V394I, V396E, T397A, T397S, E401G, P402S, V412A, T426A, D435A, E453D, E454D, A461P, S466T, N472K, K497E and D500N had occurred at the amino acid residues from serine (S) at position 25 to aspartic acid (D) at position 213 of the amino acid sequence set forth in SEQ ID NO: 2 exhibited improved stability against alkalis compared with AVR21.

**[Table 2]**

| Amino acid substitutions | Remaining activity [%] |
|---|---|
| 1319N, Y377F | 45.1 |
| L321Q | 34.2 |
| L321Q, T343S | 50.4 |
| L328P, V394A | 40.5 |
| N329K, E401G | 43.1 |
| N329S, K368R, V412A | 37.1 |
| V332A | 57.6 |
| E(V)335A | 35.8 |
| E(V)335A, V3941 | 37.3 |
| K338R, A461P | 36.4 |
| E340G | 28.1 |
| T343A, K(N)371D | 40.5 |
| Y344F, T350A, Y377N | 34.4 |
| D345G, E454D | 61.7 |
| Q347K, N472K | 58.9 |
| E360D, H389R | 46.7 |
| Q365R, V(A)381T, K497E | 42.0 |
| 1366F, D435A | 38.4 |
| L367P | 35.7 |
| L367P, V412A | 35.8 |
| L369S | 51.4 |
| K(N)371S, E453D, S466T | 57.3 |
| L376P | 42.6 |
| L376P, P402S | 35.1 |
| E378G | 58.0 |
| F(Y)379S | 54.9 |
| I(V)382I | 32.8 |
| E384D, V396E | 45.3 |
| T397A | 40.4 |
| T397S | 38.4 |
| V412A | 32.5 |
| T426A | 43.5 |
| D500N | 39.3 |
| AVR21 | 26.2 |

### REFERENCE EXAMPLE 1

Using a vector pET-AVR8g capable of expressing a polypeptide (SEQ ID NO: 22) including an AAV-binding protein AVR8g as the template, random mutagenesis was applied to the polynucleotide portion encoding the protein using error-prone PCR. It should be noted that in the polypeptide consisting of the amino acid sequence set forth in SEQ ID NO: 22, methionine (M) at position 1 to alanine (A) at position 22 form a PelB signal peptide, serine (S) at position 25 to aspartic acid (D) at position 213 form the AAV-binding protein AVR8g, and histidine (H) from position 214 to position 219 form a tag sequence. AVR8g, furthermore, is a polypeptide in which the amino acid substitutions V317D, Y342C, K362E, K371N, G390S, K399E, S476R and N487D have occurred at the amino acid residues from position 312 to position 500 of SEQ ID NO: 1, which correspond to extracellular domains 1 (PKD1) and 2 (PKD2) of KIAA0319L (UniProt No. Q8IZA0) (WO 2021/106882).

(1) Error-prone PCR was performed by the same method as in Example 2 (1) except that a plasmid pET-AVR8g into which a polynucleotide (SEQ ID NO: 30) encoding AVR8g (SEQ ID NO: 29) was inserted was used as the template, and that the concentration of this template was changed to 10 ng/µL. As a result of the error-prone PCR, mutations were introduced into the polynucleotide encoding AVR8g, and the average mutation introduction rate was 2.3 amino acid mutations per molecule.
(2) After purification of the PCR product obtained in (1), a random mutant library was constructed by the method described in Example 2 (2) and (3).
(3) The random mutant library (transformants) constructed in (2) was inoculated into 200 µL of 2YT liquid medium containing 50 µg/mL kanamycin and shake-cultured overnight at 37°C using a 96-well deep-well plate.
(4) 10 µL of the culture broth prepared in (3) was transferred into 500 µL of 2YT liquid medium containing 0.1 mM IPTG (IsoPropyl-β-D-ThioGalactopyranoside) and 50 µg/mL kanamycin, and the cells were further shake-cultured overnight at 25°C using a 96-well deep-well plate.
(5) Culture supernatant obtained by centrifuging the culture broth in (4) was diluted 16-fold with ultrapure water. 60 µL of the diluted culture supernatant and 60 µL of a 0.1 M glycine sodium hydroxide buffer (pH 10.0) were mixed, and the resulting mixture was subjected to heat and alkali treatment for 15 minutes at 51.3°C.
(6) The binding activity between the AAV-binding protein and VLP2 after the treatment in (5)
   and the binding activity between the AAV-binding protein and VLP2 without the treatment in (5) were measured by ELISA as described in Example 2 (6).

The remaining activity was calculated by dividing the binding activity between the AAV-binding protein and VLP2 after heat treatment by the binding activity between the AAV-binding protein and VLP2 without heat treatment.

(7) A random mutant library of approximately 1800 strains was evaluated by the method in (6), and a transformant expressing an AAV-binding protein with improved remaining activity compared with AVR8g, the parent molecule, was selected therefrom. The selected transformant was cultured, and an expression vector was prepared using a QIAprep Spin Miniprep kit (manufactured by QIAGEN N.V.).
(6) The sequence of the AAV-binding protein-encoding polynucleotide region inserted in the resulting expression vector was analyzed by the method described in Example 2 (8), whereby the positions of amino acid substitutions were identified.
(7) For the AAV-binding protein expressed by the transformant selected in (6) above, a summary of the positions of amino acid substitutions with respect to AVR8g and the remaining activity [%] after heat and alkali treatment is presented in Table 3.

**[Table 3]**

| Amino acid substitutions | Remaining activity [%] |
|---|---|
| Y331H, F379S, K467N, A491T | 28.1 |
| AVR8g | 8.8 |

### REFERENCE EXAMPLE 2

It was examined whether the introduction of any one of the amino acid substitutions found to be responsible for improved heat and alkaline stability of an AAV-binding protein in Reference Example 1 (Y331H, F379S, K467N and A491T) into AVR8g (SEQ ID NO: 22) improves heat and alkaline stability. Specifically, the heat and alkaline stability of the four AAV-binding proteins specified in (a) to (d) below were examined.
(a) A protein in which the amino acid substitution Y331H has been introduced into AVR8g (SEQ ID NO: 24; designated as AVR9a)
(b) A protein in which the amino acid substitution F379S has been introduced into AVR8g (SEQ ID NO: 25; designated as AVR9b)
(c) A protein in which the amino acid substitution K467N has been introduced into AVR8g (SEQ ID NO: 26; designated as AVR9c)
(d) A protein in which the amino acid substitution A491T has been introduced into AVR8g (SEQ ID NO: 27; designated as AVR9d)
   (1) Transformants (host: the Escherichia coli strain BL21(DE3)) capable of expressing any of AVR9a (SEQ ID NO: 24), AVR9b (SEQ ID NO: 25), AVR9c (SEQ ID NO: 26) or AVR9d (SEQ ID NO: 27) were each inoculated into 3 mL of 2× YT liquid medium containing 50 µg/mL kanamycin, and preculture was performed by aerobic shake culture overnight at 37°C.
   (2) Into 200 mL of 2× YT liquid medium supplemented with 50 µg/mL kanamycin in a 500-mL baffled flask, 2 mL per transformant of the preculture broth in (1) was inoculated. Then aerobic shake culture was performed at 37°C.
   (3) 2.0 hours after the start of culturing, the cultures were cooled on ice, and IPTG was added to each to a final concentration of 0.1 mM. Then aerobic shake culture was continued for 20 hours at 25°C.
   (4) After completion of culturing, each population of bacterial cells was harvested by centrifuging the culture broth at 8000 rpm for 20 minutes at 4°C.
   (5) The bacterial cells harvested in (4) were suspended in a 20 mM Tris hydrochloride buffer (pH 7.4) containing 150 mM sodium chloride and 20 mM imidazole (hereinafter also referred to as "equilibrating solution A") to 5 mL/1 g (bacterial cells). Then the bacterial cells were disrupted at a power of approximately 150 W for approximately 10 minutes at 4°C using an ultrasonic generator (INSONATOR 201M [manufactured by KUBOTA Corporation]). The cell homogenates were centrifuged twice at 8000 rpm for 20 minutes at 4°C, and the supernatant of each was collected.
   (6) The supernatant obtained in (5) was applied to an open column packed with 1.5 mL of Ni Sepharose 6 Fast Flow (manufactured by Cytiva) pre-equilibrated with equilibrating solution A. After washing with equilibrating solution A, elution was performed using a 20 mM Tris hydrochloride buffer (pH 7.4) containing 0.5 M imidazole and 150 mM sodium chloride.
   (7) Each of the eluates obtained in (6) was dialyzed using a 20 mM Tris hydrochloride buffer (pH 7.4) containing 150 mM sodium chloride. In this manner, AAV-binding proteins were prepared.
   (8) The binding activity between the AAV-binding proteins prepared in (7) and the VLP2 prepared in Example 1 was measured by ELISA as described in Example 2 (6). Based on the absorbance at 450 nm, which was the results obtained in the measurement, the AAV-binding proteins obtained in (7) were diluted with purified water such that the measured values were similar.
   (9) 60 µL of the diluted AAV-binding protein solution and 60 µL of a 0.1 M glycine sodium hydroxide buffer (pH 10.0) were mixed, and the resulting mixture was subjected to heat and alkali treatment for 15 minutes at 55.7°C, 60.1°C, 66.1°C or 70°C.
   (10) The binding activity between the AAV-binding protein and VLP2 after the treatment in (9) and the binding activity between the AAV-binding protein and VLP2 without the treatment in (9) were measured by ELISA as described in Example 2 (6). The remaining activity was calculated by dividing the binding activity between the AAV-binding protein and VLP2 after the treatment in (9) by the binding activity between the AAV-binding protein and VLP2 without the heat treatment in (9).

The results are presented in Table 4. It can be concluded that AAV-binding proteins in which at least one of any amino acid substitution from among Y331H, F379S, K467N and A491T had occurred at the amino acid residues from serine (S) at position 25 to aspartic acid (D) at position 213 of the amino acid sequence of AVR8g (SEQ ID NO: 29) exhibited improved stability against heat and alkalis compared with AVR8g.

**[Table 4]**

| AAV-binding protein | | | Remaining activity [%] | | | |
|---|---|---|---|---|---|---|
| Name | Amino acid substitution(*) | SEQ ID NO | 55.7°C | 60.1°C | 66.1°C | 70°C |
| AVR9a | Y331H | 24 | 10.6 | 12.9 | 5.73 | 13.8 |
| AVR9b | F379S | 25 | 92.5 | 55.5 | 76.2 | 55.4 |
| AVR9c | K467N | 26 | 42.3 | 26.4 | 5.45 | 32.7 |
| AVR9d | A491T | 27 | 82.3 | 65.6 | 30.2 | 55.7 |
| AVR8g | - | 22 | 0.837 | 4.18 | 0 | 3.77 |

| | | | | | | |
|---|---|---|---|---|---|---|
| (*) An amino acid substitution with respect to AVR8g | | | | | | |

### EXAMPLE 3 Preparation of AVR21 Amino Acid Substitution-Integrated Mutants

N329K, K338R, E401G, T426A and A461P were selected from the amino acid substitutions found to be responsible for improved alkaline stability of an AAV-binding protein in Example 2, and K467N was selected from the amino acid substitutions found to be responsible for improved heat and alkaline stability of an AAV-binding protein in Reference Examples 1 and 2. These amino acid substitutions were integrated into AVR21 (SEQ ID NO: 2) in an attempt to further improve alkaline stability. Specifically, the five AAV-binding proteins specified in (a) to (e) below were designed and prepared.
(a) An AAV-binding protein in which the amino acid substitutions N329K, E401G and T426A have been introduced into AVR21 (SEQ ID NO: 5; designated as AVR24a)
(b) An AAV-binding protein in which the amino acid substitutions K338R, T426A and A461P have been introduced into AVR21 (SEQ ID NO: 6; designated as AVR24b)
(c) An AAV-binding protein in which the amino acid substitutions N329K, E401G, T426A and A461P have been introduced into AVR21 (SEQ ID NO: 7; designated as AVR25a)
(d) An AAV-binding protein in which the amino acid substitutions K338R, E401G, T426A and A461P have been introduced into AVR21 (SEQ ID NO: 8; designated as AVR25b)
(e) An AAV-binding protein in which the amino acid substitutions N329K, K338R, E401G, T426A and A461P have been introduced into AVR21 (SEQ ID NO: 9; designated as AVR26)

In the following, the methods for preparing the six AAV-binding proteins specified in (a) to (e) above will be described.

A PCR primer set for creating the mutation-integrated mutants was designed. Specifically,
SEQ ID NOs: 10 (Forward) and 11 (Reverse) as PCR primers for introducing the amino acid substitution N329K,
SEQ ID NOs: 12 (Forward) and 13 (Reverse) as PCR primers for introducing the amino acid substitution K338R,
SEQ ID NOs: 14 (Forward) and 15 (Reverse) as PCR primers for introducing the amino acid substitution E401G,
SEQ ID NOs: 16 (Forward) and 17 (Reverse) as PCR primers for introducing the amino acid substitution T426A and
SEQ ID NOs: 18 (Forward) and 19 (Reverse) as PCR primers for introducing the amino acid substitution A461P
were each designed.

### (a) AVR24a

This protein was prepared by selecting N329K, E401G and T426A from among the amino acid substitutions found to be responsible for alkaline stability in Example 2 and introducing these amino acid substitutions into AVR21 (SEQ ID NO: 2).

(a-1) A vector pET-AVR21 capable of expressing a polypeptide (SEQ ID NO: 2) including AVR21 was used as the template, and oligonucleotides consisting of the sequences set forth in SEQ ID NO: 10 (5'-AGCTGAAAGTCTACGTACTGCTGGT-3') and SEQ ID NO: 11 (5'-TAGACTTTCAGCTGGGCTTCATGTT-3') were used as the PCR primers. PCR was performed by preparing a reaction solution having the composition presented in Table 5, then subjecting the reaction solution to heat treatment at 98°C for 5 minutes, carrying out 30 cycles of reaction, in which a first step at 98°C for 10 seconds, a second step at 55°C for 5 seconds and a third step at 72°C for 6 minutes constituted one cycle, and finally applying heat treatment at 72°C for 5 minutes.

**[Table 5]**

| Composition | Volume |
|---|---|
| 10 ng/µL template | 1 µL |
| 10 µM PCR forward primer (SEQ ID NOs: 10, 12, 14, 16, 18 or 29) | 1.5 µL |
| 10 µM PCR reverse primer (SEQ ID NOs: 11, 13, 15, 17, 19 or 30) | 1.5 µL |
| 10× KOD buffer (manufactured by Toyobo Co., Ltd.) | 5 µL |
| 2 mM dNTPs | 5 µL |
| 25 mM MgSO₄ | 3 µL |
| 1 U/µL KOD Plus (manufactured by Toyobo Co., Ltd.) | 1 µL |
| H₂O | Up to 50 µL |

(a-2) The PCR product obtained in (a-1) was used as the template, and oligonucleotides consisting of the sequences set forth in SEQ ID NO: 14 (5'-AGCCTGGCCCGCGCAAGAATCGTCC-3') and SEQ ID NO: 15 (5'-CGCGGGCCAGGCTCAACCGTTACGT-3') were used as the PCR primers. PCR was performed by preparing a reaction solution having the composition presented in Table 5, then subjecting the reaction solution to heat treatment at 98°C for 5 minutes, carrying out 30 cycles of reaction, in which a first step at 98°C for 10 seconds, a second step at 55°C for 5 seconds and a third step at 72°C for 6 minutes constituted one cycle, and finally applying heat treatment at 72°C for 5 minutes.
(a-3) The PCR product obtained in (a-2) was used as the template, and oligonucleotides consisting of the sequences set forth in SEQ ID NO: 16 (5'-CAAGTGCGGTGATCGACGGATCGCA-3') and SEQ ID NO: 17 (5'-ATCACCGCACTTGTAGTCGGGAGTG-3') were used as the PCR primers. PCR was performed by preparing a reaction solution having the composition presented in Table 5, then subjecting the reaction solution to heat treatment at 98°C for 5 minutes, carrying out 30 cycles of reaction, in which a first step at 98°C for 10 seconds, a second step at 55°C for 5 seconds and a third step at 72°C for 6 minutes constituted one cycle, and finally applying heat treatment at 72°C for 5 minutes.
(a-4) The Escherichia coli strain BL21(DE3) was transformed using the PCR product obtained in (a-3). The resulting transformant was cultured in an LB medium supplemented with 50 µg/mL kanamycin, and then a plasmid was extracted from bacterial cells (transformant) harvested by centrifugation. In this manner, a plasmid (expression vector) pET-AVR24a including a polynucleotide encoding AVR24a, in which amino acid substitutions at 24 positions had been introduced into the wild-type AAV-binding protein, was obtained.
(a-5) The nucleotide sequence of pET-AVR24a was analyzed and confirmed by the same method as in Example 2 (8).

The amino acid sequence of AVR24a with a signal sequence and a polyhistidine tag attached thereto is presented in SEQ ID NO: 5. It should be noted that in SEQ ID NO: 5, methionine (M) at position 1 to alanine (A) at position 22 form the PelB signal peptide, serine (S) at position 25 to aspartic acid (D) at position 213 form the AAV-binding protein AVR24a (corresponding to the region from position 25 to position 213 of SEQ ID NO: 2), and histidine (H) from position 214 to position 219 form the tag sequence. In SEQ ID NO: 5, furthermore, the aspartic acid in V317D is present at position 30, the histidine in N324H is present at position 37, the alanine in V326A is present at position 39, the lysine in N329K is present at position 42, the valine in A330V is present at position 43, the leucine in Q334L is present at position 47, the valine in E335V is present at position 48, the alanine in T341A is present at position 54, the serine in Y342S is present at position 55, the glutamic acid in K362E is present at position 75, the asparagine in K371N is present at position 84, the tyrosine in F379Y is present at position 92, the arginine in K380R is present at position 93, the alanine in V381A is present at position 94, the valine in I382V is present at position 95, the serine in G390S is present at position 103, the glutamic acid in K399E is present at position 112, the glycine in E401G is present at position 114, the alanine in T426A is present at position 139, the glutamine in K467Q is present at position 180, the arginine in S476R is present at position 189, the threonine in S482T is present at position 195, the aspartic acid in N487D is present at position 200, and the aspartic acid in N492D is present at position 205.

### (b) AVR24b

This protein was prepared by selecting K338R, T426A and A461P from among the amino acid substitutions found to be responsible for alkaline stability in Example 2 and introducing these amino acid substitutions into AVR21 (SEQ ID NO: 2).

(b-1) A vector pET-AVR21 capable of expressing a polypeptide (SEQ ID NO: 2) including AVR21 was used as the template, and oligonucleotides consisting of the sequences set forth in SEQ ID NO: 12 (5'-CACCGCGCGGGGAAGCGTCAACGTA-3') and SEQ ID NO: 13 (5'-TCCCCGCGCGGTGGAACCAGCAGTA-3') were used as the PCR primers. PCR was performed by preparing a reaction solution having the composition presented in Table 5, then subjecting the reaction solution to heat treatment at 98°C for 5 minutes, carrying out 30 cycles of reaction, in which a first step at 98°C for 10 seconds, a second step at 55°C for 5 seconds and a third step at 72°C for 6 minutes constituted one cycle, and finally applying heat treatment at 72°C for 5 minutes.
(b-2) The PCR product obtained in (b-1) was used as the template, and oligonucleotides consisting of the sequences set forth in SEQ ID NO: 16 (5'-CAAGTGCGGTGATCGACGGATCGCA-3') and SEQ ID NO: 17 (5'-ATCACCGCACTTGTAGTCGGGAGTG-3') were used as the PCR primers. PCR was performed by preparing a reaction solution having the composition presented in Table 5, then subjecting the reaction solution to heat treatment at 98°C for 5 minutes, carrying out 30 cycles of reaction, in which a first step at 98°C for 10 seconds, a second step at 55°C for 5 seconds and a third step at 72°C for 6 minutes constituted one cycle, and finally applying heat treatment at 72°C for 5 minutes.
(b-3) The PCR product obtained in (b-2) was used as the template, and oligonucleotides consisting of the sequences set forth in SEQ ID NO: 18 (5'-ATACCCCGATTCTTAAGCTCTCGCA-3') and SEQ ID NO: 19 (5'-AGAATCGGGGTATCCTCGCTGATCT-3') were used as the PCR primers. PCR was performed by preparing a reaction solution having the composition presented in Table 5, then subjecting the reaction solution to heat treatment at 98°C for 5 minutes, carrying out 30 cycles of reaction, in which a first step at 98°C for 10 seconds, a second step at 55°C for 5 seconds and a third step at 72°C for 6 minutes constituted one cycle, and finally applying heat treatment at 72°C for 5 minutes.
(b-4) The Escherichia coli strain BL21(DE3) was transformed using the PCR product obtained in (b-3). The resulting transformant was cultured in an LB medium supplemented with 50 µg/mL kanamycin, and then a plasmid was extracted from bacterial cells (transformant) harvested by centrifugation. In this manner, a plasmid (expression vector) pET-AVR24b including a polynucleotide encoding AVR24b, in which amino acid substitutions at 24 positions had been introduced into the wild-type AAV-binding protein, was obtained.
(b-5) The nucleotide sequence of pET-AVR24b was analyzed and confirmed by the same method as in Example 2 (8).

The amino acid sequence of AVR24b with a signal sequence and a polyhistidine tag attached thereto is presented in SEQ ID NO: 6. It should be noted that in SEQ ID NO: 6, methionine (M) at position 1 to alanine (A) at position 22 form the PelB signal peptide, serine (S) at position 25 to aspartic acid (D) at position 213 form the AAV-binding protein AVR24b (corresponding to the region from position 25 to position 213 of SEQ ID NO: 2), and histidine (H) from position 214 to position 219 form the tag sequence. In SEQ ID NO: 6, furthermore, the aspartic acid in V317D is present at position 30, the histidine in N324H is present at position 37, the alanine in V326A is present at position 39, the valine in A330V is present at position 43, the leucine in Q334L is present at position 47, the valine in E335V is present at position 48, the arginine in K338R is present at position 51, the alanine in T341A is present at position 54, the serine in Y342S is present at position 55, the glutamic acid in K362E is present at position 75, the asparagine in K371N is present at position 84, the tyrosine in F379Y is present at position 92, the arginine in K380R is present at position 93, the alanine in V381A is present at position 94, the valine in I382V is present at position 95, the serine in G390S is present at position 103, the glutamic acid in K399E is present at position 112, the alanine in T426A is present at position 139, the proline in A461P is present at position 174, the glutamine in K467Q is present at position 180, the arginine in S476R is present at position 189, the threonine in S482T is present at position 195, the aspartic acid in N487D is present at position 200, and the aspartic acid in N492D is present at position 205.

### (c) AVR25a

This protein was prepared by selecting N329K, E401G, T426A and A461P from among the amino acid substitutions found to be responsible for alkaline stability in Example 2 and introducing these amino acid substitutions into AVR21 (SEQ ID NO: 2).

(c-1) A vector pET-AVR21 capable of expressing a polypeptide (SEQ ID NO: 2) including AVR21 was used as the template, and oligonucleotides consisting of the sequences set forth in SEQ ID NO: 10 (5'-AGCTGAAAGTCTACGTACTGCTGGT-3') and SEQ ID NO: 11 (5'-TAGACTTTCAGCTGGGCTTCATGTT-3') were used as the PCR primers. PCR was performed by preparing a reaction solution having the composition presented in Table 5, then subjecting the reaction solution to heat treatment at 98°C for 5 minutes, carrying out 30 cycles of reaction, in which a first step at 98°C for 10 seconds, a second step at 55°C for 5 seconds and a third step at 72°C for 6 minutes constituted one cycle, and finally applying heat treatment at 72°C for 5 minutes.
(c-2) The PCR product obtained in (c-1) was used as the template, and oligonucleotides consisting of the sequences set forth in SEQ ID NO: 14 (5'-AGCCTGGCCCGCGCAAGAATCGTCC-3') and SEQ ID NO: 15 (5'-CGCGGGCCAGGCTCAACCGTTACGT-3') were used as the PCR primers. PCR was performed by preparing a reaction solution having the composition presented in Table 5, then subjecting the reaction solution to heat treatment at 98°C for 5 minutes, carrying out 30 cycles of reaction, in which a first step at 98°C for 10 seconds, a second step at 55°C for 5 seconds and a third step at 72°C for 6 minutes constituted one cycle, and finally applying heat treatment at 72°C for 5 minutes.
(c-3) The PCR product obtained in (c-2) was used as the template, and oligonucleotides consisting of the sequences set forth in SEQ ID NO: 16 (5'-CAAGTGCGGTGATCGACGGATCGCA-3') and SEQ ID NO: 17 (5'-ATCACCGCACTTGTAGTCGGGAGTG-3') were used as the PCR primers. PCR was performed by preparing a reaction solution having the composition presented in Table 5, then subjecting the reaction solution to heat treatment at 98°C for 5 minutes, carrying out 30 cycles of reaction, in which a first step at 98°C for 10 seconds, a second step at 55°C for 5 seconds and a third step at 72°C for 6 minutes constituted one cycle, and finally applying heat treatment at 72°C for 5 minutes.
(c-4) The PCR product obtained in (c-3) was used as the template, and oligonucleotides consisting of the sequences set forth in SEQ ID NO: 18 (5'-ATACCCCGATTCTTAAGCTCTCGCA-3') and SEQ ID NO: 19 (5'-AGAATCGGGGTATCCTCGCTGATCT-3') were used as the PCR primers. PCR was performed by preparing a reaction solution having the composition presented in Table 5, then subjecting the reaction solution to heat treatment at 98°C for 5 minutes, carrying out 30 cycles of reaction, in which a first step at 98°C for 10 seconds, a second step at 55°C for 5 seconds and a third step at 72°C for 6 minutes constituted one cycle, and finally applying heat treatment at 72°C for 5 minutes.
(c-5) The Escherichia coli strain BL21(DE3) was transformed using the PCR product obtained in (c-4). The resulting transformant was cultured in an LB medium supplemented with 50 µg/mL kanamycin, and then a plasmid was extracted from bacterial cells (transformant) harvested by centrifugation. In this manner, a plasmid (expression vector) pET-AVR25a including a polynucleotide encoding AVR25a, in which amino acid substitutions at 25 positions had been introduced into the wild-type AAV-binding protein, was obtained.
(c-6) The nucleotide sequence of pET-AVR25a was analyzed and confirmed by the same method as in Example 2 (8).

The amino acid sequence of AVR25a with a signal sequence and a polyhistidine tag attached thereto is presented in SEQ ID NO: 7. It should be noted that in SEQ ID NO: 7, methionine (M) at position 1 to alanine (A) at position 22 form the PelB signal peptide, serine (S) at position 25 to aspartic acid (D) at position 213 form the AAV-binding protein AVR25a (corresponding to the region from position 25 to position 213 of SEQ ID NO: 2), and histidine (H) from position 214 to position 219 form the tag sequence. In SEQ ID NO: 7, furthermore, the aspartic acid in V317D is present at position 30, the histidine in N324H is present at position 37, the alanine in V326A is present at position 39, the lysine in N329K is present at position 42, the valine in A330V is present at position 43, the leucine in Q334L is present at position 47, the valine in E335V is present at position 48, the alanine in T341A is present at position 54, the serine in Y342S is present at position 55, the glutamic acid in K362E is present at position 75, the asparagine in K371N is present at position 84, the tyrosine in F379Y is present at position 92, the arginine in K380R is present at position 93, the alanine in V381A is present at position 94, the valine in I382V is present at position 95, the serine in G390S is present at position 103, the glutamic acid in K399E is present at position 112, the glycine in E401G is present at position 114, the alanine in T426A is present at position 139, the proline in A461P is present at position 174, the glutamine in K467Q is present at position 180, the arginine in S476R is present at position 189, the threonine in S482T is present at position 195, the aspartic acid in N487D is present at position 200, and the aspartic acid in N492D is present at position 205.

### (d) AVR25b

This protein was prepared by selecting K338R, E401G, T426A and A461P from among the amino acid substitutions found to be responsible for alkaline stability in Example 2 and introducing these amino acid substitutions into AVR21 (SEQ ID NO: 2).

(d-1) A vector pET-AVR21 capable of expressing a polypeptide (SEQ ID NO: 2) including AVR21 was used as the template, and oligonucleotides consisting of the sequences set forth in SEQ ID NO: 12 (5'-CACCGCGCGGGGAAGCGTCAACGTA-3') and SEQ ID NO: 13 (5'-TCCCCGCGCGGTGGAACCAGCAGTA-3') were used as the PCR primers. PCR was performed by preparing a reaction solution having the composition presented in Table 5, then subjecting the reaction solution to heat treatment at 98°C for 5 minutes, carrying out 30 cycles of reaction, in which a first step at 98°C for 10 seconds, a second step at 55°C for 5 seconds and a third step at 72°C for 6 minutes constituted one cycle, and finally applying heat treatment at 72°C for 5 minutes.
(d-2) The PCR product obtained in (d-1) was used as the template, and oligonucleotides consisting of the sequences set forth in SEQ ID NO: 14 (5'-AGCCTGGCCCGCGCAAGAATCGTCC-3') and SEQ ID NO: 15 (5'-CGCGGGCCAGGCTCAACCGTTACGT-3') were used as the PCR primers. PCR was performed by preparing a reaction solution having the composition presented in Table 5, then subjecting the reaction solution to heat treatment at 98°C for 5 minutes, carrying out 30 cycles of reaction, in which a first step at 98°C for 10 seconds, a second step at 55°C for 5 seconds and a third step at 72°C for 6 minutes constituted one cycle, and finally applying heat treatment at 72°C for 5 minutes.
(d-3) The PCR product obtained in (d-2) was used as the template, and oligonucleotides consisting of the sequences set forth in SEQ ID NO: 16 (5'-CAAGTGCGGTGATCGACGGATCGCA-3') and SEQ ID NO: 17 (5'-ATCACCGCACTTGTAGTCGGGAGTG-3') were used as the PCR primers. PCR was performed by preparing a reaction solution having the composition presented in Table 5, then subjecting the reaction solution to heat treatment at 98°C for 5 minutes, carrying out 30 cycles of reaction, in which a first step at 98°C for 10 seconds, a second step at 55°C for 5 seconds and a third step at 72°C for 6 minutes constituted one cycle, and finally applying heat treatment at 72°C for 5 minutes.
(d-4) The PCR product obtained in (d-3) was used as the template, and oligonucleotides consisting of the sequences set forth in SEQ ID NO: 18 (5'-ATACCCCGATTCTTAAGCTCTCGCA-3') and SEQ ID NO: 19 (5'-AGAATCGGGGTATCCTCGCTGATCT-3') were used as the PCR primers. PCR was performed by preparing a reaction solution having the composition presented in Table 5, then subjecting the reaction solution to heat treatment at 98°C for 5 minutes, carrying out 30 cycles of reaction, in which a first step at 98°C for 10 seconds, a second step at 55°C for 5 seconds and a third step at 72°C for 6 minutes constituted one cycle, and finally applying heat treatment at 72°C for 5 minutes.
(d-5) The Escherichia coli strain BL21(DE3) was transformed using the PCR product obtained in (d-4). The resulting transformant was cultured in an LB medium supplemented with 50 µg/mL kanamycin, and then a plasmid was extracted from bacterial cells (transformant) harvested by centrifugation. In this manner, a plasmid (expression vector) pET-AVR25b including a polynucleotide encoding AVR25b, in which amino acid substitutions at 25 positions had been introduced into the wild-type AAV-binding protein, was obtained.
(d-6) The nucleotide sequence of pET-AVR25b was analyzed and confirmed by the same method as in Example 2 (8).

The amino acid sequence of AVR25b with a signal sequence and a polyhistidine tag attached thereto is presented in SEQ ID NO: 8. It should be noted that in SEQ ID NO: 8, methionine (M) at position 1 to alanine (A) at position 22 form the PelB signal peptide, serine (S) at position 25 to aspartic acid (D) at position 213 form the AAV-binding protein AVR25b (corresponding to the region from position 25 to position 213 of SEQ ID NO: 2), and histidine (H) from position 214 to position 219 form the tag sequence. In SEQ ID NO: 8, furthermore, the aspartic acid in V317D is present at position 30, the histidine in N324H is present at position 37, the alanine in V326A is present at position 39, the valine in A330V is present at position 43, the leucine in Q334L is present at position 47, the valine in E335V is present at position 48, the arginine in K338R is present at position 51, the alanine in T341A is present at position 54, the serine in Y342S is present at position 55, the glutamic acid in K362E is present at position 75, the asparagine in K371N is present at position 84, the tyrosine in F379Y is present at position 92, the arginine in K380R is present at position 93, the alanine in V381A is present at position 94, the valine in I382V is present at position 95, the serine in G390S is present at position 103, the glutamic acid in K399E is present at position 112, the glycine in E401G is present at position 114, the alanine in T426A is present at position 139, the proline in A461P is present at position 174, the glutamine in K467Q is present at position 180, the arginine in S476R is present at position 189, the threonine in S482T is present at position 195, the aspartic acid in N487D is present at position 200, and the aspartic acid in N492D is present at position 205.

### (e) AVR26

This protein was prepared by selecting N329K, K338R, E401G, T426A and A461P from among the amino acid substitutions found to be responsible for alkaline stability in Example 2 and introducing these amino acid substitutions into AVR21 (SEQ ID NO: 2).

(e-1) A vector pET-AVR21 capable of expressing a polypeptide (SEQ ID NO: 2) including AVR21 was used as the template, and oligonucleotides consisting of the sequences set forth in SEQ ID NO: 10 (5'-AGCTGAAAGTCTACGTACTGCTGGT-3') and SEQ ID NO: 11 (5'-TAGACTTTCAGCTGGGCTTCATGTT-3') were used as the PCR primers. PCR was performed by preparing a reaction solution having the composition presented in Table 5, then subjecting the reaction solution to heat treatment at 98°C for 5 minutes, carrying out 30 cycles of reaction, in which a first step at 98°C for 10 seconds, a second step at 55°C for 5 seconds and a third step at 72°C for 6 minutes constituted one cycle, and finally applying heat treatment at 72°C for 5 minutes.
(e-2) The PCR product obtained in (e-1) was used as the template, and oligonucleotides consisting of the sequences set forth in SEQ ID NO: 12 (5'-CACCGCGCGGGGAAGCGTCAACGTA-3') and SEQ ID NO: 13 (5'-TCCCCGCGCGGTGGAACCAGCAGTA-3') were used as the PCR primers. PCR was performed by preparing a reaction solution having the composition presented in Table 5, then subjecting the reaction solution to heat treatment at 98°C for 5 minutes, carrying out 30 cycles of reaction, in which a first step at 98°C for 10 seconds, a second step at 55°C for 5 seconds and a third step at 72°C for 6 minutes constituted one cycle, and finally applying heat treatment at 72°C for 5 minutes.
(e-3) The PCR product obtained in (e-2) was used as the template, and oligonucleotides consisting of the sequences set forth in SEQ ID NO: 14 (5'-AGCCTGGCCCGCGCAAGAATCGTCC-3') and SEQ ID NO: 15 (5'-CGCGGGCCAGGCTCAACCGTTACGT-3') were used as the PCR primers. PCR was performed by preparing a reaction solution having the composition presented in Table 5, then subjecting the reaction solution to heat treatment at 98°C for 5 minutes, carrying out 30 cycles of reaction, in which a first step at 98°C for 10 seconds, a second step at 55°C for 5 seconds and a third step at 72°C for 6 minutes constituted one cycle, and finally applying heat treatment at 72°C for 5 minutes.
(e-4) The PCR product obtained in (e-3) was used as the template, and oligonucleotides consisting of the sequences set forth in SEQ ID NO: 16 (5'-CAAGTGCGGTGATCGACGGATCGCA-3') and SEQ ID NO: 17 (5'-ATCACCGCACTTGTAGTCGGGAGTG-3') were used as the PCR primers. PCR was performed by preparing a reaction solution having the composition presented in Table 5, then subjecting the reaction solution to heat treatment at 98°C for 5 minutes, carrying out 30 cycles of reaction, in which a first step at 98°C for 10 seconds, a second step at 55°C for 5 seconds and a third step at 72°C for 6 minutes constituted one cycle, and finally applying heat treatment at 72°C for 5 minutes.
(e-5) The PCR product obtained in (e-4) was used as the template, and oligonucleotides consisting of the sequences set forth in SEQ ID NO: 18 (5'-ATACCCCGATTCTTAAGCTCTCGCA-3') and SEQ ID NO: 19 (5'-AGAATCGGGGTATCCTCGCTGATCT-3') were used as the PCR primers. PCR was performed by preparing a reaction solution having the composition presented in Table 5, then subjecting the reaction solution to heat treatment at 98°C for 5 minutes, carrying out 30 cycles of reaction, in which a first step at 98°C for 10 seconds, a second step at 55°C for 5 seconds and a third step at 72°C for 6 minutes constituted one cycle, and finally applying heat treatment at 72°C for 5 minutes.
(e-6) The Escherichia coli strain BL21(DE3) was transformed using the PCR product obtained in (e-5). The resulting transformant was cultured in an LB medium supplemented with 50 µg/mL kanamycin, and then a plasmid was extracted from bacterial cells (transformant) harvested by centrifugation. In this manner, a plasmid (expression vector) pET-AVR26 including a polynucleotide encoding AVR26, in which amino acid substitutions at 26 positions had been introduced into the wild-type AAV-binding protein, was obtained.
(e-7) The nucleotide sequence of pET-AVR26 was analyzed and confirmed by the same method as in Example 2 (8).

The amino acid sequence of AVR26 with a signal sequence and a polyhistidine tag attached thereto is presented in SEQ ID NO: 9. It should be noted that in SEQ ID NO: 9, methionine (M) at position 1 to alanine (A) at position 22 form the PelB signal peptide, serine (S) at position 25 to aspartic acid (D) at position 213 form the AAV-binding protein AVR26 (corresponding to the region from position 25 to position 213 of SEQ ID NO: 2), and histidine (H) from position 214 to position 219 form the tag sequence. In SEQ ID NO: 9, furthermore, the aspartic acid in V317D is present at position 30, the histidine in N324H is present at position 37, the alanine in V326A is present at position 39, the lysine in N329K is present at position 42, the valine in A330V is present at position 43, the leucine in Q334L is present at position 47, the valine in E335V is present at position 48, the arginine in K338R is present at position 51, the alanine in T341A is present at position 54, the serine in Y342S is present at position 55, the glutamic acid in K362E is present at position 75, the asparagine in K371N is present at position 84, the tyrosine in F379Y is present at position 92, the arginine in K380R is present at position 93, the alanine in V381A is present at position 94, the valine in I382V is present at position 95, the serine in G390S is present at position 103, the glutamic acid in K399E is present at position 112, the glycine in E401G is present at position 114, the alanine in T426A is present at position 139, the proline in A461P is present at position 174, the glutamine in K467Q is present at position 180, the arginine in S476R is present at position 189, the threonine in S482T is present at position 195, the aspartic acid in N487D is present at position 200, and the aspartic acid in N492D is present at position 205.

### EXAMPLE 4 Evaluation of Alkaline Stability of the AVR21 Amino Acid Substitution-Integrated Mutants

(1) As transformants capable of expressing AAV-binding proteins, transformants obtained by transforming the Escherichia coli strain BL21(DE3) with plasmids including a polynucleotide encoding any of the six AAV-binding proteins obtained in Example 3 (AVR21 amino acid substitution-integrated mutants, or, specifically, AVR24a [SEQ ID NO: 5], AVR24b [SEQ ID NO: 6], AVR25a [SEQ ID NO: 7], AVR25b [SEQ ID NO: 8] and AVR26 [SEQ ID NO: 9] and AVR21 (SEQ ID NO: 2) were each inoculated into 3 mL of 2× YT liquid medium containing 50 µg/mL kanamycin. Then preculture was performed by aerobic shake culture overnight at 37°C.
(2) Into 200 mL of 2× YT liquid medium supplemented with 50 µg/mL kanamycin in a 1-L baffled flask, 2 mL per transformant of the preculture broth in (1) was inoculated. Then aerobic shake culture was performed at 37°C.
(3) 2.0 hours after the start of culturing, the cultures were cooled on ice, and IPTG was added to each to a final concentration of 0.1 mM. Then aerobic shake culture was continued for 20 hours at 25°C.
(4) After completion of culturing, each population of cultured bacterial cells (transformant) was harvested by centrifuging the culture broth at 8000 rpm for 20 minutes at 4°C.
(5) The bacterial cells harvested in (4) were suspended in a 20 mM Tris hydrochloride buffer (pH 7.4) containing 150 mM sodium chloride and 20 mM imidazole (hereinafter also referred to as "equilibrating solution A") to 5 mL/1 g (bacterial cells). Then the bacterial cells were disrupted at a power of approximately 150 W for approximately 10 minutes at 8°C using an ultrasonic generator (INSONATOR 201M (manufactured by KUBOTA Corporation)). The cell homogenates were centrifuged twice at 8000 rpm for 20 minutes at 4°C, and the supernatant of each was collected.
(6) The supernatant obtained in (5) was applied to an open column packed with 1.5 mL of Ni Sepharose 6 Fast Flow (manufactured by Cytiva) pre-equilibrated with equilibrating solution A. After washing with equilibrating solution A, elution was performed using a 20 mM Tris hydrochloride buffer (pH 7.4) containing 0.5 M imidazole and 150 mM sodium chloride.
(7) Each of the eluates obtained in (6) was dialyzed using a 20 mM Tris hydrochloride buffer (pH 7.4) containing 150 mM sodium chloride. In this manner, AAV-binding proteins were prepared.
(8) The binding activity between the AAV-binding proteins prepared in (7) and the VLP2 prepared in Example 1 was measured by ELISA as described in Example 2 (6). Based on the absorbance at 450 nm, which was the results obtained in the measurement, the AAV-binding proteins obtained in (7) were diluted with purified water such that the measured values were similar.
(9) Each of the AAV-binding protein solutions diluted in (8) was divided into two fractions. One of the fractions was subjected to alkali treatment in which it was mixed with an equal volume of a 0.5 M aqueous solution of sodium hydroxide, and the resulting mixture was allowed to stand at a constant temperature/for certain durations (treatment temperature, 30°C; treatment durations, 0, 30 and 60 minutes), whereas the other fraction was not subjected to this alkali treatment (corresponding to the "Start" of the alkali treatment).
(10) The fraction after the treatment in (9) was adjusted to a pH of approximately 6 by mixing a 0.5 M MES (2-(N-Morpholino)EthaneSulfonic acid) buffer (pH 6.0) into it at a ratio of 8:2, and then the binding activity to VLP2 was measured by ELISA as described in Example 2 (6).
(11) The remaining activity was calculated by dividing the absorbance at 450 nm after the alkali treatment in (10) by the absorbance at 450 nm at a treatment duration of 0 hours.

The results are presented in Table 6. It can be understood that the AVR21 amino acid substitution-integrated mutants obtained in Example 3 all exhibited higher remaining activity values compared with AVR21 (SEQ ID NO: 2), indicating improved stability against alkalis.

**[Table 6]**

| AAV-binding protein | | Remaining activity [%] | | |
|---|---|---|---|---|
| Name | SEQ ID NO | Start | 30 minutes | 60 minutes |
| AVR24a | 5 | 100 | 37.6 | 0.3 |
| AVR24b | 6 | 100 | 89.1 | 17.6 |
| AVR25a | 7 | 100 | 100 | 47.9 |
| AVR25b | 8 | 100 | 100 | 1.5 |
| AVR26 | 9 | 100 | 100 | 23.9 |
| AVR21 | 2 | 100 | 16.9 | 0 |

### EXAMPLE 5 Preparation of an AAV Vector (Part 2)

An AAV vector to be used as an AAV in the subsequent examples was prepared by the method described below.

(1) A nucleotide sequence (SEQ ID NO: 21) was designed in which a restriction enzyme EcoRI recognition sequence (GAATTC) had been attached to the 5' end of a polynucleotide encoding an EGFP (Enhanced Green Fluorescent Protein) consisting of the amino acid sequence set forth in SEQ ID NO: 20, and in which a stop codon (TAG) and a BamHI recognition sequence (GGATTC) had been attached to the 3' end.
(2) A polynucleotide consisting of the sequence set forth in SEQ ID NO: 21 was totally synthesized and cloned into a plasmid (outsourced to FASMAC Co., Ltd.; designated as pUC-EGFP). The Escherichia coli strain JM109 was transformed with pUC-EGFP, and the resulting transformant was cultured. From the culture broth, pUC-EGFP was extracted using a QIAprep Spin Miniprep kit (manufactured by QIAGEN N.V.).
(3) The pUC-EGFP obtained in (2) was digested with the restriction enzymes EcoRI and BamHI. Then the digested product was ligated into the expression vector pAAV-CMV (manufactured by Takara Bio Inc.) that had been predigested with the restriction enzymes EcoRI and BamHI, and the Escherichia coli strain JM109 was transformed using the ligation product.
(4) The transformant obtained in (3) was shake-cultured overnight at 37°C in a 5-L baffled flask containing 1 L of 2YT medium (1.6% (w/v) Tryptone, 1% (w/v) Yeast Extract and 0.5% (w/v) sodium chloride) containing 100 µg/mL carbenicillin. After completion of culturing, the bacterial cells were harvested by centrifugation, and a large amount of pAAV-EGFP was prepared from the harvested bacterial cells using a Plasmid Mega Kit (manufactured by QIAGEN N.V.).
(5) The Escherichia coli strain JM109 was transformed using a plasmid including a polynucleotide encoding capsids of cerotype 8 (AAV8) (hereinafter also referred to as "pRC8 Vector") and the pHelper Vector (manufactured by Takara Bio Inc.). By performing the same operation as in (4) using the resulting transformant, large amounts of pRCX Vector and pHelper were prepared.
(6) HEK293T cells were cultured in ten T-225 flasks (manufactured by Thermo Fisher Scientific Inc.) containing 45 mL of D-MEM medium (manufactured by FUJIFILM Wako Pure Chemical Corporation) containing 10% (v/v) bovine serum. Gene transfer was performed by adding a complex of the pAAV-EGFP prepared in (4), the pRC8 Vector and pHelper prepared in (5) and polyethyleneimine (manufactured by Polysciences, Inc.), and the cells were statically cultured for 3 days under conditions of 5% (v/v) carbon dioxide and 37°C. After culturing, cells detached by centrifugation were harvested and stored frozen at -80°C, with the cells from five T-225 flasks separated from those from the other five.
(7) The frozen cells obtained in (6) were thawed and suspended in 10 mL of a 20 mM Tris hydrochloride buffer (pH 7.4) containing 0.5 M sodium chloride, 4 mM magnesium chloride and 0.01% (w/v) Tween 20 (manufactured by Sigma-Aldrich Co. LLC). After being allowed to stand for 1 hour at 37°C with a 1/2000 volume of Benzonase (manufactured by Merck Millipore Corporation) added thereto, the mixture was centrifuged at 13000× g for 10 minutes at 4°C to yield a supernatant. Ammonium sulfate was added to the resulting supernatant to 15% saturation, and the resulting mixture was centrifuged under the same conditions again. The resulting supernatant was passed through a filter having a pore size of 0.45 µm to remove suspended matter.
(8) The supernatant from which suspended matter was removed was applied to a 7-mL POROS AAVX column (manufactured by Thermo Fisher Scientific Inc.) pre-equilibrated with a 20 mM Tris hydrochloride buffer (pH 8.0) containing 0.5 M sodium chloride (hereinafter also referred to as "equilibrating solution B").
(9) After washing with equilibrating solution B, elution was performed using a 0.1 M acetate buffer (pH 2.5) containing 0.5 M sodium chloride. The resulting eluate was neutralized by adding a 1/4 volume of a 1 M Tris hydrochloride buffer (pH 8.5) containing 20 mM magnesium chloride. In this manner, a solution of AAV8-EGFP, an AAV vector, was obtained.
(10) The concentration of AAV8-EGFP in the solution obtained in (9) was quantified by qPCR using an AAVpro Titration Kit (manufactured by Takara Bio Inc.). The solution, furthermore, was subjected to SDS-PAGE and silver-stained using a Pierce Silver Stain Kit (manufactured by Thermo Fisher Scientific Inc.), whereby the purity of the AAV vector contained in the solution was confirmed.

### EXAMPLE 6 Introduction of an Amino Acid Substitution to AAV-Binding Protein AVR25a

From among the AVR21 amino acid substitution-integrated mutants evaluated for alkaline stability in Example 4, AVR25a (SEQ ID NO: 7) was selected. An AVR21 amino acid substitution-integrated mutant in which the amino acid substitution K467N, found to be an amino acid substitution that improves heat and alkaline stability in Reference Examples 1 and 2, had been introduced into this AVR25a was prepared (designated as AVR25c). In addition, SEQ ID NOs: 29 (Forward) and 30 (Reverse) were each designed as a PCR primer for introducing the amino acid substitution K467N.

(1) PCR was performed by the same method as in Example 3 (a-1) except that a vector pET-AVR25a capable of expressing a polypeptide (SEQ ID NO: 7) including AVR25a was used as the template, and that oligonucleotides consisting of the sequences set forth in SEQ ID NO: 29 (5'-GAGAGCTTAAGAATGGGGGTATCCTC-3') and SEQ ID NO: 30 (5'-TTCTTAAGCTCTCGAATCTGGTACC-3') were used as the PCR primers.
(2) From the PCR product obtained in (1), a plasmid (expression vector) pET-AVR25c including a polynucleotide encoding AVR25c, in which amino acid substitutions at 25 positions had been introduced into the wild-type AAV-binding protein, was obtained by the same method as in Example 3 (a-4).
(3) The nucleotide sequence of pET-AVR25c was analyzed and confirmed by the same method as in Example 2 (8).

The amino acid sequence of AVR25c with a signal sequence and a polyhistidine tag attached thereto is presented in SEQ ID NO: 28. It should be noted that in SEQ ID NO: 28, methionine (M) at position 1 to alanine (A) at position 22 form the PelB signal peptide, serine (S) at position 25 to aspartic acid (D) at position 213 form the AAV-binding protein AVR25c (corresponding to the region from position 25 to position 213 of SEQ ID NO: 2), and histidine (H) from position 214 to position 219 form the tag sequence. In SEQ ID NO: 28, furthermore, the aspartic acid in V317D is present at position 30, the histidine in N324H is present at position 37, the alanine in V326A is present at position 39, the lysine in N329K is present at position 42, the valine in A330V is present at position 43, the leucine in Q334L is present at position 47, the valine in E335V is present at position 48, the alanine in T341A is present at position 54, the serine in Y342S is present at position 55, the glutamic acid in K362E is present at position 75, the asparagine in K371N is present at position 84, the tyrosine in F379Y is present at position 92, the arginine in K380R is present at position 93, the alanine in V381A is present at position 94, the valine in I382V is present at position 95, the serine in G390S is present at position 103, the glutamic acid in K399E is present at position 112, the glycine in E401G is present at position 114, the alanine in T426A is present at position 139, the proline in A461P is present at position 174, the asparagine in K467N is present at position 180, the arginine in S476R is present at position 189, the threonine in S482T is present at position 195, the aspartic acid in N487D is present at position 200, and the aspartic acid in N492D is present at position 205.

### EXAMPLE 7 Evaluation of Alkaline Stability of AAV-Binding Protein AVR25c

Transformants obtained by transforming the Escherichia coli strain BL21(DE3) with a plasmid including a polynucleotide (SEQ ID NO: 28) encoding AVR25c, obtained in Example 6, or a plasmid encoding the AAV-binding protein AVR25a (SEQ ID NO: 7), obtained in Example 3 (c), were each cultured by the same method as in Example 4 (1) to (3). Then the cultured bacterial cells were harvested by the method described in Example 4 (4), AAV-binding proteins were prepared by the method described in Example 4 (5) to (8), and alkaline stability was evaluated by the method described in Example 4 (9) to (11).

The results are presented in Table 7. It can be understood that AVR25c (SEQ ID NO: 28), into which the amino acid substitution K467N had been introduced, exhibited higher remaining activity compared with AVR25a (SEQ ID NO: 7), which lacked this amino acid substitution, indicating improved stability against alkalis.

**[Table 7]**

| AAV-binding protein | | Remaining activity [%] | | |
|---|---|---|---|---|
| Name | SEQ ID NO | Start | 30 minutes | 90 minutes |
| AVR25c | 28 | 100 | 91.2 | 66.9 |
| AVR25a | 7 | 100 | 62.3 | 0 |

### EXAMPLE 8 Mutation Library Construction and Screening of AAV-Binding Proteins (Part 3)

Using the vector pET-AVR25c, prepared in Example 6 and capable of expressing a polypeptide (SEQ ID NO: 28) including the AAV-binding protein AVR25c, as the template, random mutagenesis was applied to the polynucleotide portion encoding the protein using error-prone PCR.

(1) Error-prone PCR was performed by the same method as described in Example 2 (1) except that the vector pET-AVR25c, capable of expressing a polypeptide (SEQ ID NO: 28) including AVR25c, was used as the template. As a result of the error-prone PCR, mutations were successfully introduced into the polynucleotide encoding the AAV-binding protein. The average mutation introduction rate was 1.8 amino acid mutations per molecule.
(2) From the PCR product obtained in (1), a random mutant library was constructed by the method described in Example 2 (2) and (3).
(3) The random mutant library (transformants) constructed in (2) was inoculated into 200 µL of 2× YT liquid medium containing 50 µg/mL kanamycin and shake-cultured overnight at 37°C using a 96-well deep-well plate.
(4) The culture broth in (3) was centrifuged, and the resulting culture supernatant was diluted two-fold with ultrapure water. 50 µL of the diluted culture supernatant and 50 µL of a 1 M aqueous solution of sodium hydroxide were mixed, and the resulting mixture was subjected to alkali treatment for 15 minutes at 30°C.
(5) The binding activity between the AAV-binding protein and VLP2 after the treatment in (4) and the binding activity between the AAV-binding protein and VLP2 without the treatment in (4) were evaluated by ELISA as described in Example 2 (6). The remaining activity was calculated by dividing the binding activity between the AAV-binding protein and VLP2 after alkali treatment by the binding activity between the AAV-binding protein and VLP2 without alkali treatment.
(6) A random mutant library of approximately 1800 strains was evaluated by the method in (5), and transformants expressing AAV-binding proteins with improved remaining activity compared with AVR25c, the parent molecule, were selected therefrom.
(7) The transformants selected in (6) were cultured, and expression vectors were prepared using a QIAprep Spin Miniprep kit (manufactured by QIAGEN N.V.). Then the sequence of the AAV-binding protein-encoding polynucleotide region inserted in these vectors was analyzed by the method described in Example 2 (8) (outsourced to FASMAC Co., Ltd.), whereby the positions of amino acid substitutions were identified.

For the AAV-binding proteins expressed by the transformants selected in (7) above, a summary of the positions of amino acid substitutions with respect to AVR25c and the remaining activity [%] after alkali treatment is presented in Table 8.

It can be concluded that AAV-binding proteins in which at least one of any amino acid substitution from among G314D, V(D)317G, Q318R, P322A, E325V, Q347R, T350A, E362D, H363P, I366F, E378G, F(Y)379N, G392E, V394A, V398A, E399K, R406H, Q415R, F416L, L421P, S433G, T434S, D437V, E445K, K455R, S457R, T474R, T478I, A485V, T486A, L493M, N496D, K497E, V499I and D500G had occurred at the amino acid residues from serine (S) at position 25 to aspartic acid (D) at position 500 of the amino acid sequence set forth in SEQ ID NO: 2 exhibited improved stability against alkalis compared with AVR25c (SEQ ID NO: 28).

**[Table 8]**

| Amino acid substitutions | Remaining activity [%] |
|---|---|
| G314D, E399K, E445K | 62.5 |
| G314D, V4991 | 79.1 |
| V(D)317G, T4781 | 82.0 |
| Q318R, E362D, E378G | 66.1 |
| P322A, T350A, L421P | 49.6 |
| E325V, D437V | 76.7 |
| Q347R, T486A, D500G | 72.8 |
| H363P, K497E | 69.6 |
| I366F, V394A, K455R | 71.9 |
| E378G, V398A | 67.5 |
| F(Y)379N | 55.1 |
| G392E, S433G, A485V, L493M, N496D | 61.0 |
| R406H, F416L | 41.9 |
| Q415R, S457R | 55.1 |
| T434S | 77.3 |
| T474R | 100 |
| AVR25c | 33.5 |

### EXAMPLE 9 Preparation of AVR25c Amino Acid Substitution-Integrated Mutants (Part 1)

G314D, I366F, V394A, K455R, T474R, K467E and V499I were selected from the amino acid substitutions found to be responsible for improved alkaline stability of an AAV-binding protein in Example 8, and these amino acid substitutions were integrated into AVR25c (SEQ ID NO: 28) in an attempt to further improve alkaline stability. Specifically, the nine AAV-binding proteins specified in (a) to (i) below were designed and prepared.
(a) An AAV-binding protein in which the amino acid substitutions I366F and T474R have been introduced into AVR25c (SEQ ID NO: 31; designated as AVR27b)
(b) An AAV-binding protein in which the amino acid substitutions V394A and T474R have been introduced into AVR25c (SEQ ID NO: 32; designated as AVR27c)
(c) An AAV-binding protein in which the amino acid substitutions K455R and T474R have been introduced into AVR25c (SEQ ID NO: 33; designated as AVR27d)
(d) An AAV-binding protein in which the amino acid substitutions I366F, V394A and K455R have been introduced into AVR25c (SEQ ID NO: 34; designated as AVR28)
(e) An AAV-binding protein in which the amino acid substitutions I366F, V394A, K455R and T474R have been introduced into AVR25c (SEQ ID NO: 35; designated as AVR29a)
(f) An AAV-binding protein in which the amino acid substitutions G314D, I366F, V394A and K455R have been introduced into AVR25c (SEQ ID NO: 36; designated as AVR29b)
(g) An AAV-binding protein in which the amino acid substitutions I366F, V394A, K455R and K497E have been introduced into AVR25c (SEQ ID NO: 37; designated as AVR29c)
(h) An AAV-binding protein in which the amino acid substitutions I366F, V394A, K455R and V499I have been introduced into AVR25c (SEQ ID NO: 38; designated as AVR29d)
(i) An AAV-binding protein in which the amino acid substitutions G314D, I366F, V394A, K455R, K497E and V499I have been introduced into AVR25c (SEQ ID NO: 39; designated as AVR31)

In the following, the methods for preparing the nine AAV-binding proteins specified in (a) to (i) above will be described.

A PCR primer set for creating the mutation-integrated mutants was designed. Specifically,
SEQ ID NOs: 40 (Forward) and 41 (Reverse) as PCR primers for introducing the amino acid substitution G314D,
SEQ ID NOs: 42 (Forward) and 43 (Reverse) as PCR primers for introducing the amino acid substitution I366F,
SEQ ID NOs: 44 (Forward) and 45 (Reverse) as PCR primers for introducing the amino acid substitution V394A,
SEQ ID NOs: 46 (Forward) and 47 (Reverse) as PCR primers for introducing the amino acid substitution K455R,
SEQ ID NOs: 48 (Forward) and 49 (Reverse) as PCR primers for introducing the amino acid substitution T474R,
SEQ ID NOs: 50 (Forward) and 51 (Reverse) as PCR primers for introducing the amino acid substitution K497E,
SEQ ID NOs: 52 (Forward) and 53 (Reverse) as PCR primers for introducing the amino acid substitution V499I and
SEQ ID NOs: 54 (Forward) and 55 (Reverse) as PCR primers for introducing the amino acid substitutions K497E and V499I
were each designed.

### (a) AVR27b

This protein was prepared by selecting I366F and T474R from among the amino acid substitutions found to be responsible for alkaline stability in Example 9 and introducing I366F, of these amino acid substitutions, into an AAV-binding protein in which the amino acid substitution T474R had been introduced into AVR25c (see Table 8; hereinafter also referred to as "AVR25c_T474R"), obtained in Example 8.

(a-1) A vector capable of expressing AVR25c_T474R was used as the template, and oligonucleotides consisting of the sequences set forth in SEQ ID NO: 42 (5'-GTCAGTTTCTGAAATTATCCAACTT-3') and SEQ ID NO: 43 (5'-TTCAGAAACTGACTGTGTTCGCCTT-3') were used as the PCR primers. PCR was performed by preparing a reaction solution having the composition presented in Table 9, then subjecting the reaction solution to heat treatment at 98°C for 5 minutes, carrying out 30 cycles of reaction, in which a first step at 98°C for 10 seconds, a second step at 55°C for 5 seconds and a third step at 72°C for 6 minutes constituted one cycle, and finally applying heat treatment at 72°C for 5 minutes.

**[Table 9]**

| Composition | Volume |
|---|---|
| 50 ng/µL template | 1 µL |
| 10 µM PCR forward primer (SEQ ID NO: 40, 42, 44, 46, 48, 50, 52, 54 or 56) | 1 µL |
| 10 µM PCR reverse primer (SEQ ID NO: 41, 43, 45, 47, 49, 51, 53, 55 or 57) | 1 µL |
| 10× KOD buffer (manufactured by Toyobo Co., Ltd.) | 5 µL |
| 2 mM dNTPs | 5 µL |
| 25 mM MgSO₄ | 3 µL |
| 1 U/µL KOD Plus (manufactured by Toyobo Co., Ltd.) | 1 µL |
| H₂O | Up to 50 µL |

(a-2) The PCR product obtained in (a-1) was treated with DpnI (manufactured by New England Biolabs) to digest the template chain, and then the Escherichia coli strain BL21(DE3) was transformed using the PCR product. The resulting transformant was cultured in an LB medium supplemented with 50 µg/mL kanamycin, and then a plasmid was extracted from bacterial cells (transformant) harvested by centrifugation. In this manner, a plasmid (expression vector) pET-AVR27b including a polynucleotide encoding AVR27b, in which amino acid substitutions at 27 positions had been introduced into the wild-type AAV-binding protein, was obtained.
(a-3) The nucleotide sequence of pET-AVR27b was analyzed and confirmed by the same method as in Example 2 (8).

The amino acid sequence of AVR27b with a signal sequence and a polyhistidine tag attached thereto is presented in SEQ ID NO: 31. It should be noted that in SEQ ID NO: 31, methionine (M) at position 1 to alanine (A) at position 22 form the PelB signal peptide, serine (S) at position 25 to aspartic acid (D) at position 213 form the AAV-binding protein AVR27b (corresponding to the region from position 25 to position 213 of SEQ ID NO: 2), and histidine (H) from position 214 to position 219 form the tag sequence. In SEQ ID NO: 31, furthermore, the aspartic acid in V317D is present at position 30, the histidine in N324H is present at position 37, the alanine in V326A is present at position 39, the lysine in N329K is present at position 42, the valine in A330V is present at position 43, the leucine in Q334L is present at position 47, the valine in E335V is present at position 48, the alanine in T341A is present at position 54, the serine in Y342S is present at position 55, the glutamic acid in K362E is present at position 75, the phenylalanine in I366F is present at position 79, the asparagine in K371N is present at position 84, the tyrosine in F379Y is present at position 92, the arginine in K380R is present at position 93, the alanine in V381A is present at position 94, the valine in I382V is present at position 95, the serine in G390S is present at position 103, the glutamic acid in K399E is present at position 112, the glycine in E401G is present at position 114, the alanine in T426A is present at position 139, the proline in A461P is present at position 174, the asparagine in K467N is present at position 180, the arginine in T474R is present at position 187, the arginine in S476R is present at position 189, the threonine in S482T is present at position 195, the aspartic acid in N487D is present at position 200, and the aspartic acid in N492D is present at position 205.

### (b) AVR27c

This protein was prepared by selecting V394A and T474R from among the amino acid substitutions found to be responsible for alkaline stability in Example 8 and introducing V394A, of these amino acid substitutions, into AVR25c_T474R.

(b-1) PCR was performed by the same method as in (a-1) except that a vector capable of expressing AVR25c_T474R was used as the template, and that oligonucleotides consisting of the sequences set forth in SEQ ID NO: 44 (5'-GGTATGCGAACGTAACGGTTGAGCC-3') and SEQ ID NO: 45 (5'-ACGTTCGCATACCCCTCGGAATGCG-3') were used as the PCR primers.
(b-2) From the PCR product obtained in (b-1), a plasmid (expression vector) pET-AVR27c including a polynucleotide encoding AVR27c, in which amino acid substitutions at 27 positions had been introduced into the wild-type AAV-binding protein, was obtained by the same method as in (a-2).
(b-3) The nucleotide sequence of pET-AVR27c was analyzed and confirmed by the same method as in Example 2 (8).

The amino acid sequence of AVR27c with a signal sequence and a polyhistidine tag attached thereto is presented in SEQ ID NO: 32. It should be noted that in SEQ ID NO: 32, methionine (M) at position 1 to alanine (A) at position 22 form the PelB signal peptide, serine (S) at position 25 to aspartic acid (D) at position 213 form the AAV-binding protein AVR27c (corresponding to the region from position 25 to position 213 of SEQ ID NO: 2), and histidine (H) from position 214 to position 219 form the tag sequence. In SEQ ID NO: 32, furthermore, the aspartic acid in V317D is present at position 30, the histidine in N324H is present at position 37, the alanine in V326A is present at position 39, the lysine in N329K is present at position 42, the valine in A330V is present at position 43, the leucine in Q334L is present at position 47, the valine in E335V is present at position 48, the alanine in T341A is present at position 54, the serine in Y342S is present at position 55, the glutamic acid in K362E is present at position 75, the asparagine in K371N is present at position 84, the tyrosine in F379Y is present at position 92, the arginine in K380R is present at position 93, the alanine in V381A is present at position 94, the valine in I382V is present at position 95, the serine in G390S is present at position 103, the alanine in V394A is present at position 107, the glutamic acid in K399E is present at position 112, the glycine in E401G is present at position 114, the alanine in T426A is present at position 139, the proline in A461P is present at position 174, the asparagine in K467N is present at position 180, the arginine in T474R is present at position 187, the arginine in S476R is present at position 189, the threonine in S482T is present at position 195, the aspartic acid in N487D is present at position 200, and the aspartic acid in N492D is present at position 205.

### (c) AVR27d

This protein was prepared by selecting K455R and T474R from among the amino acid substitutions found to be responsible for alkaline stability in Example 8 and introducing K455R, of these amino acid substitutions, into AVR25c_T474R.

(c-1) PCR was performed by the same method as in (a-1) except that a vector capable of expressing AVR25c_T474R was used as the template, and that oligonucleotides consisting of the sequences set forth in SEQ ID NO: 46 (5'-AAGAACGCATCAGCGAGGATACCCC-3') and SEQ ID NO: 47 (5'-CTGATGCGTTCTTCACGGAGCGGGC-3') were used as the PCR primers.
(c-2) From the PCR product obtained in (c-1), a plasmid (expression vector) pET-AVR27d including a polynucleotide encoding AVR27d, in which amino acid substitutions at 27 positions had been introduced into the wild-type AAV-binding protein, was obtained by the same method as in (a-2).
(c-3) The nucleotide sequence of pET-AVR27d was analyzed and confirmed by the same method as in Example 2 (8).

The amino acid sequence of AVR27d with a signal sequence and a polyhistidine tag attached thereto is presented in SEQ ID NO: 33. It should be noted that in SEQ ID NO: 33, methionine (M) at position 1 to alanine (A) at position 22 form the PelB signal peptide, serine (S) at position 25 to aspartic acid (D) at position 213 form the AAV-binding protein AVR27d (corresponding to the region from position 25 to position 213 of SEQ ID NO: 2), and histidine (H) from position 214 to position 219 form the tag sequence. In SEQ ID NO: 33, furthermore, the aspartic acid in V317D is present at position 30, the histidine in N324H is present at position 37, the alanine in V326A is present at position 39, the lysine in N329K is present at position 42, the valine in A330V is present at position 43, the leucine in Q334L is present at position 47, the valine in E335V is present at position 48, the alanine in T341A is present at position 54, the serine in Y342S is present at position 55, the glutamic acid in K362E is present at position 75, the asparagine in K371N is present at position 84, the tyrosine in F379Y is present at position 92, the arginine in K380R is present at position 93, the alanine in V381A is present at position 94, the valine in I382V is present at position 95, the serine in G390S is present at position 103, the glutamic acid in K399E is present at position 112, the glycine in E401G is present at position 114, the alanine in T426A is present at position 139, the arginine in K455R is present at position 168, the proline in A461P is present at position 174, the asparagine in K467N is present at position 180, the arginine in T474R is present at position 187, the arginine in S476R is present at position 189, the threonine in S482T is present at position 195, the aspartic acid in N487D is present at position 200, and the aspartic acid in N492D is present at position 205.

### (d) AVR28

For this protein, I366F, V394A and K455R were selected from among the amino acid substitutions found to be responsible for alkaline stability in Example 8. It should be noted that this protein had already been obtained in Example 8 (see Table 8) and therefore was used as is.

The amino acid sequence of AVR28 with a signal sequence and a polyhistidine tag attached thereto is presented in SEQ ID NO: 34. It should be noted that in SEQ ID NO: 34, methionine (M) at position 1 to alanine (A) at position 22 form the PelB signal peptide, serine (S) at position 25 to aspartic acid (D) at position 213 form the AAV-binding protein AVR28a (corresponding to the region from position 25 to position 213 of SEQ ID NO: 2), and histidine (H) from position 214 to position 219 form the tag sequence. In SEQ ID NO: 34, furthermore, the aspartic acid in V317D is present at position 30, the histidine in N324H is present at position 37, the alanine in V326A is present at position 39, the lysine in N329K is present at position 42, the valine in A330V is present at position 43, the leucine in Q334L is present at position 47, the valine in E335V is present at position 48, the alanine in T341A is present at position 54, the serine in Y342S is present at position 55, the glutamic acid in K362E is present at position 75, the phenylalanine in I366F is present at position 79, the asparagine in K371N is present at position 84, the tyrosine in F379Y is present at position 92, the arginine in K380R is present at position 93, the alanine in V381A is present at position 94, the valine in I382V is present at position 95, the serine in G390S is present at position 103, the alanine in V394A is present at position 107, the glutamic acid in K399E is present at position 112, the glycine in E401G is present at position 114, the alanine in T426A is present at position 139, the arginine in K455R is present at position 168, the proline in A461P is present at position 174, the asparagine in K467N is present at position 180, the arginine in S476R is present at position 189, the threonine in S482T is present at position 195, the aspartic acid in N487D is present at position 200, and the aspartic acid in N492D is present at position 205.

### (e) AVR29a

This protein was prepared by selecting T474R from among the amino acid substitutions found to be responsible for alkaline stability in Example 8 and introducing this amino acid substitution into AVR28 (SEQ ID NO: 34).

(e-1) PCR was performed by the same method as in (a-1) except that a vector pET-AVR28a capable of expressing a polypeptide (SEQ ID NO: 34) including AVR28 was used as the template, and that oligonucleotides consisting of the sequences set forth in SEQ ID NO: 48 (5'-ACTACCGCTTTCGCCTGACCGTGAC-3') and SEQ ID NO: 49 (5'-CGAAAGCGGTAGTTACCTGGTACCA-3') were used as the PCR primers.
(e-2) From the PCR product obtained in (e-1), a plasmid (expression vector) pET-AVR29a including a polynucleotide encoding AVR29a, in which amino acid substitutions at 29 positions had been introduced into the wild-type AAV-binding protein, was obtained by the same method as in (a-2).
(e-3) The nucleotide sequence of pET-AVR29a was analyzed and confirmed by the same method as in Example 2 (8).

The amino acid sequence of AVR29a with a signal sequence and a polyhistidine tag attached thereto is presented in SEQ ID NO: 35. It should be noted that in SEQ ID NO: 35, methionine (M) at position 1 to alanine (A) at position 22 form the PelB signal peptide, serine (S) at position 25 to aspartic acid (D) at position 213 form the AAV-binding protein AVR29a (corresponding to the region from position 25 to position 213 of SEQ ID NO: 2), and histidine (H) from position 214 to position 219 form the tag sequence. In SEQ ID NO: 35, furthermore, the aspartic acid in V317D is present at position 30, the histidine in N324H is present at position 37, the alanine in V326A is present at position 39, the lysine in N329K is present at position 42, the valine in A330V is present at position 43, the leucine in Q334L is present at position 47, the valine in E335V is present at position 48, the alanine in T341A is present at position 54, the serine in Y342S is present at position 55, the glutamic acid in K362E is present at position 75, the phenylalanine in I366F is present at position 79, the asparagine in K371N is present at position 84, the tyrosine in F379Y is present at position 92, the arginine in K380R is present at position 93, the alanine in V381A is present at position 94, the valine in I382V is present at position 95, the serine in G390S is present at position 103, the alanine in V394A is present at position 107, the glutamic acid in K399E is present at position 112, the glycine in E401G is present at position 114, the alanine in T426A is present at position 139, the arginine in K455R is present at position 168, the proline in A461P is present at position 174, the asparagine in K467N is present at position 180, the arginine in T474R is present at position 187, the arginine in S476R is present at position 189, the threonine in S482T is present at position 195, the aspartic acid in N487D is present at position 200, and the aspartic acid in N492D is present at position 205.

### (f) AVR29b

This protein was prepared by selecting G314D from among the amino acid substitutions found to be responsible for alkaline stability in Example 9 and introducing this amino acid substitution into AVR28 (SEQ ID NO: 34).

(f-1) PCR was performed by the same method as in (e-1) except that oligonucleotides consisting of the sequences set forth in SEQ ID NO: 40 (5'-CTGCAGATGAAAGCGACCAAATCAC-3') and SEQ ID NO: 41 (5'-CTTTCATCTGCAGAGCCCATGGCCA-3') were used as the PCR primers.
(f-2) From the PCR product obtained in (f-1), a plasmid (expression vector) pET-AVR29b including a polynucleotide encoding AVR29b, in which amino acid substitutions at 29 positions had been introduced into the wild-type AAV-binding protein, was obtained by the same method as in (a-2).
(f-3) The nucleotide sequence of pET-AVR29b was analyzed and confirmed by the same method as in Example 2 (8).

The amino acid sequence of AVR29b with a signal sequence and a polyhistidine tag attached thereto is presented in SEQ ID NO: 36. It should be noted that in SEQ ID NO: 36, methionine (M) at position 1 to alanine (A) at position 22 form the PelB signal peptide, serine (S) at position 25 to aspartic acid (D) at position 213 form the AAV-binding protein AVR29b (corresponding to the region from position 25 to position 213 of SEQ ID NO: 2), and histidine (H) from position 214 to position 219 form the tag sequence. In SEQ ID NO: 36, furthermore, the aspartic acid in G314D is present at position 27, the aspartic acid in V317D is present at position 30, the histidine in N324H is present at position 37, the alanine in V326A is present at position 39, the lysine in N329K is present at position 42, the valine in A330V is present at position 43, the leucine in Q334L is present at position 47, the valine in E335V is present at position 48, the alanine in T341A is present at position 54, the serine in Y342S is present at position 55, the glutamic acid in K362E is present at position 75, the phenylalanine in I366F is present at position 79, the asparagine in K371N is present at position 84, the tyrosine in F379Y is present at position 92, the arginine in K380R is present at position 93, the alanine in V381A is present at position 94, the valine in I382V is present at position 95, the serine in G390S is present at position 103, the alanine in V394A is present at position 107, the glutamic acid in K399E is present at position 112, the glycine in E401G is present at position 114, the alanine in T426A is present at position 139, the arginine in K455R is present at position 168, the proline in A461P is present at position 174, the asparagine in K467N is present at position 180, the arginine in S476R is present at position 189, the threonine in S482T is present at position 195, the aspartic acid in N487D is present at position 200, and the aspartic acid in N492D is present at position 205.

### (g) AVR29c

This protein was prepared by selecting K497E from among the amino acid substitutions found to be responsible for alkaline stability in Example 8 and introducing this amino acid substitution into AVR28 (SEQ ID NO: 34).

(g-1) PCR was performed by the same method as in (e-1) except that oligonucleotides consisting of the sequences set forth in SEQ ID NO: 50 (5'-TTAACGAAGCCGTCGACCATCATCA-3') and SEQ ID NO: 51 (5'-ACGGCTTCGTTAACGGTCAAGTCTG-3') were used as the PCR primers.
(g-2) From the PCR product obtained in (g-1), a plasmid (expression vector) pET-AVR29c including a polynucleotide encoding AVR29c, in which amino acid substitutions at 29 positions had been introduced into the wild-type AAV-binding protein, was obtained by the same method as in (a-2).
(g-3) The nucleotide sequence of pET-AVR29c was analyzed and confirmed by the same method as in Example 2 (8).

The amino acid sequence of AVR29c with a signal sequence and a polyhistidine tag attached thereto is presented in SEQ ID NO: 37. It should be noted that in SEQ ID NO: 37, methionine (M) at position 1 to alanine (A) at position 22 form the PelB signal peptide, serine (S) at position 25 to aspartic acid (D) at position 213 form the AAV-binding protein AVR29c (corresponding to the region from position 25 to position 213 of SEQ ID NO: 2), and histidine (H) from position 214 to position 219 form the tag sequence. In SEQ ID NO: 37, furthermore, the aspartic acid in V317D is present at position 30, the histidine in N324H is present at position 37, the alanine in V326A is present at position 39, the lysine in N329K is present at position 42, the valine in A330V is present at position 43, the leucine in Q334L is present at position 47, the valine in E335V is present at position 48, the alanine in T341A is present at position 54, the serine in Y342S is present at position 55, the glutamic acid in K362E is present at position 75, the phenylalanine in I366F is present at position 79, the asparagine in K371N is present at position 84, the tyrosine in F379Y is present at position 92, the arginine in K380R is present at position 93, the alanine in V381A is present at position 94, the valine in I382V is present at position 95, the serine in G390S is present at position 103, the alanine in V394A is present at position 107, the glutamic acid in K399E is present at position 112, the glycine in E401G is present at position 114, the alanine in T426A is present at position 139, the arginine in K455R is present at position 168, the proline in A461P is present at position 174, the asparagine in K467N is present at position 180, the arginine in S476R is present at position 189, the threonine in S482T is present at position 195, the aspartic acid in N487D is present at position 200, the aspartic acid in N492D is present at position 205, and the glutamic acid in K497E is present at position 210.

### (h) AVR29d

This protein was prepared by selecting V499I from among the amino acid substitutions found to be responsible for alkaline stability in Example 8 and introducing this amino acid substitution into AVR28 (SEQ ID NO: 34).

(h-1) PCR was performed by the same method as in (e-1) except that oligonucleotides consisting of the sequences set forth in SEQ ID NO: 52 (5'-AAGCCATTGACCATCATCATCATCA-3') and SEQ ID NO: 53 (5'-TGGTCAATGGCTTTGTTAACGGTCA-3') were used as the PCR primers.
(h-2) From the PCR product obtained in (h-1), a plasmid (expression vector) pET-AVR29d including a polynucleotide encoding AVR29d, in which amino acid substitutions at 29 positions had been introduced into the wild-type AAV-binding protein, was obtained by the same method as in (a-2).
(h-3) The nucleotide sequence of pET-AVR29d was analyzed and confirmed by the same method as in Example 2 (8).

The amino acid sequence of AVR29d with a signal sequence and a polyhistidine tag attached thereto is presented in SEQ ID NO: 38. It should be noted that in SEQ ID NO: 38, methionine (M) at position 1 to alanine (A) at position 22 form the PelB signal peptide, serine (S) at position 25 to aspartic acid (D) at position 213 form the AAV-binding protein AVR29d (corresponding to the region from position 25 to position 213 of SEQ ID NO: 2), and histidine (H) from position 214 to position 219 form the tag sequence. In SEQ ID NO: 38, furthermore, the aspartic acid in V317D is present at position 30, the histidine in N324H is present at position 37, the alanine in V326A is present at position 39, the lysine in N329K is present at position 42, the valine in A330V is present at position 43, the leucine in Q334L is present at position 47, the valine in E335V is present at position 48, the alanine in T341A is present at position 54, the serine in Y342S is present at position 55, the glutamic acid in K362E is present at position 75, the phenylalanine in I366F is present at position 79, the asparagine in K371N is present at position 84, the tyrosine in F379Y is present at position 92, the arginine in K380R is present at position 93, the alanine in V381A is present at position 94, the valine in I382V is present at position 95, the serine in G390S is present at position 103, the alanine in V394A is present at position 107, the glutamic acid in K399E is present at position 112, the glycine in E401G is present at position 114, the alanine in T426A is present at position 139, the arginine in K455R is present at position 168, the proline in A461P is present at position 174, the asparagine in K467N is present at position 180, the arginine in S476R is present at position 189, the threonine in S482T is present at position 195, the aspartic acid in N487D is present at position 200, the aspartic acid in N492D is present at position 205, and the isoleucine in V499I is present at position 212.

### (i) AVR31

This protein was prepared by selecting K497E and V499I from among the amino acid substitutions found to be responsible for alkaline stability in Example 8 and introducing these amino acid substitutions into AVR29b (SEQ ID NO: 36).

(i-1) PCR was performed by the same method as in (a-1) except that a vector pET-AVR29b capable of expressing a polypeptide (SEQ ID NO: 36) including AVR29b was used as the template, and that oligonucleotides consisting of the sequences set forth in SEQ ID NO: 54 (5'-AACGAAGCCATTGACCATCATCATCATCAT-3') and SEQ ID NO: 55 (5'-GTCAATGGCTTCGTTAACGGTCAAGTCTGC-3') were used as the PCR primers.
(i-2) From the PCR product obtained in (i-1), a plasmid (expression vector) pET-AVR31 including a polynucleotide encoding AVR31, in which amino acid substitutions at 31 positions had been introduced into the wild-type AAV-binding protein, was obtained by the same method as in (a-2).
(i-3) The nucleotide sequence of pET-AVR31 was analyzed and confirmed by the same method as in Example 2 (8).

The amino acid sequence of AVR31 with a signal sequence and a polyhistidine tag attached thereto is presented in SEQ ID NO: 39. It should be noted that in SEQ ID NO: 39, methionine (M) at position 1 to alanine (A) at position 22 form the PelB signal peptide, serine (S) at position 25 to aspartic acid (D) at position 213 form the AAV-binding protein AVR31 (corresponding to the region from position 25 to position 213 of SEQ ID NO: 2), and histidine (H) from position 214 to position 219 form the tag sequence. In SEQ ID NO: 39, furthermore, the aspartic acid in G314D is present at position 27, the aspartic acid in V317D is present at position 30, the histidine in N324H is present at position 37, the alanine in V326A is present at position 39, the lysine in N329K is present at position 42, the valine in A330V is present at position 43, the leucine in Q334L is present at position 47, the valine in E335V is present at position 48, the alanine in T341A is present at position 54, the serine in Y342S is present at position 55, the glutamic acid in K362E is present at position 75, the phenylalanine in I366F is present at position 79, the asparagine in K371N is present at position 84, the tyrosine in F379Y is present at position 92, the arginine in K380R is present at position 93, the alanine in V381A is present at position 94, the valine in I382V is present at position 95, the serine in G390S is present at position 103, the alanine in V394A is present at position 107, the glutamic acid in K399E is present at position 112, the glycine in E401G is present at position 114, the alanine in T426A is present at position 139, the arginine in K455R is present at position 168, the proline in A461P is present at position 174, the asparagine in K467N is present at position 180, the arginine in S476R is present at position 189, the threonine in S482T is present at position 195, the aspartic acid in N487D is present at position 200, the aspartic acid in N492D is present at position 205, the glutamic acid in K497E is present at position 210, and the isoleucine in V499I is present at position 212.

### EXAMPLE 10 Preparation of AVR25c Amino Acid Substitution-Integrated Mutants (Part 2)

(1) Culturing of transformants and harvesting of bacterial cells were performed by the same method as in Example 4 (1) to (4) except that transformants obtained by transforming the Escherichia coli strain BL21(DE3) with plasmids including a polynucleotide encoding any of the nine AAV-binding proteins obtained in Example 9 (AVR25c amino acid substitution-integrated mutants, or, specifically, AVR27b (SEQ ID NO: 31), AVR27c (SEQ ID NO: 32), AVR27d (SEQ ID NO: 33), AVR28 (SEQ ID NO: 34), AVR29a (SEQ ID NO: 35), AVR29b (SEQ ID NO: 36), AVR29c (SEQ ID NO: 37), AVR29d (SEQ ID NO: 38) and AVR31 (SEQ ID NO: 39)), AVR21 (SEQ ID NO: 2) or AVR25c (SEQ ID NO: 28) were used as transformants capable of expressing AAV-binding proteins.
(2) Bugbuster 10× (manufactured by Novagen) and Benzonase nuclease, Purity > 90% (manufactured by Merck KGaA) were mixed into a 20 mM Tris hydrochloride buffer (pH 7.4) containing 20 mM imidazole (hereinafter also referred to as "equilibrating solution D") such that the volume of Bugbuster was 1/10 of the total volume and that the concentration of Benzonase nuclease was 25 U/mL. The bacterial cells harvested in (1) were suspended to 5 mL/1 g (bacterial cells) using the resulting mixture, and the bacterial cells were disrupted by shaking for 20 minutes at 15°C. The cell homogenates were centrifuged at 8000 rpm for 20 minutes at 4°C, and the supernatant of each was collected.
(3) The supernatant obtained in (2) was applied to an open column packed with 1.5 mL of Ni-NTA Agarose (manufactured by FUJIFILM Wako Pure Chemical Corporation) pre-equilibrated with equilibrating solution D. After washing with equilibrating solution A, elution was performed using a 20 mM Tris hydrochloride buffer (pH 7.4) containing 0.5 M imidazole.
(4) Each of the eluates obtained in (3) was desalted using PD MiniTrap G-25 (manufactured by Cytiva). In this manner, AAV-binding protein solutions were prepared.

### EXAMPLE 11 Evaluation of Alkaline Stability of the AVR25c Amino Acid Substitution-Integrated Mutants (Part 1)

The evaluation of alkaline stability was performed using AVR25c (SEQ ID NO: 28), AVR28 (SEQ ID NO: 34) and AVR29a (SEQ ID NO: 35), among the AAV-binding proteins prepared in Example 10.

(1) After concentration measurement using NanoDrop OneC (manufactured by Thermo Fisher Scientific Inc.), the AAV-binding protein solutions prepared in Example 10 were diluted with a 20 mM Tris hydrochloride buffer (pH 7.4) to a protein concentration of 5 µg/mL.
(2) Each of the AAV-binding protein solutions diluted in (1) was subjected to alkali treatment in which it was mixed with an equal volume of a 1 M aqueous solution of sodium hydroxide containing 1 mM calcium chloride, and the resulting mixture was allowed to stand at a constant temperature/for certain durations (treatment temperature, 30°C; treatment durations, 0, 30 and 60 minutes)
(3) The fraction after the treatment in (2) was adjusted to a pH of approximately 6 by mixing a 0.5 M MES buffer (pH 6.0) into it at a ratio of 8:2, and then the binding activity to VLP2 was measured by ELISA as described in Example 2 (6).
(4) The remaining activity was calculated by dividing the absorbance at 450 nm after the alkali treatment in (3) by the absorbance at 450 nm at a treatment duration of 0 hours.

The results are presented in Table 10. It can be understood that AVR28 (SEQ ID NO: 34) and AVR29a (SEQ ID NO: 35) both exhibited higher remaining activity values compared with AVR25c (SEQ ID NO: 28), indicating improved stability against alkalis.

**[Table 10]**

| AAV-binding protein | | Remaining activity [%] | | |
|---|---|---|---|---|
| Name | SEQ ID NO | Start | 30 minutes | 60 minutes |
| AVR28 | 34 | 100 | 22.5 | 3.00 |
| AVR29a | 35 | 100 | 24.2 | 5.07 |
| AVR25c | 28 | 100 | 2.87 | 0.197 |

### EXAMPLE 12 Evaluation of Alkaline Stability of the AVR25c Amino Acid Substitution-Integrated Mutants (Part 2)

The evaluation of alkaline stability was performed using AVR25c (SEQ ID NO: 28), AVR27b (SEQ ID NO: 31), AVR27c (SEQ ID NO: 32) and AVR27d (SEQ ID NO: 33), among the AAV-binding proteins prepared in Example 10. This evaluation was performed by the same method as in Example 11 except that the treatment duration was 0, 30 minutes or 45 minutes.

The results are presented in Table 11. It can be understood that AVR27b (SEQ ID NO: 31), AVR27c (SEQ ID NO: 32) and AVR27d (SEQ ID NO: 33) all exhibited higher remaining activity values compared with AVR25c (SEQ ID NO: 28), indicating improved stability against alkalis.

**[Table 11]**

| AAV-binding protein | | Remaining activity [%] | | |
|---|---|---|---|---|
| Name | SEQ ID NO | Start | 30 minutes | 45 minutes |
| AVR27b | 31 | 100 | 17.1 | 4.54 |
| AVR27c | 32 | 100 | 13.6 | 1.73 |
| AVR27d | 33 | 100 | 15.8 | 5.11 |
| AVR25c | 28 | 100 | 5.28 | 0 |

### EXAMPLE 13 Evaluation of Alkaline Stability of the AVR25c Amino Acid Substitution-Integrated Mutants (Part 3)

The evaluation of alkaline stability was performed using AVR25c (SEQ ID NO: 28), AVR29b (SEQ ID NO: 36), AVR29c (SEQ ID NO: 37) and AVR29d (SEQ ID NO: 38), among the AVR25c amino acid substitution-integrated mutants prepared in Example 10. This evaluation was performed by the same method as in Example 11 except that the treatment duration was 0, 20 minutes or 45 minutes.

The results are presented in Table 12. It can be understood that AVR29b (SEQ ID NO: 36), AVR29c (SEQ ID NO: 37) and AVR29d (SEQ ID NO: 38) all exhibited higher remaining activity values compared with AVR25c (SEQ ID NO: 28), indicating improved stability against alkalis.

**[Table 12]**

| AAV-binding protein | | Remaining activity [%] | | |
|---|---|---|---|---|
| Name | SEQ ID NO | Start | 20 minutes | 45 minutes |
| AVR29b | 36 | 100 | 58.7 | 1.31 |
| AVR29c | 37 | 100 | 78.7 | 1.93 |
| AVR29d | 38 | 100 | 63.0 | 4.47 |
| AVR25c | 28 | 100 | 12.1 | 0 |

### EXAMPLE 14 Evaluation of Alkaline Stability of the AVR25c Amino Acid Substitution-Integrated Mutants (Part 4)

The evaluation of alkaline stability was performed using AVR28 (SEQ ID NO: 34), AVR29b (SEQ ID NO: 36), AVR29c (SEQ ID NO: 37) and AVR29d (SEQ ID NO: 38), among the AAV-binding proteins prepared in Example 10. This evaluation was performed by the same method as in Example 11 except that the treatment duration was 0, 20 minutes or 60 minutes.

The results are presented in Table 13. It can be understood that AVR29b (SEQ ID NO: 36), AVR29c (SEQ ID NO: 37) and AVR29d (SEQ ID NO: 38) all exhibited higher remaining activity values compared with AVR28 (SEQ ID NO: 34), indicating improved stability against alkalis.

**[Table 13]**

| AAV-binding protein | | Remaining activity [%] | | |
|---|---|---|---|---|
| Name | SEQ ID NO | Start | 20 minutes | 60 minutes |
| AVR29b | 36 | 100 | 63.7 | 1.08 |
| AVR29c | 37 | 100 | 93.2 | 2.39 |
| AVR29d | 38 | 100 | 68.6 | 1.06 |
| AVR28 | 34 | 100 | 42.5 | 1.22 |

### EXAMPLE 15 Evaluation of Alkaline Stability of the AVR25c Amino Acid Substitution-Integrated Mutants (Part 5)

The evaluation of alkaline stability was performed by the method described in Example 11 using AVR25c (SEQ ID NO: 28), AVR28 (SEQ ID NO: 34), AVR29c (SEQ ID NO: 37) and AVR31 (SEQ ID NO: 39), among the AAV-binding proteins prepared in Example 10.

The results are presented in Table 14. It can be understood that AVR28 (SEQ ID NO: 34), AVR29c (SEQ ID NO: 37) and AVR31 (SEQ ID NO: 39) all exhibited higher remaining activity values compared with AVR25c (SEQ ID NO: 28), indicating improved stability against alkalis.

**[Table 14]**

| AAV-binding protein | | Remaining activity [%] | | |
|---|---|---|---|---|
| Name | SEQ ID NO | Start | 30 minutes | 60 minutes |
| AVR28 | 34 | 100 | 42.0 | 6.29 |
| AVR29c | 37 | 100 | 57.7 | 15.7 |
| AVR31 | 39 | 100 | 48.3 | 10.6 |
| AVR25c | 28 | 100 | 22.0 | 2.19 |

### EXAMPLE 16 Evaluation of Alkaline Stability of the AVR25c Amino Acid Substitution-Integrated Mutants (Part 6)

The evaluation of alkaline stability was performed by the method described in Example 11 using AVR21 (SEQ ID NO: 2), AVR25c (SEQ ID NO: 28) and AVR31 (SEQ ID NO: 39), among the AAV-binding proteins prepared in Example 10.

The results are presented in Table 15. It can be understood that AVR31 (SEQ ID NO: 39) exhibited higher remaining activity values compared with AVR21 (SEQ ID NO: 2) and AVR25c (SEQ ID NO: 28), indicating improved stability against alkalis.

**[Table 15]**

| AAV-binding protein | | Remaining activity [%] | | |
|---|---|---|---|---|
| Name | SEQ ID NO | Start | 30 minutes | 60 minutes |
| AVR25c | 28 | 100 | 16.3 | 1.96 |
| AVR31 | 39 | 100 | 53.8 | 13.3 |
| AVR21 | 2 | 100 | 1.53 | 0.694 |

### EXAMPLE 17 Preparation and Evaluation of an AAV-Binding Protein-Immobilized Gel

(1) A gel was prepared in which iodoacetamide groups had been introduced to a hydrophilic vinyl polymer (manufactured by Tosoh Corporation: TOYOPEARL) for use as a separating agent by applying a chemical modification to hydroxy groups on its surface. To 1.0 g of the prepared gel, 6 mg of AVR29c (SEQ ID NO: 37) and TCEP (Tris(2-CarboxyEthyl) Phosphine) corresponding to a final concentration of 0.3 mM were added as an AAV-binding protein prepared in Example 10 and as a reducing agent, respectively. The reaction was allowed to proceed by shaking for 3 hours under conditions of pH 7.4 and 25°C. Through this, a gel onto which AVR29c had been immobilized (designated as an AVR29c-immobilized gel), which was an AAV adsorbent, was prepared.
(2) An AAV adsorbent column was prepared by packing 2.0 mL of the AVR29c-immobilized gel prepared in (1) into an empty column (φ10 mm × 50 mm, manufactured by Cytiva) (hereinafter also referred to as "AVR29c column").
(3) The AVR29c column prepared in (2) was equilibrated with a 20 mM Tris hydrochloride buffer (pH 8.0) containing 0.15 mM sodium chloride (hereinafter also referred to as "equilibrating solution E").
(4) The AAV8-EGFP solution obtained in Example 5 was applied to this column. After washing with equilibrating solution E, elution was performed using a 0.1 M acetate buffer (pH 2.5) containing 0.15 M sodium chloride hereinafter also referred to as "eluent B"). In this manner, a solution of AAV8-EGFP, an AAV vector, was obtained.

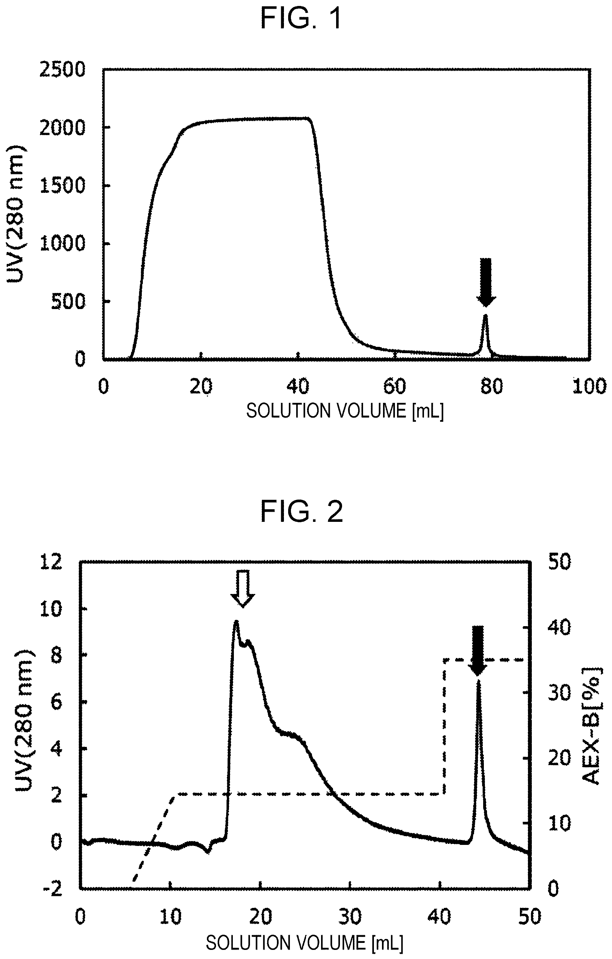

The obtained chromatogram is presented in Fig. 1. It should be noted that in Fig. 1, the peak indicated by the black arrow corresponds to the peak for the fraction containing the AAV vector (AAV8-EGFP). From the fact that large part of the impurities contained in the AAV8-EGFP solution passed through the AVR29c column, it can be understood that an AAV vector contained in a solution can be purified using an AVR29c column.

### EXAMPLE 18 Purification Combining Affinity Chromatography and Anion-Exchange Chromatography (Part 1)

An AAV vector solution after purification by affinity chromatography was applied to an anion-exchange column in an attempt to further purify the AAV vector.

(1) The sample to be applied to the anion-exchange column was prepared by diluting the AAV vector solution obtained in Example 17 (4) five-fold with a 20 mM Tris hydrochloride buffer (pH 9.0) (hereinafter also referred to as solution AEX-A).
(2) The sample prepared in (1) was applied at 0.1 mL/min to a column prepared by pre-equilibrating 0.5 mL of TOYOPEARL GigaCap Q-650S (a high-capacity anion-exchange chromatography support for separation and purification, manufactured by Tosoh Corporation) with solution AEX-A and packing it into an empty column (φ5 mm × 50 mm, manufactured by Cytiva) (hereinafter also referred to simply as "GigaCap Q column").
(3) After the GigaCap Q column was washed by passing 10 CV (Column Volumes) of solution AEX-A at 0.5 mL/min, a linear gradient was applied until solution AEX-A containing 1 M choline chloride (hereinafter also referred to as solution AEX-B) constituted 14.5%. By passing 60 CV while maintaining this state (the state in which AEX-B constituted 14.5%), impurities were eluted.
(4) The AAV vector (AAV8-EGFP) was eluted by applying a step gradient that brought the percentage of solution AEX-B to 35%.

The obtained chromatogram is presented in Fig. 2. It should be noted that in Fig. 2, the peak indicated by the black arrow corresponds to the peak for the fraction containing the AAV vector (AAV8-EGFP). The impurities remaining in the AAV vector solution obtained in Example 18 (4) were removed by the anion-exchange column (the peak under the white arrow in Fig. 2 corresponds to the impurities). From these results, it can be understood that by purifying a solution containing an AAV vector by affinity chromatogram using an AAV-binding protein-immobilized gel and then further purifying it by anion-exchange chromatography, the purity of the AAV vector can be further improved.

### EXAMPLE 19 Measurement of the Percentage of AAV Vectors Including a Gene (Full Ratio)

For the fraction obtained in Example 17 (4) (the peak under the black arrow in Fig. 1) and the fractions obtained in Example 18 (3) (the peak under the white arrow in Fig. 2) and Example 18 (4) (the peak under the black arrow in Fig. 2), the percentage of AAV vectors including a gene to the AAV vectors contained in the fraction (Full ratio) was measured by the method described below.

(1) Each fraction was diluted four-fold with solution AEX-A, and the diluted fraction was applied to TSKgel Q-STAT (manufactured by Tosoh Corporation), a column for anion-exchange chromatography.
(2) AAV vectors lacing a gene (Empty AAV vectors) were eluted by applying a linear gradient at a flow rate of 1 mL/min such that solution AEX-B reached 17.0% in 7 minutes.
(3) After the state in which solution AEX-B constituted 17.0% was maintained for 3 minutes, AAV vectors including a gene (Full AAV vectors) were eluted by applying a linear gradient at a flow rate of 1 mL/min such that solution AEX-B reached 45.0% in 10 minutes.
(4) The percentage of Full AAV vectors (Full ratio) was calculated by quantifying the peak area for each of the fraction obtained in (2) (containing Empty AAV vectors) and the fraction obtained in (3) (containing Full AAV vectors) based on fluorescence intensity at 350 nm with excitation light at 280 nm and determining the percentage of the peak area for the fraction in (3) in relation to the sum of the peak areas for the fractions in (2) and (3).

The calculated Full ratios were:
12.1% for the fraction obtained in Example 17 (4), i.e., the fraction purified using an AVR29c column;
8.5% for the fraction obtained in Example 18 (3), i.e., the wash fraction from a GigaCap Q column; and
82% for the fraction obtained in Example 18 (4), i.e., the fraction purified using a GigaCap Q column.

From these results, it can be understood that by purifying a solution containing an AAV vector by affinity chromatography using an AAV adsorbent and then further purifying it by anion-exchange chromatography, not only is the purify of the AAV vector improved, but the Full ratio (i.e., the percentage of AAV vectors including a gene) is also improved.

### REFERENCE EXAMPLE 3 AAV Purification Solely by Anion-Exchange Chromatography

(1) The AAV8-EGFP solution obtained in Example 5 was prepared to pH 9.0 using a Tris buffer.
(2) The AAV8-EGFP solution pH-prepared in (1) was purified using a GigaCap Q column by the method described in Example 18 (2) to (4).
(3) Each of the resulting fractions (peaks) was analyzed using a column packed with TSKgel G6000PW_{XL} (a gel for size-exclusion chromatography, manufactured by Tosoh Corporation).

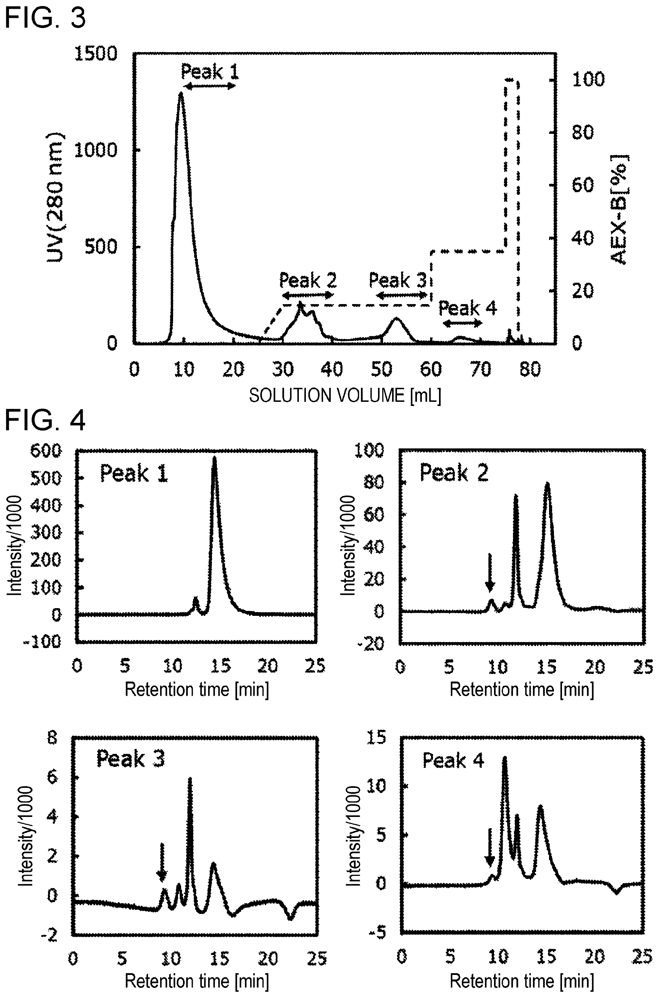

The results of the purification with a GigaCap Q column (chromatogram) are presented in Fig. 3, and the results of the analysis of each resulting fraction with TSKgel G6000PW_{XL} are presented in Fig. 4. It should be noted that the black arrows in Fig. 4 correspond to the peaks for fractions containing the AAV vector (AAV8-EGFP). It can be understood that although peaks corresponding to the AAV vector were observed as peak 2 to peak 4 in Fig. 3, large amounts of impurities were also contained. From these results, it can be understood that even if a solution containing an AAV vector is purified solely by anion-exchange chromatography, it is difficult to obtain a high-purity AAV.

### EXAMPLE 20 Preparation of an AAV-Binding Protein with the Removal of a Signal Peptide Sequence

(1) PCR was performed by the same method as in Example 10 (a-1) except that a vector capable of expressing AVR29c (SEQ ID NO: 37) was used as the template, and that oligonucleotides consisting of the sequences set forth in SEQ ID NO: 56 (5'-ACATATGTCTGCAGGCGAAAGCGAC-3') and SEQ ID NO: 57 (5'-CCTGCAGACATATGTATATCTCCTTC-3') were used as the PCR primers.
(2) From the PCR product obtained in (1), a plasmid (expression vector) pET-AVR29c(-) including a polynucleotide encoding AVR29c, in which amino acid substitutions at 29 positions had been introduced into the wild-type AAV-binding protein, and lacking a signal sequence at the N-terminus was obtained by the same method as in Example 9 (a-2).
(3) The nucleotide sequence of pET-AVR29c(-) was analyzed and confirmed by the same method as in Example 2 (8).

The amino acid sequence of AVR29c(-) with a polyhistidine tag attached thereto is presented in SEQ ID NO: 58. It should be noted that in SEQ ID NO: 58, serine (S) at position 2 to aspartic acid (D) at position 190 form the AAV-binding protein AVR29c(-) (corresponding to the region from position 25 to position 213 of SEQ ID NO: 2), and histidine (H) from position 191 to position 196 form the tag sequence. In SEQ ID NO: 58, furthermore, the aspartic acid in V317D is present at position 7, the histidine in N324H is present at position 14, the alanine in V326A is present at position 16, the lysine in N329K is present at position 19, the valine in A330V is present at position 20, the leucine in Q334L is present at position 24, the valine in E335V is present at position 25, the alanine in T341A is present at position 31, the serine in Y342S is present at position 32, the glutamic acid in K362E is present at position 52, the phenylalanine in I366F is present at position 56, the asparagine in K371N is present at position 61, the tyrosine in F379Y is present at position 69, the arginine in K380R is present at position 70, the alanine in V381A is present at position 71, the valine in I382V is present at position 72, the serine in G390S is present at position 80, the alanine in V394A is present at position 84, the glutamic acid in K399E is present at position 89, the glycine in E401G is present at position 91, the alanine in T426A is present at position 116, the arginine in K455R is present at position 145, the proline in A461P is present at position 151, the asparagine in K467N is present at position 157, the arginine in S476R is present at position 166, the threonine in S482T is present at position 172, the aspartic acid in N487D is present at position 177, the aspartic acid in N492D is present at position 182, and the glutamic acid in K497E is present at position 187.

(4) Culturing of a transformant and harvesting of bacterial cells were performed by the same method as in Example 4 (1) to (4) except that a transformant obtained by transforming the Escherichia coli strain BL21(DE3) with a plasmid including a polynucleotide encoding the AAV-binding protein AVR29c(-) (SEQ ID NO: 58) obtained in (3).
(5) An AVR29c(-) solution was prepared from the harvested bacterial cells by the same method as in Example 10 (2) to (4).
(6) The AVR29c prepared in Example 10 and the AVR29c(-) prepared in (5) were subjected to SDS-PAGE and stained using a CBB (Coomassie Brilliant Blue) staining solution (manufactured by FUJIFILM Wako Pure Chemical Corporation).

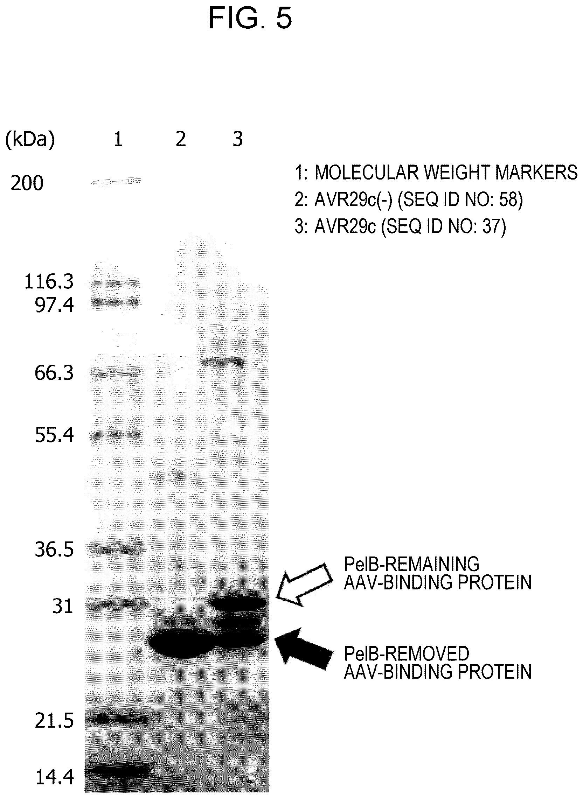

The obtained results are presented in Fig. 5. Whereas an AVR29c + His tag was observed as the main band for AVR29c(-) (SEQ ID NO: 58, lane 2), both an AVR29c + His tag with the signal peptide PelB remaining and an AVR29c + His tag with the PelB removed were present for AVR29c (SEQ ID NO: 37, lane 3), indicating low homogeneity.

### EXAMPLE 21 Performance Evaluation of AAV-Binding Proteins

The evaluation of affinity for an AAV was performed using the AVR29c (SEQ ID NO: 37) prepared in Example 10 and the AVR29c(-) prepared in Example 20 (SEQ ID NO: 58).

(1) Immobilization of the VLP2 prepared in Example 1 in the wells of a 96-well microplate (manufactured by Thermo Fisher Scientific Inc.) and blocking were performed by the method described in Example 2 (6-1).
(2) After the wells were washed with the washing buffer described in Example 2 (6-2), a solution containing AVR29c or AVR29c(-) was added to the wells and allowed to react with VLP2 (1 hour at 30°C)
(3) After completion of the reaction, a volume of an eluting buffer (a 200 mM acetate buffer (pH 3.0 or pH 2.0) containing 0.5 M sodium chloride) or a 20 mM Tris hydrochloride buffer (pH 7.4) equal to that of the solution added in (2) was added, and the reaction was allowed to proceed for 10 minutes at 25°C.
(4) After completion of the reaction, the solution in the wells was removed, and the wells were washed with the washing buffer described in Example 2 (6-2). Then 100 µL/well of Anti-6 His Antibody (manufactured by Bethyl Laboratories, Inc.), diluted to 100 ng/mL, was added.
(5) The reaction and absorbance measurement were performed by the same method as in Example 2 (6-4). The percentage of the AAV-binding protein remaining in the wells was calculated by dividing the absorbance of the wells after the reaction with the addition of eluting buffer in (3) by the absorbance of the wells after the reaction with the addition of a Tris hydrochloride buffer.

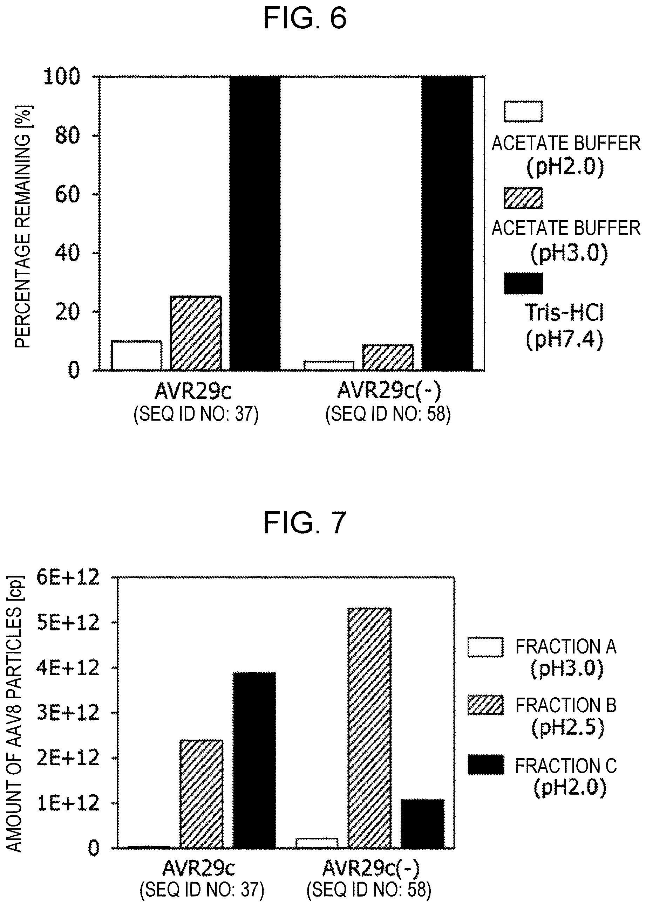

The measurement results are presented in Fig. 6. The percentage of the AAV-binding protein remaining in the wells after the addition of eluting buffer (acetate buffer) was lower for AVR29c(-) (SEQ ID NO: 58) than for AVR29c (SEQ ID NO: 37); AVR29c(-) was dissociated more efficiently from the VLP2 immobilized in the wells. As illustrated in Fig. 5 (Example 20), AVR29c(-) (SEQ ID NO: 58) has higher homogeneity as an AAV-binding protein than AVR29c (SEQ ID NO: 37). The inventors, therefore, presume that this high homogeneity helped achieve the efficient dissociation of the AAV through the addition of eluting buffer.

### EXAMPLE 22 Preparation and Performance Evaluation of an AAV-Binding Protein-Immobilized Gel

(1) A gel in which the AVR29c(-) prepared in Example 22 had been immobilized onto a hydrophilic vinyl polymer for use as a separating agent (designated as an AVR29c(-)-immobilized gel) was prepared by the same method as in Example 13 (1).
(2) Approximately 10 mg each of the AVR29c-immobilized gel prepared in Example 13 (1) and the AVR29c(-)-immobilized gel prepared in (1) were applied to a 0.2-µm Cosmospin Filter (manufactured by Nacalai Tesque, Inc.), and the AAV8-EGFP solution prepared in Example 5 was added to reach 9.7 × 10¹² cp. Then the mixture was stirred for 1 hour at 25°C to cause AAV8-EGFP to be adsorbed onto the immobilized gel.
(3) The AAV8-EGFP solution was removed from the immobilized gel through a centrifugation operation, and then washing of the gel was performed by adding 100 µL of a washing solution (a 20 mM Tris hydrochloride buffer (pH 7.4) containing 0.5 M sodium chloride and 0.5 mM calcium chloride) and stirring for 3 minutes at 25°C.
(4) The washing solution in (3) was separated and removed from the immobilized gel through a centrifugation operation. Then 100 µL of eluent A (a 0.1 M acetate buffer (pH 3.0) containing 0.5 M sodium chloride) was added, and the mixture was stirred for 3 minutes at 25°C. After stirring, elution of the AAV8 adsorbed on the immobilized gel was performed by separating eluent A from the immobilized gel through a centrifugation operation. This operation was performed once again, and the fractions eluted in the two operations were combined. The combined fraction was defined as "fraction A."
(5) 100 µL of eluent B (a 0.1 M acetic acid (pH 2.5) containing 0.5 M sodium chloride) was added, and the mixture was stirred for 3 minutes at 25°C. After stirring, elution of the AAV8 remaining on the immobilized gel was performed by separating eluent B from the immobilized gel through a centrifugation operation. This operation was performed once again, and the fractions eluted in the two operations were combined. The combined fraction was defined as "fraction B."
(6) 100 µL of eluent C (a 0.1 M acetic acid (pH 2.0) containing 0.5 M sodium chloride) was added, and the mixture was stirred for 3 minutes at 25°C. After stirring, elution of the AAV8 remaining on the immobilized gel was performed by separating eluent C from the immobilized gel through a centrifugation operation. This operation was performed once again, and the fractions eluted in the two operations were combined. The combined fraction was defined as "fraction C."
(7) A TSKgel G6000PW size-exclusion chromatography column (manufactured by Tosoh Corporation) was connected to a Nexera ultra-high-performance liquid chromatograph (manufactured by Shimadzu Corporation) and equilibrated with eluent A (a 50 mM sodium acetate buffer (pH 6.0) containing 0.5 M sodium chloride, 0.01% (w/v) Tween 20 (manufactured by Sigma-Aldrich Co. LLC) and 0.01% (w/v) Pluronic F-68 (manufactured by Sigma-Aldrich Co. LLC)).
(8) A known concentration of AAV8-EGFP and fraction A obtained in (4), fraction B obtained in (5) and fraction C obtained in (6) were each applied to the column equilibrated in (7) and delivered at a flow rate of 1 mL/min, whereby a separation peak derived from AAV8-EGFP in each sample was obtained. The separation peak was detected by fluorescence intensity (280 nm excitation/350 nm emission).
(9) Based on the area of the separation peak obtained for the known concentration of AAV8-EGFP, the concentrations of AAV8 contained in fractions A, B and C were each quantified.

The obtained results are presented in Fig. 7. For the AVR29c-immobilized gel, the AAV8 concentration was highest in fraction C (pH 2.0). For the AVR29c(-)-immobilized gel, however, the AAV8 concentration was highest in fraction B (pH 2.5). As illustrated in Fig. 5 (Example 20), AVR29c(-) (SEQ ID NO: 58), used as the ligand in the immobilized gel, has higher homogeneity as an AAV-binding protein than AVR29c (SEQ ID NO: 37). The inventors, therefore, presume that this high homogeneity helped achieve the elution of the AAV under milder conditions.

### EXAMPLE 23 Purification Combining Affinity Chromatography and Anion-Exchange Chromatography (Part 2)

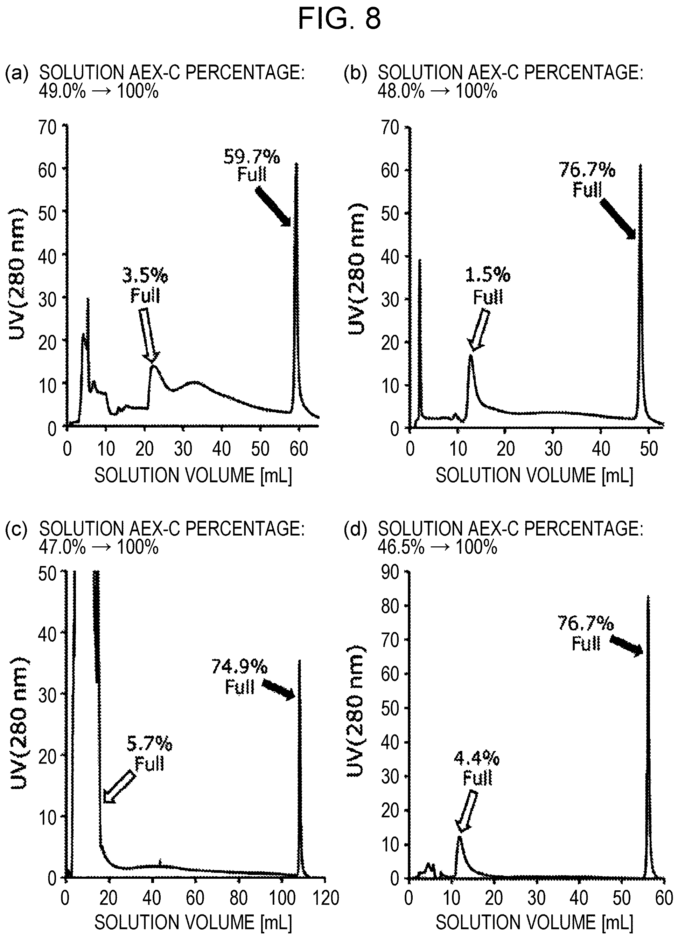

(1) The AVR29c column prepared in Example 17 was equilibrated with a 20 mmol/L Tris hydrochloride buffer (pH 7.4) containing 500 mmol/L sodium chloride (hereinafter also referred to as "equilibrating solution D").
(2) The AAV8-EGFP solution obtained in Example 5 was applied to the AVR29c column. After washing with equilibrating solution D, elution was performed using a 20 mmol/L Tris hydrochloride buffer (pH 7.4) containing 2.0 mol/L magnesium chloride. In this manner, a solution of AAV8-EGFP, an AAV vector, was obtained.
(3) The sample to be applied to the anion-exchange column was prepared by dialyzing the AAV vector (AAV8-EGFP) solution obtained in (2) with a volume of solution AEX-A 100 times larger than that of the vector solution.
(4) The AAV8-EGFP solution prepared in (3) was applied at 0.1 mL/min to 1.0-mL SkillPak TOYOPEARL GigaCap Q-650S (a high-capacity anion-exchange chromatography support-prepacked column for separation and purification, manufactured by Tosoh Corporation; hereinafter also referred to simply as "SkillPak GigaCap Q column") pre-equilibrated with solution AEX-A.
(5) After the SkillPak GigaCap Q column was washed by passing 2 CV of solution AEX-A at 0.5 mL/min, impurities adsorbed on the column were eluted by varying the electrical conductivity of the solution through adjustment of the mixing percentages of solution AEX-A and a 20 mmol/L Tris hydrochloride buffer (pH 9.0) containing 300 mmol/L choline chloride (hereinafter also referred to as "solution AEX-C"). Specifically, the mixing percentages were adjusted such that the percentage of solution AEX-C was any of:
   (a) 49.0% (electrical conductivity: 13.3 mS/cm (millisiemens per centimeter));
   (b) 48.0% (electrical conductivity: 13.0 mS/cm);
   (c) 47.0% (electrical conductivity: 12.8 mS/cm); or
   (d) 46.5% (electrical conductivity: 12.7 mS/cm).
   While this state was maintained, impurities were eluted by passing 30 CV to 60 CV.
(6) The AAV vector (AAV8-EGFP) was eluted by applying a step gradient that brought the percentage of solution AEX-C to 100% (electrical conductivity: 24.5 mS/cm).
(7) The percentages of Full AAVs contained in the eluted fractions obtained in (5) and (6) were each measured by Mass Photometry using Refeyn Two (manufactured by Refeyn Ltd.).
(8) The obtained chromatograms are presented in Fig. 8. The results of measurement of the Full ratio of the AAV vectors contained in the eluted fractions obtained in (5) (the peaks under the white arrows in Fig. 8) and (6) (the peaks under the black arrows in Fig. 8), furthermore, are presented in Table 16. It can be understood that when the electrical conductivity of the eluent used to elute impurities is set to 13.5 mS/cm or less while the electrical conductivity of the eluent used to elute the AAV vector is set to 15.0 mS/cm or more, a solution containing AAV vectors with a higher Full ratio can be obtained.

**[Table 16]**

| | | (a) | (b) | (c) | (d) |
|---|---|---|---|---|---|
| Eluent used in Example 23 (5) | | | | | |
| | AEX-C [%] | 49.0 | 48.0 | 47.0 | 46.5 |
| | Electrical conductivity [mS/cm] | 13.3 | 13.0 | 12.8 | 12.7 |

| Eluent used in Example 23 (6) | | | | | |
|---|---|---|---|---|---|
| | AEX-C [%] | 100 | 100 | 100 | 100 |
| | Electrical conductivity [mS/cm] | 24.5 | 24.5 | 24.5 | 24.5 |

| Full ratio [%] of the AAV vectors contained in the eluted fraction | | | | | |
|---|---|---|---|---|---|
| | Example 23 (5) (white arrow in Fig. 8) | 3.5 | 1.5 | 5.7 | 4.4 |
| | Example 23 (6) (black arrow in Fig. 8) | 59.7 | 76.7 | 74.9 | 76.7 |

### EXAMPLE 24 Purification Combining Affinity Chromatography and Anion-Exchange Chromatography (Part 3)

(1) The sample (AAV8-EGFP solution) to be applied to the anion-exchange column was prepared by purifying the AAV8-EGFP solution obtained in Example 5 using an AVR29c column by the method described in Example 23 (1) to (3).
(2) The AAV8-EGFP solution prepared in (1) was applied at 0.1 mL/min to a 5.0-mL SkillPak GigaCap Q column pre-equilibrated with solution AEX-A.
(3) After the SkillPak GigaCap Q column was washed by passing 2 CV of solution AEX-A at 0.5 mL/min, impurities were eluted by adjusting the mixing percentages such that the percentage of solution AEX-C was 46.0% (electrical conductivity: 12.5 mS/cm) and passing 20 CV while maintaining this state.
(4) The AAV vector (AAV8-EGFP) was eluted by applying a step gradient that brought the percentage of solution AEX-C to 100% (electrical conductivity: 24.5 mS/cm).
(5) The Full ratios contained in the AAV8-EGFP solution obtained in (1) and the eluted fractions obtained in (3) and (4) were each measured by Mass Photometry using Refeyn Two (manufactured by Refeyn Ltd.).

The chromatogram obtained by affinity chromatography (AF) purification (purification using an AVR29c column) is presented in Fig. 9 (a), and the chromatogram obtained by anion-exchange chromatography (AEX) purification (purification using a SkillPak GigaCap Q column) is presented in Fig. 9 (b). The results of measurement of the Full ratio of the AAV vectors contained in the AAV8-EGFP solution obtained in (1) (the peak indicated by the black arrow in Fig. 9 (a), AF-purified fraction) and in the eluted fractions obtained in (3) (the peak indicated by the white arrow in Fig. 9 (b), Fr18) and (4) (the peak indicated by the black arrow in Fig. 9 (b), Fr38), furthermore, are presented in Table 17. It can be understood that even if the capacity of the SkillPak GigaCap Q column is upsized from 1.0 mL (Example 23) to 5.0 mL, a solution containing AAV vectors with a high Full ratio can be obtained by the purification method according to the present disclosure.

**[Table 17]**

| | Full ratio [%] |
|---|---|
| AF-purified fraction | 35.1 |
| AEX-purified Fr18 | 0.7 |
| AEX-purified Fr38 | 67.8 |

### EXAMPLE 25 Evaluation of Infectivity after Purification by Anion-Exchange Chromatography

The AF-purified fraction obtained in Example 24 (1), Fr18 obtained in Example 24 (3) and Fr38 obtained in Example 24 (4) were each dialyzed with PBS (Phosphate Buffered Saline) containing 1 mmol/L magnesium chloride.

Then AAV-HT1080 cells (manufactured by Agilent Technologies, Inc.) were infected with 62500 particles to 10⁶ particles per cell.

The number of particles per cell was prepared by drawing a calibration curve using a size-exclusion chromatography column (G6000PW_{XL} column).

After culturing at 37°C in 5% CO₂ for 3 days, the cells were harvested. The solution was suspended several times using a pipette, and then the intensity of fluorescence derived from EGFP expressed in the cells was measured using Guava easyCyte (manufactured by Luminex Corporation). By measuring the percentage of cells with enhanced fluorescence intensity compared with cells without the addition of the AAV-infected solution, the percentage of cells infected with the AAV8-mutant vector (infection rate) was calculated.

The results of comparisons of positive rates at 3 days after infection are illustrated in Fig. 10.

It should be noted that in Fig. 10 (a), the viral dose, indicated on the X-axis, is represented by the number of particles per cell, and in Fig. 10 (b), it is represented by the number of vector genomes (VG) per cell calculated based on the Full ratio measured in Example 10 (5). In Fig. 10 (b), results of infection of cells with particles within Fr18, obtained in Example 24 (3), are excluded because they were out of range.

The number of vector genomes per cell was measured by qPCR. The PCR instrument used was the QuantStudio3 Real-Time PCR System (manufactured by Thermo Fisher Scientific Inc.). DNase I treatment was performed according to the protocol using the reagents included in the AAVpro Titration Kit (manufactured by Takara Bio Inc.) as pretreatment, and a portion was detected by PCR. The PCR reagent was TaqPath qPCR Master Mix (manufactured by Thermo Fisher Scientific Inc.), and the primers were SEQ ID NOs: 59 (CTCCATCACTAGGGGTTC) and 60 (TTGGGATTCCAGGCATGC). The probe was a TaqMan probe prepared by attaching FAM to SEQ ID NO: 61 (TCCCTTCCCTGTCCTT). For the vector concentration of the resulting solution, a calibration curve was constructed using positive standards included in the AAVpro Titration Kit (manufactured by Takara Bio Inc.).

The results for positive rates were consistent with the order of the Full ratios measured in Example 24 (5): highest in Fr38 (black circles), then in the AF-purified fraction (black triangles), and lowest in Fr18 (white circles, not illustrated in Fig. 7 (b) as being out of range). From these results, it can be understood that if choline chloride is contained in the eluent for the AAV adsorbed on the support for anion-exchange chromatography in the purification method according to the present disclosure, Full AAVs can be purified with high purity and in a state in which they have infectivity.

### Industrial Applicability

In an aspect, the adeno-associated virus (AAV)-binding protein according to the present disclosure is a protein in which amino acid residues at particular positions in a region corresponding to extracellular domains 1 (PKD1) and 2 (PKD2) of KIAA0319L (UniProt No. Q8IZA0) have been substituted with other amino acid residues. The AAV-binding protein according to the present disclosure can exhibit significantly improved alkaline resistance compared with known AAV-binding proteins. Accordingly, an adsorbent for an AAV in which the AAV-binding protein is used as the ligand can be washed with an alkali and can be repeatedly used for the purification of the AAV. The inventors, therefore, believe that the AAV-binding protein is useful for industrial manufacturing of AAVs.

In an aspect, the present disclosure is characterized in that an AAV contained in a sample is purified by a method comprising a step of applying the sample containing the AAV to an adsorbent including an insoluble support and an AAV-binding protein immobilized on the support to cause the AAV to be adsorbed onto the adsorbent, a step of eluting the AAV adsorbed on the adsorbent using an eluent, a step of applying an AAV-containing fraction eluted in the elution step to a support for anion-exchange chromatography to cause the AAV to be adsorbed onto the support and a step of eluting the AAV adsorbed on the support using an eluent. This can provide the advantage of enabling high-purity and easy purification of AAV vectors including a gene (Full AAV vectors), which have a therapeutic effect, from among the AAV vectors contained in the sample.

## Claims

1. An AAV-binding protein selected from any of (i) to (iii) below:
(i) an AAV-binding protein comprising at least amino acid residues from serine at position 25 to aspartic acid at position 213 of an amino acid sequence set forth in SEQ ID NO: 2, provided that at least any one of amino acid substitutions of (1) to (74) below has occurred at the amino acid residues from position 25 to position 213, the protein having AAV-binding activity;
(1) substitution of threonine at position 139 of SEQ ID NO: 2 with alanine,
(2) substitution of isoleucine at position 32 of SEQ ID NO: 2 with asparagine,
(3) substitution of leucine at position 34 of SEQ ID NO: 2 with glutamine,
(4) substitution of leucine at position 41 of SEQ ID NO: 2 with proline,
(5) substitution of asparagine at position 42 of SEQ ID NO: 2 with lysine or serine,
(6) substitution of valine at position 45 of SEQ ID NO: 2 with alanine,
(7) substitution of valine at position 48 of SEQ ID NO: 2 with alanine,
(8) substitution of lysine at position 51 of SEQ ID NO: 2 with arginine,
(9) substitution of glutamic acid at position 53 of SEQ ID NO: 2 with glycine,
(10) substitution of threonine at position 56 of SEQ ID NO: 2 with alanine or serine,
(11) substitution of tyrosine at position 57 of SEQ ID NO: 2 with phenylalanine,
(12) substitution of aspartic acid at position 58 of SEQ ID NO: 2 with glycine,
(13) substitution of glutamine at position 60 of SEQ ID NO: 2 with lysine,
(14) substitution of threonine at position 63 of SEQ ID NO: 2 with alanine,
(15) substitution of glutamic acid at position 73 of SEQ ID NO: 2 with aspartic acid,
(16) substitution of glutamine at position 78 of SEQ ID NO: 2 with arginine,
(17) substitution of isoleucine at position 79 of SEQ ID NO: 2 with phenylalanine,
(18) substitution of leucine at position 80 of SEQ ID NO: 2 with proline,
(19) substitution of lysine at position 81 of SEQ ID NO: 2 with arginine,
(20) substitution of leucine at position 82 of SEQ ID NO: 2 with serine,
(21) substitution of asparagine at position 84 of SEQ ID NO: 2 with aspartic acid or serine,
(22) substitution of leucine at position 89 of SEQ ID NO: 2 with proline,
(23) substitution of tyrosine at position 90 of SEQ ID NO: 2 with phenylalanine or asparagine,
(24) substitution of glutamic acid at position 91 of SEQ ID NO: 2 with glycine,
(25) substitution of tyrosine at position 92 of SEQ ID NO: 2 with serine,
(26) substitution of alanine at position 94 of SEQ ID NO: 2 with threonine,
(27) substitution of valine at position 95 of SEQ ID NO: 2 with isoleucine,
(28) substitution of glutamic acid at position 97 of SEQ ID NO: 2 with aspartic acid,
(29) substitution of histidine at position 102 of SEQ ID NO: 2 with arginine,
(30) substitution of valine at position 107 of SEQ ID NO: 2 with alanine or isoleucine,
(31) substitution of valine at position 109 of SEQ ID NO: 2 with glutamic acid,
(32) substitution of threonine at position 110 of SEQ ID NO: 2 with alanine or serine,
(33) substitution of glutamic acid at position 114 of SEQ ID NO: 2 with glycine,
(34) substitution of proline at position 115 of SEQ ID NO: 2 with serine,
(35) substitution of valine at position 125 of SEQ ID NO: 2 with alanine,
(36) substitution of aspartic acid at position 148 of SEQ ID NO: 2 with alanine,
(37) substitution of glutamic acid at position 166 of SEQ ID NO: 2 with aspartic acid,
(38) substitution of glutamic acid at position 167 of SEQ ID NO: 2 with aspartic acid,
(39) substitution of alanine at position 174 of SEQ ID NO: 2 with proline,
(40) substitution of serine at position 179 of SEQ ID NO: 2 with threonine,
(41) substitution of glutamine at position 180 of SEQ ID NO: 2 with asparagine,
(42) substitution of asparagine at position 185 of SEQ ID NO: 2 with lysine,
(43) substitution of lysine at position 210 of SEQ ID NO: 2 with glutamic acid,
(44) substitution of aspartic acid at position 213 of SEQ ID NO: 2 with asparagine,
(45) substitution of glycine at position 27 of SEQ ID NO: 2 with aspartic acid,
(46) substitution of aspartic acid at position 30 of SEQ ID NO: 2 with glycine,
(47) substitution of glutamine at position 31 of SEQ ID NO: 2 with arginine,
(48) substitution of proline at position 35 of SEQ ID NO: 2 with alanine,
(49) substitution of glutamic acid at position 38 of SEQ ID NO: 2 with valine,
(50) substitution of glutamine at position 60 of SEQ ID NO: 2 with arginine,
(51) substitution of glutamic acid at position 75 of SEQ ID NO: 2 with aspartic acid,
(52) substitution of histidine at position 76 of SEQ ID NO: 2 with proline,
(53) substitution of tyrosine at position 92 of SEQ ID NO: 2 with asparagine,
(54) substitution of glycine at position 105 of SEQ ID NO: 2 with glutamic acid,
(55) substitution of valine at position 111 of SEQ ID NO: 2 with alanine,
(56) substitution of glutamic acid at position 112 of SEQ ID NO: 2 with lysine,
(57) substitution of arginine at position 119 of SEQ ID NO: 2 with histidine,
(58) substitution of glutamine at position 128 of SEQ ID NO: 2 with arginine,
(59) substitution of phenylalanine at position 129 of SEQ ID NO: 2 with leucine,
(60) substitution of leucine at position 134 of SEQ ID NO: 2 with proline,
(61) substitution of serine at position 146 of SEQ ID NO: 2 with glycine,
(62) substitution of threonine at position 147 of SEQ ID NO: 2 with serine,
(63) substitution of aspartic acid at position 150 of SEQ ID NO: 2 with valine,
(64) substitution of glutamic acid at position 158 of SEQ ID NO: 2 with lysine,
(65) substitution of lysine at position 168 of SEQ ID NO: 2 with arginine,
(66) substitution of serine at position 170 of SEQ ID NO: 2 with arginine,
(67) substitution of threonine at position 187 of SEQ ID NO: 2 with arginine,
(68) substitution of threonine at position 191 of SEQ ID NO: 2 with isoleucine,
(69) substitution of alanine at position 198 of SEQ ID NO: 2 with valine,
(70) substitution of threonine at position 199 of SEQ ID NO: 2 with alanine,
(71) substitution of leucine at position 206 of SEQ ID NO: 2 with methionine,
(72) substitution of asparagine at position 209 of SEQ ID NO: 2 with glutamic acid,
(73) substitution of valine at position 212 of SEQ ID NO: 2 with isoleucine and
(74) substitution of aspartic acid at position 213 of SEQ ID NO: 2 with glycine;
(ii) an AAV-binding protein comprising at least amino acid residues from serine at position 25 to aspartic acid at position 213 of an amino acid sequence set forth in SEQ ID NO: 2, provided that at least any one of the amino acid substitutions of (1) to (74) has occurred at the amino acid residues from position 25 to position 213, and any one or more of substitution, deletion, insertion or addition of one or several amino acid residues at one or several positions, other than the amino acid substitutions specified in (1) to (74), have further occurred, the protein having AAV-binding activity; or
(iii) an AAV-binding protein comprising an amino acid sequence having a homology of 70% or more to an entire amino acid sequence in which at least any one of the amino acid substitutions of (1) to (74) has occurred in an amino acid sequence from serine at position 25 to aspartic acid at position 213 of an amino acid sequence set forth in SEQ ID NO: 2, wherein the at least any one amino acid substitution is retained, the protein having AAV-binding activity.

2. The AAV-binding protein according to Claim 1 selected from any of (iv) to (vi) below:
(iv) an AAV-binding protein comprising at least amino acid residues from serine at position 25 to aspartic acid at position 213 of an amino acid sequence set forth in SEQ ID NO: 2, provided that at least an amino acid substitution of (1) below has occurred at the amino acid residues from position 25 to position 213, the protein having AAV-binding activity;
(1) substitution of threonine at position 139 of SEQ ID NO: 2 with alanine;
(v) an AAV-binding protein comprising at least amino acid residues from serine at position 25 to aspartic acid at position 213 of an amino acid sequence set forth in SEQ ID NO: 2, provided that the amino acid substitution of (1) has occurred at the amino acid residues from position 25 to position 213, and any one or more of substitution, deletion, insertion or addition of one or several amino acid residues at one or several positions, other than the amino acid substitution specified in (1), have further occurred, the protein having AAV-binding activity; or
(vi) an AAV-binding protein comprising an amino acid sequence having a homology of 70% or more to an entire amino acid sequence in which at least the amino acid substitution of (1) has occurred in an amino acid sequence from serine at position 25 to aspartic acid at position 213 of an amino acid sequence set forth in SEQ ID NO: 2, wherein the at least any one amino acid substitution is retained, the protein having AAV-binding activity.

3. The AAV-binding protein according to Claim 1, wherein the protein includes at least amino acid residues from serine at position 25 to aspartic acid at position 213 of an amino acid sequence set forth in SEQ ID NO: 2, provided that at least amino acid substitutions specified in any of (A) to (P) below have occurred at the amino acid residues from position 25 to position 213, the protein having AAV-binding activity;
(A) substitution of asparagine at position 42 of SEQ ID NO: 2 with lysine, substitution of glutamic acid at position 114 of SEQ ID NO: 2 with glycine and substitution of threonine at position 139 of SEQ ID NO: 2 with alanine,
(B) substitution of lysine at position 51 of SEQ ID NO: 2 with arginine, substitution of threonine at position 139 of SEQ ID NO: 2 with alanine and substitution of alanine at position 174 of SEQ ID NO: 2 with proline,
(C) substitution of asparagine at position 42 of SEQ ID NO: 2 with lysine, substitution of glutamic acid at position 114 of SEQ ID NO: 2 with glycine, substitution of threonine at position 139 of SEQ ID NO: 2 with alanine and substitution of alanine at position 174 of SEQ ID NO: 2 with proline,
(D) substitution of lysine at position 51 of SEQ ID NO: 2 with arginine, substitution of glutamic acid at position 114 of SEQ ID NO: 2 with glycine, substitution of threonine at position 139 of SEQ ID NO: 2 with alanine and substitution of alanine at position 174 of SEQ ID NO: 2 with proline,
(E) substitution of asparagine at position 42 of SEQ ID NO: 2 with lysine, substitution of lysine at position 51 of SEQ ID NO: 2 with arginine, substitution of glutamic acid at position 114 of SEQ ID NO: 2 with glycine, substitution of threonine at position 139 of SEQ ID NO: 2 with alanine and substitution of alanine at position 174 of SEQ ID NO: 2 with proline,
(F) substitution of asparagine at position 42 of SEQ ID NO: 2 with lysine, substitution of glutamic acid at position 114 of SEQ ID NO: 2 with glycine, substitution of threonine at position 139 of SEQ ID NO: 2 with alanine, substitution of alanine at position 174 of SEQ ID NO: 2 with proline and substitution of glutamine at position 180 of SEQ ID NO: 2 with asparagine,
(G) substitution of asparagine at position 42 of SEQ ID NO: 2 with lysine, substitution of isoleucine at position 79 of SEQ ID NO: 2 with phenylalanine, substitution of glutamic acid at position 114 of SEQ ID NO: 2 with glycine, substitution of threonine at position 139 of SEQ ID NO: 2 with alanine, substitution of alanine at position 174 of SEQ ID NO: 2 with proline, substitution of glutamine at position 180 of SEQ ID NO: 2 with asparagine and substitution of threonine at position 187 of SEQ ID NO: 2 with arginine,
(H) substitution of asparagine at position 42 of SEQ ID NO: 2 with lysine, substitution of valine at position 107 of SEQ ID NO: 2 with alanine, substitution of glutamic acid at position 114 of SEQ ID NO: 2 with glycine, substitution of threonine at position 139 of SEQ ID NO: 2 with alanine, substitution of alanine at position 174 of SEQ ID NO: 2 with proline, substitution of glutamine at position 180 of SEQ ID NO: 2 with asparagine and substitution of threonine at position 187 of SEQ ID NO: 2 with arginine,
(I) substitution of asparagine at position 42 of SEQ ID NO: 2 with lysine, substitution of glutamic acid at position 114 of SEQ ID NO: 2 with glycine, substitution of threonine at position 139 of SEQ ID NO: 2 with alanine, substitution of lysine at position 168 of SEQ ID NO: 2 with arginine, substitution of alanine at position 174 of SEQ ID NO: 2 with proline, substitution of glutamine at position 180 of SEQ ID NO: 2 with asparagine and substitution of threonine at position 187 of SEQ ID NO: 2 with arginine,
(J) substitution of asparagine at position 42 of SEQ ID NO: 2 with lysine, substitution of isoleucine at position 79 of SEQ ID NO: 2 with phenylalanine, substitution of valine at position 107 of SEQ ID NO: 2 with alanine, substitution of glutamic acid at position 114 of SEQ ID NO: 2 with glycine, substitution of threonine at position 139 of SEQ ID NO: 2 with alanine, substitution of lysine at position 168 of SEQ ID NO: 2 with arginine, substitution of alanine at position 174 of SEQ ID NO: 2 with proline and substitution of glutamine at position 180 of SEQ ID NO: 2 with asparagine,
(K) substitution of asparagine at position 42 of SEQ ID NO: 2 with lysine, substitution of isoleucine at position 79 of SEQ ID NO: 2 with phenylalanine, substitution of valine at position 107 of SEQ ID NO: 2 with alanine, substitution of glutamic acid at position 114 of SEQ ID NO: 2 with glycine, substitution of threonine at position 139 of SEQ ID NO: 2 with alanine, substitution of lysine at position 168 of SEQ ID NO: 2 with arginine, substitution of alanine at position 174 of SEQ ID NO: 2 with proline, substitution of glutamine at position 180 of SEQ ID NO: 2 with asparagine and substitution of threonine at position 187 of SEQ ID NO: 2 with arginine,
(L) substitution of glycine at position 27 of SEQ ID NO: 2 with aspartic acid, substitution of asparagine at position 42 of SEQ ID NO: 2 with lysine, substitution of isoleucine at position 79 of SEQ ID NO: 2 with phenylalanine, substitution of valine at position 107 of SEQ ID NO: 2 with alanine, substitution of glutamic acid at position 114 of SEQ ID NO: 2 with glycine, substitution of threonine at position 139 of SEQ ID NO: 2 with alanine, substitution of lysine at position 168 of SEQ ID NO: 2 with arginine, substitution of alanine at position 174 of SEQ ID NO: 2 with proline and substitution of glutamine at position 180 of SEQ ID NO: 2 with asparagine,
(M) substitution of asparagine at position 42 of SEQ ID NO: 2 with lysine, substitution of isoleucine at position 79 of SEQ ID NO: 2 with phenylalanine, substitution of valine at position 107 of SEQ ID NO: 2 with alanine, substitution of glutamic acid at position 114 of SEQ ID NO: 2 with glycine, substitution of threonine at position 139 of SEQ ID NO: 2 with alanine, substitution of lysine at position 168 of SEQ ID NO: 2 with arginine, substitution of alanine at position 174 of SEQ ID NO: 2 with proline, substitution of glutamine at position 180 of SEQ ID NO: 2 with asparagine and substitution of lysine at position 210 of SEQ ID NO: 2 with glutamic acid,
(N) substitution of asparagine at position 42 of SEQ ID NO: 2 with lysine, substitution of isoleucine at position 79 of SEQ ID NO: 2 with phenylalanine, substitution of valine at position 107 of SEQ ID NO: 2 with alanine, substitution of glutamic acid at position 114 of SEQ ID NO: 2 with glycine, substitution of threonine at position 139 of SEQ ID NO: 2 with alanine, substitution of lysine at position 168 of SEQ ID NO: 2 with arginine, substitution of alanine at position 174 of SEQ ID NO: 2 with proline, substitution of glutamine at position 180 of SEQ ID NO: 2 with asparagine and substitution of valine at position 212 of SEQ ID NO: 2 with isoleucine and
(O) substitution of glycine at position 27 of SEQ ID NO: 2 with aspartic acid, substitution of asparagine at position 42 of SEQ ID NO: 2 with lysine, substitution of isoleucine at position 79 of SEQ ID NO: 2 with phenylalanine, substitution of valine at position 107 of SEQ ID NO: 2 with alanine, substitution of glutamic acid at position 114 of SEQ ID NO: 2 with glycine, substitution of threonine at position 139 of SEQ ID NO: 2 with alanine, substitution of lysine at position 168 of SEQ ID NO: 2 with arginine, substitution of alanine at position 174 of SEQ ID NO: 2 with proline, substitution of glutamine at position 180 of SEQ ID NO: 2 with asparagine, substitution of lysine at position 210 of SEQ ID NO: 2 with glutamic acid and substitution of valine at position 212 of SEQ ID NO: 2 with isoleucine.

4. A polynucleotide encoding the AAV-binding protein according to any of Claims 1 to 3.

5. An expression vector comprising the polynucleotide according to Claim 4.

6. A transformant obtained by transforming Escherichia coli with the expression vector according to Claim 5.

7. A method for manufacturing an AAV-binding protein, the method comprising a step of allowing an AAV-binding protein to be expressed by culturing the transformant according to Claim 6 and a step of recovering the expressed AAV-binding protein from a resulting culture.

8. An AAV adsorbent comprising an insoluble support and the AAV-binding protein according to any of Claims 1 to 3 immobilized on the support.

9. A column comprising the AAV adsorbent according to Claim 8.

10. A method for purifying or analyzing an AAV, the method comprising a step of applying a solution containing an AAV to the column according to Claim 9 to cause the AAV to be adsorbed onto the adsorbent and a step of eluting the AAV adsorbed on the adsorbent using an eluent.

11. The method according to Claim 10 for purifying or analyzing an AAV, further comprising a step of washing, with an alkali solution, the AAV adsorbent that has completed the step of eluting the AAV.

12. A method for purifying an AAV contained in a sample, the method comprising:
a step of applying a sample containing an AAV to an adsorbent including an insoluble support and an AAV-binding protein immobilized on the insoluble support to cause the AAV to be adsorbed onto the adsorbent;
a step of eluting the AAV adsorbed on the adsorbent;
a step of applying an AAV-containing fraction eluted in the elution step to a support for anion-exchange chromatography to cause the AAV to be adsorbed onto the support for anion-exchange chromatography; and
a step of eluting the AAV adsorbed on the support for anion-exchange chromatography.

13. A method for purifying an AAV contained in a sample, the method comprising:
a step of applying a sample containing an AAV to an adsorbent including an insoluble support and an AAV-binding protein immobilized on the insoluble support to cause the AAV to be adsorbed onto the adsorbent;
a step of eluting the AAV adsorbed on the adsorbent;
a step of applying an AAV-containing fraction eluted in the elution step to a support for anion-exchange chromatography to cause the AAV to be adsorbed onto the support for anion-exchange chromatography; and
a step of eluting the AAV adsorbed on the support for anion-exchange chromatography, wherein:
the AAV-binding protein is the AAV-binding protein according to any of Claims 1 to 3.

14. The purification method according to Claim 12 or 13, wherein the step of eluting the AAV adsorbed on the support for anion-exchange chromatography is a step of eluting the AAV adsorbed on the support using an eluent having an electrical conductivity of 13.5 mS/cm or less and then eluting the AAV remaining on the support using an eluent having an electrical conductivity of 15.0 mS/cm or more.

15. The purification method according to Claim 14, wherein the eluents contain choline chloride.
